# EUROPEAN PATENT SPECIFICATION

(11) **EP 1 631 316 B1**
(45) Date of publication and mention of the grant of the patent: **11.04.2012**
(21) Application number: 04752149.7
(22) Date of filing: 14.05.2004
(51) Int. Cl.: A61K 39/40, A61K 39/385, C07K 16/00, C12P 21/08

(54) **TARGETED BIOCIDES**
GEZIELTE BIOZIDE
BIOCIDES CIBLES

(30) Priority: 15.05.2003 US 470841 P; 13.05.2004 US 844837
(43) Date of publication of application: 08.03.2006
(60) Divisional application: 10179345.3
(73) Proprietor: Iogenetics, Inc., Middleton, WI 53562 (US)
(72) Inventor: IMBODEN, Michael, Madison, WI 53711 (US); HOMAN, Jane, Hillpoint, WI 53937 (US); BREMEL, Robert, D., Hillpoint, WI 53937 (US)
(74) Representative: Glawe, Delfs, Moll
(86) International application number: PCT/US2004/015047
(87) International publication number: WO 2004/110143

(56) References cited:
- US-A1- 2003 114 377
- US-A1- 2004 052 814
- US-B1- 6 265 187
- US-B1- 6 562 617
- YOSHIDA S ET AL: "Bacteria expressing single-chain immunotoxin inhibit malaria parasite development in mosquitoes" MOLECULAR AND BIOCHEMICAL PARASITOLOGY, vol. 113, no. 1, March 2001 (2001-03), pages 89-96, XP002429871 ISSN: 0166-6851
- SCHULTZ ET AL: 'BPI-ANCA is found in reactive arthritis caused by Yersinia and Salmonell infection and recignise exclusively the C-terminal part of the BPI molecule.' SCAND. J. REUMALTOL. vol. 29, 2000, pages 226 - 231, XP008050132
- MEDVEDEV A.E. ET AL: 'Regulation of fas and fas-ligand expression in NK cells by cytokines and the involvement of fas-ligand in NK/LAK cell-mediated cytotoxicity.' CYTOKINE. vol. 9, no. 6, 06 June 1997, pages 394 - 404, XP002988698

## Description

This Application claims priority to provisional patent application serial number 60/470,841 filed 05/15/03.

### FIELD OF THE INVENTION

The present invention is defined in the claims and relates to retroviral constructs that encode novel fusion proteins, and to methods of using and producing the same. In particular, the present invention relates to methods of producing a fusion protein comprising a microorganism targeting molecule wherein said microorganism targeting molecule is CD14 and a biocide (*e.g.*, bactericidal enzyme) in cell cultures. The present invention also relates to therapeutic and prophylactic methods of using a fusion protein comprising CD14 and a biocide in health care (*e.g*., human and veterinary), agriculture (*e.g.,* animal and plant production), and food processing (*e.g.*, beef carcass processing). The present invention also relates to methods of using a fusion protein comprising CD14 and a biocide in various diagnostic applications in number of diverse fields such as agriculture, medicine, and national defense.

### BACKGROUND OF THE INVENTION

The majority of people in the industrialized world have access to an abundance of inexpensive processed food products. The safety, quality, and wholesomeness of these products are usually unquestioned. The availability of inexpensive food products is largely a result of advances in farm mechanization and improved industries of scale in food processing and distribution operations. The mechanization of the family farm has not come without certain drawbacks however.
One of the drawbacks of large-scale food processing operations, and of meat processing in particular, is the occasional contamination (*e.g.*, bacterial, fungal, *etc*.) and subsequent distribution of large quantities of contaminated products sometimes with dire consequences. Food safety researchers have determined that the introduction of even a few contaminated carcasses into the production lines of large scale food processing operations is often enough to contaminate entire batches of product. The meat packing industry is particularly susceptible to carcass contamination during dehiding, evisceration, splitting, chilling, and fabrication. Further contamination of previously uncontaminated meat products may occur during grinding, processing, and transport. This type of contamination has lead to several major meat product recalls, including the recall of 24 million pounds of ground beef by the Hudson Beef Co. in 1997, and more recently, the recall of 19 million pounds of beef and related products by the ConAgra Beef Company in July 2002. (*See,* Recall Release, FSIS-RC-055-2002). The economic impact of food safety and spoilage is very large. USDA ERS estimates that the leading six bacterial food borne pathogens cause $2.9-6.7 billion in medical costs and lost productivity annually in the US (Buzby et al., Bacterial Foodborne Disease: Medical Costs and Productivity Losses. 1996. Food and Consumer Economics Division, Economic Research Service U.S. Department of Agriculture. Agricultural Economic Report 741)

Many meat product recalls are the result of contamination by the bacterium *Escherichia coli* O157:H7. This bacterium is commonly isolated from the gastrointestinal tract and feces of cattle. Direct contact with cattle can be a source of human infection. However, the principal route of transmission to humans is through fecal contamination of carcasses at slaughter. (J. Tuttle et al., Epidemiol Infect., 122:185-192 [1999]). Every year in the United States the O157:H7 bacterium causes about 70,000 cases of hemorrhagic diarrhea and renal disease. Children, the elderly, and the immunocompromised are most susceptible to foodborne illness caused by *Escherichia coli* O157:H7. Virulent strains of *Escherichia coli* are not the only foodborne pathogens of concern.

*Listeria monocytogenes* has emerged as another dangerous, but relatively uncommon foodborne pathogen. Despite being an uncommon source of illness, *L. monocytogenes* is ubiquitous in agricultural and food processing environments and can cause serious human and animal infections. The infection caused by *L. monocytogenes* is commonly called Listeriosis. Listeriosis occurs in sporadic and epidemic forms throughout the world. (*See e.g.,* B. Lorber, Clin. Infect. Dis., 24(1):1-9 [1997]; J.M. Farber et al., Microbiol. Rev., 55:476-511 [1991]; and W.F. Schlech, Clin. Infect. Dis., 31:770-775 [2000]). A multistate outbreak of Listeriosis has been reported in the United States. (Morb. Mortal. Wkly. Report, 49(50):1129-1130 [2000] *erratum* in Morb. Mortal. Wkly. Report, 50(6):101 [2001]). Since May 2000, 29 illnesses caused by a strain of *Listeria monocytogenes* have been identified in 10 states: New York (15 cases); Georgia (3 cases); Connecticut, Ohio, and Michigan (2 cases each); and California, Pennsylvania, Tennes*See,* Utah, and Wisconsin (1 case each).

Listeriosis, in its most severe form, is an invasive disease that affects immunocompromised patients and has the highest case-fatality rate of any foodborne illnesses. (B.G. Gellin et al., Amer. J. Epidemiol., 133:392-401 [1991]; D.B. Louria et al., Ann. NY Acad. Sci., 174:545-551 [1970]; J. McLauchlin, Epidemiol. Infect., 104:191-201 [1990]; V. Goulet and P. Marchetti, Scand. J. Infect. Dis., 28:367-374 [1996]; and C.J. Bula et al., Clin. Infect Dis., 20:66-72 [1995]). In immunocompetent persons, it can also cause severe disease as well as outbreaks of benign febrile gastroenteritis. (P. Aureli et al., New Engl. J. Med., 342:1236-41 [2000]). Another form of human disease is perinatal infection, which is associated with a high rate of fetal loss (including full-term stillbirths) and serious neonatal disease (J. McLauchlin, Epidemiol. Infect., 104:181-190 [1990]).

Most, perhaps all, of listeriosis in humans occurs after consumption of contaminated food (*e.g.,* meat and cheese) products. (A. Schuchat et al., J. Amer. Med. Assoc., 267:2041-2045 [1992]). While uncommon, Listeriosis causes about half the foodborne disease fatalities in the US each year. Additionally, many mild cases of listeriosis and inapparent *Listeria* infections go unreported. For those susceptible to listeriosis, ingestion of even small doses of *L. monocytogenes* is often sufficient for infection. About 2,500 cases of listeriosis are reported in the US each year, of these about 20% or 500 cases are fatal.

In 1989, the USDA FSIS implemented a testing program for *L. monocytogenes* in cooked meat products and adopted a zero tolerance position for *L. monocytogenes* contamination in ready to eat products. Guidelines promulgated by the American Association of Meat Processors for current Good Manufacturing Practices for Ready to Eat meat products address the need for environmental monitoring for Listeria as a component of HACCP programs. The ecology of *L. monocytogenes* and its increasing prevalence and/or detection in food preparation establishments has lead to major recalls of processed meat products. In October 2002 the USDA issued a recall notice, which when further expanded, constituted the largest meat product recall on record for 28 million pounds of processed turkey products (*See,* USDA FSIS Recall Notification Report 090-2002 EXP Recall from Pilgrims Pride Corp dba Wampler Foods Inc. 11-4-2002). The recall was in response to detection of *L. monocytogenes* at multiple points in the facilities and equipment used to process the recalled turkey.

A number of approaches have been tried to increase the safety and wholesomeness of the nation's meat and agricultural products. For example, some approaches have focused on the exposing food products to one or more types of pathogen destroying processes, including ionizing radiation or ultra high temperatures and pressures. (*See e.g.,* US 5,891,490; 6,013,918; 6,086,936; and 6,165,526 *etc*.). US 6,165,526 is representative of these approaches. This patent describes an UV radiation and ultra high temperature method for sterilizing food products.

A number of other approaches have focused on providing mixtures of chemicals (e.g., acids, surfactants, emulsifying agents, and organic phosphates) that inactivate bacteria and bacterial spores in food products. (*See e.g.,* US 5,550,145; and 5,618,840). US 5,618,840, for instance, describes an antibacterial oil-in-water emulsion for inhibiting the growth of *Helicobacter pylori.*

The various compositions and methods previously described for food sterilization have certain advantages and certain other disadvantages. One disadvantage is that the manufacture and additional or large quantities of artificial chemicals to food products can be costly and logistically difficult. Moreover, the current chemical food sterilization agents are indiscriminate and are thus inappropriate for addition into food products such as cheese and yogurt that require the beneficial action of certain bacteria for their production. The addition of artificial chemical compounds to food products or subjecting the products to irradiation or temperature and pressure extremes can also produce unpleasant organoleptic qualities. Another disadvantage is the publics' generally negative perception of food irradiation and the addition of chemical additives.

Still other efforts have been directed to producing food washes to remove residual surface impurities such as waxes and pesticides sometimes acquired during food product production, processing, and transporting. For instance, US 6,367,488 describes a chemical wash for fruits and vegetables made from surfactants, such as oleate, and alcohol ethoxylates, and neutralized phosphoric acid. While these washes are useful for removing surface contaminates and surface bacteria from solid food products, these compositions are inappropriate for sterilizing homogenized food products such as ground beef.

While each of these above-mentioned compositions and methods has particular advantages and disadvantages, the need still exists for compositions and methods that reduce the amount of pathogenic bacteria shed by feedlot animals (*e.g.*, bovines, porcines, and the like), that induce immunity in feedlot animals to pathogens, and for edible compositions that safety destroy harmful foodborne pathogens.

A further major economic problem confronting the food processing industry is that of bacterial spoilage. In particular, dairy and processed meat products are susceptible to bacterial spoilage by organisms such as the Lactic acid bacteria (*e.g., Lactobacillus etc.)* (Kraft AA. Health hazards vs. food spoilage. Boca Raton, FL.: CRC Press, Inc., 1992). These organisms are widely distributed in nature, and can easily out-compete other bacteria under low oxygen tension and low pH conditions that are common in processed dairy and meat foods (Stamer. Lactic acid bacteria. In: Defigueiredo MP and Splittstoesser DF eds. Westport, CT: AVI Publishing, 1976). Over 20% of the fruit and vegetable products harvested for human consumption are believed to be lost to post-harvest microbial spoilage (Jay, J. Modem Food Microbiology 4th ed Van Norstand Reinhold New York, 1992).

### SUMMARY OF THE INVENTION

The present invention is defined in the claims and relates to retroviral constructs that encode novel fusion proteins, and to methods of using and producing the same Examples and figures which are not covered by the claims are used as referential examples which are useful for understanding the invention and are not embodiments of the invention. In particular, the present invention relates to methods of producing a fusion protein comprising a microorganism targeting molecule wherein said microorganism targeting molecule is CD14 and a biocide (*e.g.*, bactericidal enzyme) in cell cultures. The present invention also relates to therapeutic and prophylactic methods of using a fusion protein comprising CD14 and a biocide in health care (*e.g.,* human and veterinary), agriculture (*e.g.*, animal and plant production), and food processing (*e.g*., beef carcass processing). The present invention also relates to methods of using a fusion protein comprising CD14 and a biocide in various diagnostic applications in number of diverse fields such as agriculture, medicine, and national defense.

In some embodiments, the present invention provides a composition comprising a recombinant fusion protein, wherein the protein comprises CD14 joined to at least a portion of a protein biocide molecule, wherein CD14 binds to a pathogenic or spoilage microorganism. The microorganism targeting molecule may bind to pathogen associated molecular patterns. The microorganism targeting molecule is CD14. Further described herein is a microorganism targeting molecule which comprises at least a portion of an immunoglobulin molecule. The microorganism targeting molecule may bind to a bacteria, a parasite, a protozoan, an apicomplexan protozoan (*e.g.,* coccidian, cryptosporidian, toxoplasman, malarian or trypanosomatid protozoans), a fungus, a virus, or a foodborne or waterborne pathogen (*e.g., E coli spp., Listeria monocytogenes, Salmonella spp, Staphylococcus, Clostridium botulinum, Clostridium perfringens* or *Cryptosporidium parvum)* or a food spoilage organism (e.g., *Lactobacillus spp, Leuconostoc spp, Pediococcus spp,* and *Streptococcus spp).* The immunoglobulin molecule can be derived from a monoclonal antibody. The monoclonal antibody can be dimeric. The monoclonal antibody can be trimeric. The monoclonal antibody can be tetrameric. The monoclonal antibody can be pentameric. The monoclonal antibody can be hexameric. The monoclonal antibody may comprises IgG1, IgG2, IgG3, IgG4, IgM, IgA1, IgA2, IgA_{sec}, IgD, or IgE. The protein biocide molecule may be attached to a J chain of the monoclonal antibody. The microorganism targeting molecule and the at least a portion of a protein biocide molecule may be joined by a poly amino acid linker molecule from about 2 to 500, preferably from about 5 to 100, and even more preferably from about 10 to 30 amino acids long. In some embodiments, the poly amino acid linker molecule consists of amino acids selected from the group consisting of Gly, Ser, Asn, Thr, Pro, and Ala. In some embodiments, the amino acid linker comprises a sequence of amino acid residues having the formula:

(Serₙ₋Glyₓ)_{y}

wherein n ≥ 1,
wherein x ≥ 1, and
wherein y ≥ 1. In some embodiments, n =1, wherein x = 4, and wherein y ≥ 1. In other embodiments, y = 1, 2, 3, 4, 5, 6, 7, or 8. The protein biocide may comprise at least an active portion of an enzyme. In some embodiments, the enzyme comprises lysozyme, phopholipase A2, lactoferrin, lactoperoxidase, bacterial permeability increasing protein, lysostaphin, or aprotinin.

The present invention further provides a retroviral construct comprising a nucleic acid sequence encoding a microorganism targeting molecule linked to a portion of a biocide molecule, wherein the microorganism targeting molecule is CD14.

Described herein is a method of treating an object, comprising: providing a recombinant fusion protein, wherein the protein comprises a microorganism targeting molecule joined to at least a portion of a biocide molecule, wherein the microorganism targeting molecule binds to a pathogen or spoilage organism; and an object suspected of being contaminated with a pathogen or spoilage organism; and applying the recombinant fusion protein to the object under conditions such that the recombinant fusion protein neutralizes the pathogen or spoilage organism suspected of contaminating the surface. The object may be food processing equipment, military equipment (e.g., tanks), personal protective gear, medical devices, building structures (e.g., heating and ventilation equipment, walls, wall cavities, plumbing systems) surfaces in a household, or household equipment (e.g., appliances, sinks, toilet bowls, bathroom tiles, bath tubs, or showers).

Described herein is a method of treating an animal carcass or part thereof comprising: providing a recombinant fusion protein comprising microorganism targeting molecule joined to at least a portion of a biocide molecule, wherein the microorganism targeting molecule binds to a foodborne pathogen; and an animal carcass suspected of being contaminated with a pathogen; and applying the recombinant fusion protein to the animal carcass or part thereof under conditions such that the recombinant fusion protein neutralizes the pathogen suspected of contaminating the animal carcass. The animal carcass may comprise a bovine carcass or part thereof. The animal carcass may comprise a porcine carcass or part thereof. The animal carcass may comprise an avian carcass or part thereof. The animal carcass may comprise an aquatic animal carcass or part thereof.

Described herein is a method of treating a food product (e.g., a meat product, a processed meat, a dairy product, wine, beer, animal feed or a processing component thereof) comprising: providing a recombinant fusion protein comprising microorganism targeting molecule joined to at least a portion of a biocide molecule, wherein the microbial targeting molecule binds to a foodborne pathogen or a spoilage organism; and a food product suspected of being contaminated with a pathogen or spoilage organism; and applying the recombinant fusion protein to the food product under conditions such that the recombinant fusion protein neutralizes the pathogen or spoilage organism suspected of contaminating the food product.

Described herein is a method of treating a subject comprising: providing a recombinant fusion protein comprising a microorganism targeting molecule joined to at least a portion of a biocide molecule, wherein the microorganism targeting molecule binds to a pathogen; and a subject suspected of being contaminated or infected with a pathogen; applying the recombinant fusion protein to the subject under conditions such that the recombinant fusion protein neutralizes the pathogen suspected of contaminating or infecting the subject. The subject can be a mammal (*e.g.*, a ruminant (*e.g.*, bovine) or a human. The subject can be an avian species. The subject can be a plant. The subject may be contaminated with or infected with an antibiotic resistance organism or an artificially engineered organism (e.g., a bioterrorism agent). The subject may be deceased.

Described herein is a method of supplementing a food ration comprising: providing a recombinant fusion protein comprising a microorganism targeting molecule joined to at least a portion of a biocide molecule by a poly amino acid linker molecule, wherein the immunoglobulin binds to a foodborne pathogen; and a food ration; and supplementing the food ration with the recombinant fusion protein.

Further described herein is a food comprising a recombinant fusion protein comprising a microorganism targeting molecule joined to at least a portion of a biocide molecule, wherein the immunoglobulin binds to a foodborne pathogen and at least one foodstuff. The food may be for humans. The food may be for a farm animal or a companion animal (*e.g*. a horse, dog, or cat).

Described herein is a composition comprising a milk protein and a recombinant fusion protein wherein the protein comprises a microorganism targeting molecule joined to at least a portion of a protein biocide molecule, wherein the immunoglobulin binds to an infectious microorganism.

Described herein is a method of treating a plant or a part of a plant, comprising: providing a recombinant fusion protein wherein the protein comprises a microorganism targeting molecule joined to at least a portion of a protein biocide molecule, wherein the microorganism targeting molecule binds to a microorganism; and a plant or plant part (e.g., a seed, a growing plant, a fruit, a vegetable, a root, a stem, a leaf, an agricultural crop plant, a horticultural plant or an ornamental plant) suspected of being infected or contaminated by a microorganism; and applying the recombinant fusion protein to the plant or plant part under conditions such that the recombinant fusion protein neutralizes the microorganism. The microorganism may be an agricultural bioterrorism agent.

Described herein is a transgenic non-human organism (e.g., an animal, a plant, or a microorganism) comprising a nucleic acid sequence encoding a microorganism targeting molecule linked to at least a portion of a protein biocide molecule.

### DESCRIPTION OF THE FIGURES

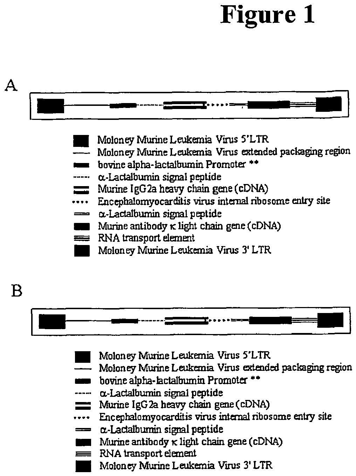

Figures which are not covered by the claims are used as referential examples which are useful for understanding the invention and are not embodiments of the invention. Fig. 1 shows one retrovector construct.

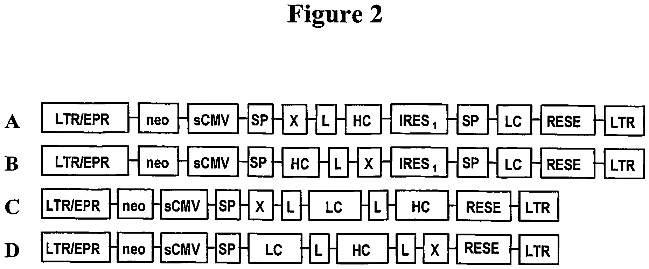

Figures 2A-2D show various retrovector elements used for production in mammalian cell culture of certain biocide fusions. Figure 2A shows a full size antibody with biocide linked to the N-terminus of the heavy chain. Figure 2B shows a full size antibody with biocide linked to the C-terminus of the heavy chain. Figure 2C shows a single chain antibody with biocide linked to the N-terminus of the light chain. Figure 2D shows a single chain antibody with biocide linked to the C-terminus of the heavy chain. In Figures 2A-2D abbreviations used are as follows: LTR, long terminal repeat; EPR, extended packaging region; neo, neomycin selection marker; sCMV, simian cytomegalovirus; SP, signal peptide; X, biocide; L, (G4S)3-4 linker; HC, antibody heavy chain; IRES1, internal ribosome entry site from encephalomyocarditis virus; LC, antibody light chain; and RESE (RNA stabilization element).
Figure 3 shows PLA2 neutralization of *C*. *parvum*.
Figure 4A shows retrovector elements used for mammalian cell culture production of recombinant 3E2 IgM antibody as a hexamer. Figure 4B shows retrovector gene construct used for GPEX production of recombinant 3E2 IgM antibody as a pentamer with J-chain. Figure 4C shows C a retrovector construct used for transgenic production of recombinant 3E2 IgM antibody as a hexamer. Figure 4D, shows a retrovector gene construct used for transgenic production of recombinant 3E2 IgM antibody as a pentamer with J-chain
Figures 5A-5D show retrovector elements used for mammalian cell culture production of biocide fusion proteins described herein. Figure 5A shows a full size antibody with biocide linked to the N-terminus of the heavy chain. Figure 5B shows a full size antibody with biocide linked to the C-terminus of the heavy chain. Figure 5C shows a single chain antibody with biocide linked to the N-terminus of the light chain. Figure 5D shows a single chain antibody with biocide linked to the C-terminus of the heavy chain.
Figure 6 shows the components of constructs described herein featuring a (Gly₄Ser)₃ linker.
Figure 7 shows the components of constructs described herein that contain an immunoglobulin and a biocide.

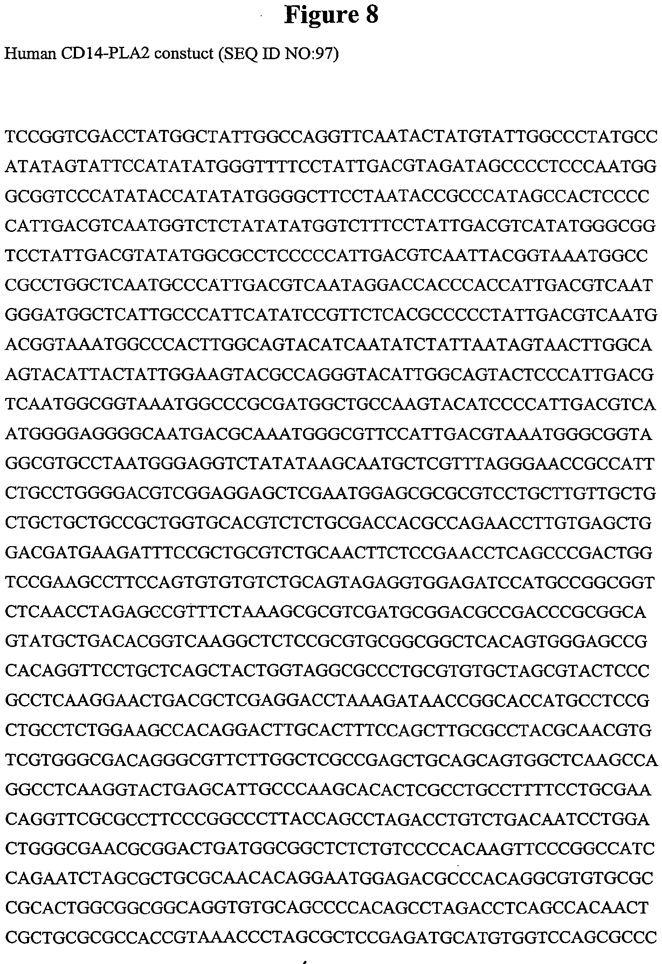

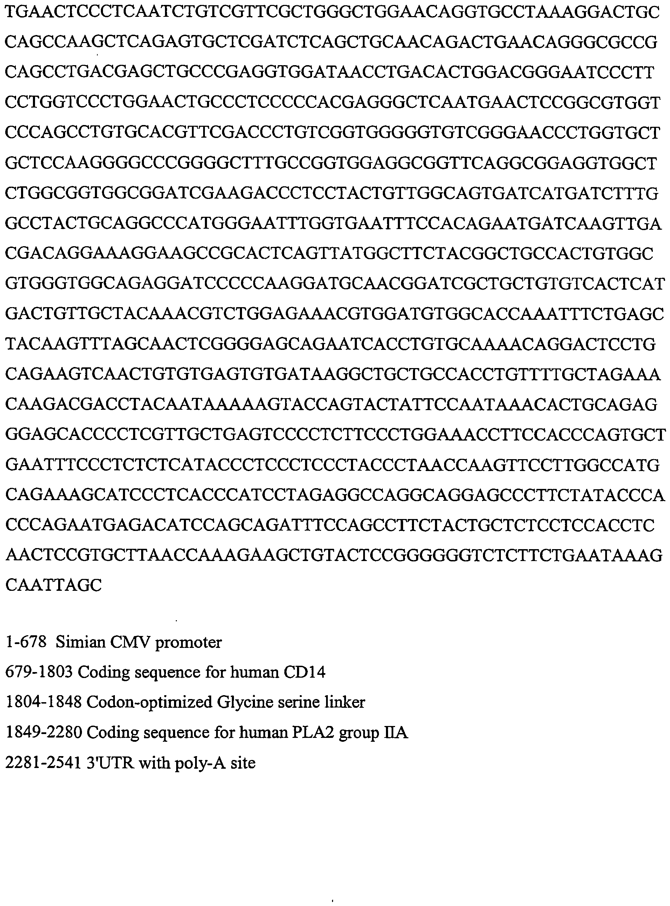

Figure 8 shows an exemplary Human CD14-PLA2 construct of the present invention (SEQ ID NO:97).

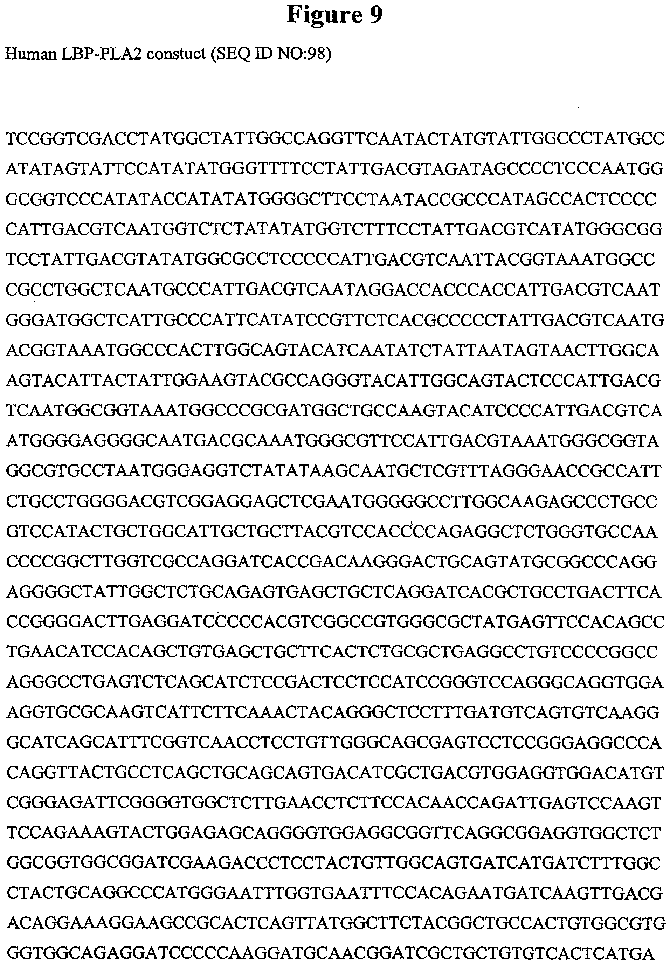

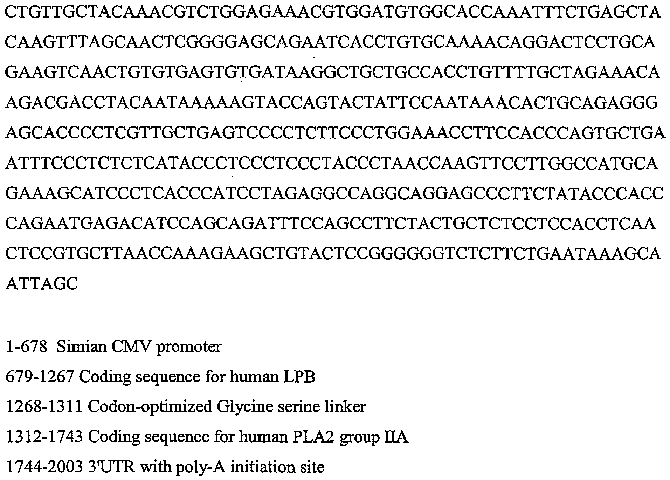

Figure 9 shows an exemplary Human LBP-PLA2 construct described herein (SEQ ID NO:98).

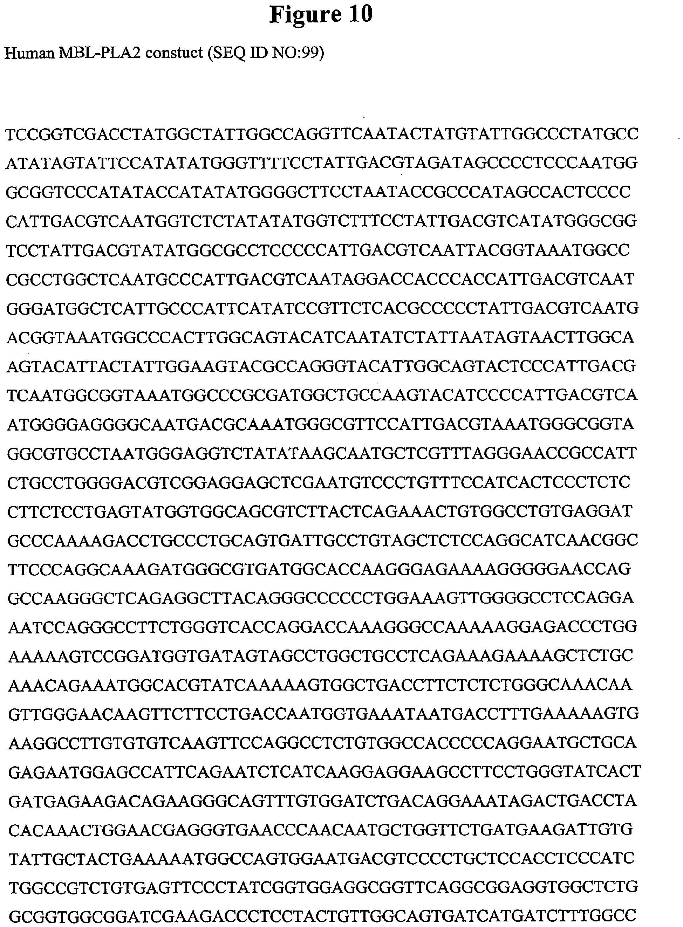

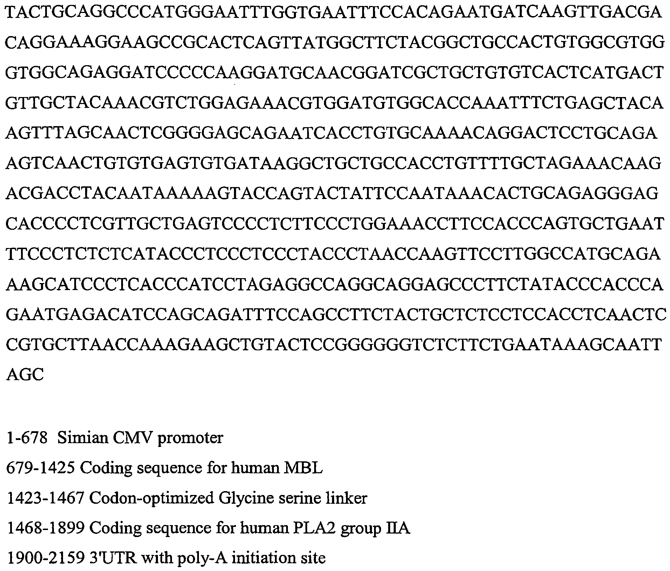

Figure 10 shows an exemplary Human MBL-PLA2 construct described herein (SEQ ID NO:99).

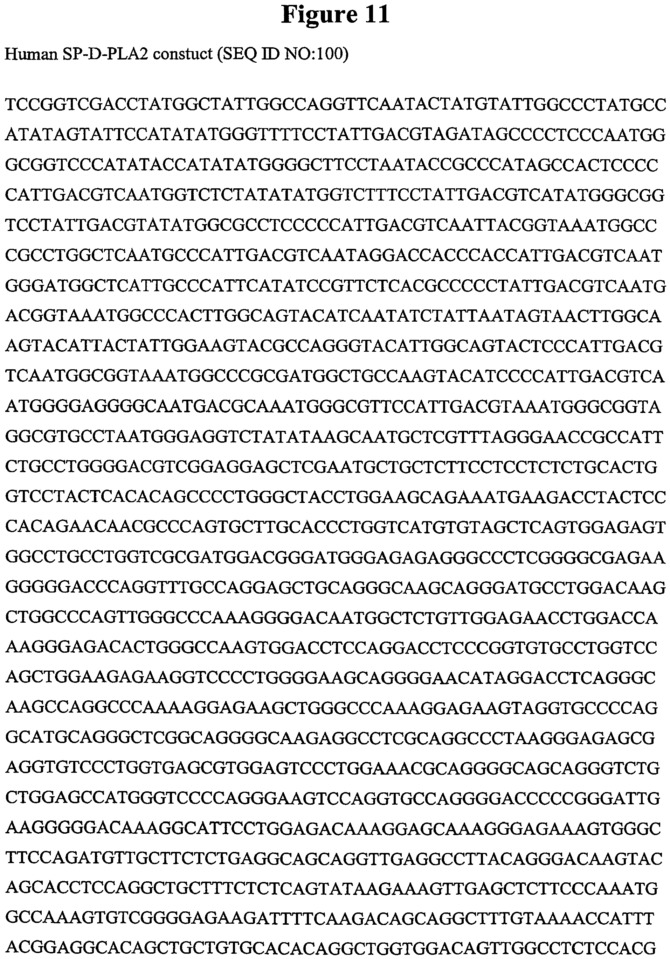

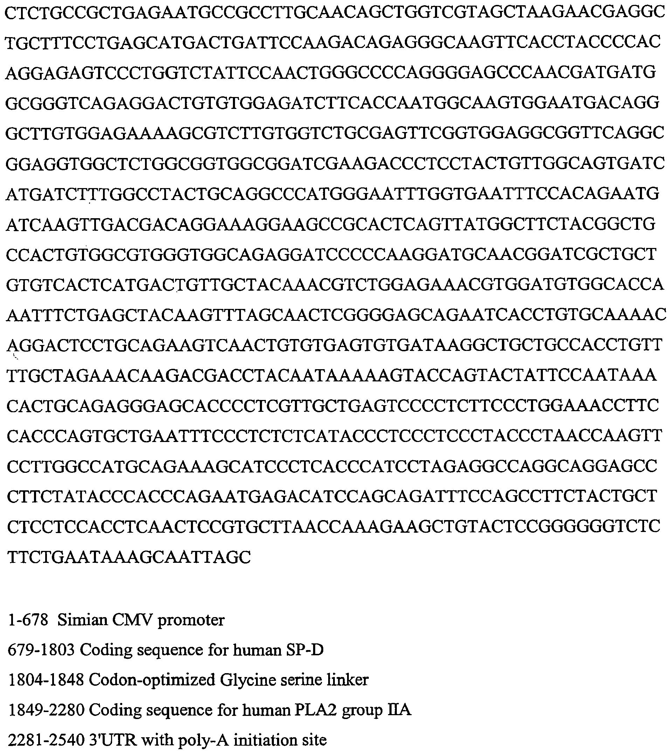

Figure 11 shows an exemplary Human SP-D-PLA2 construct described herein (SEQ ID NO: 100).

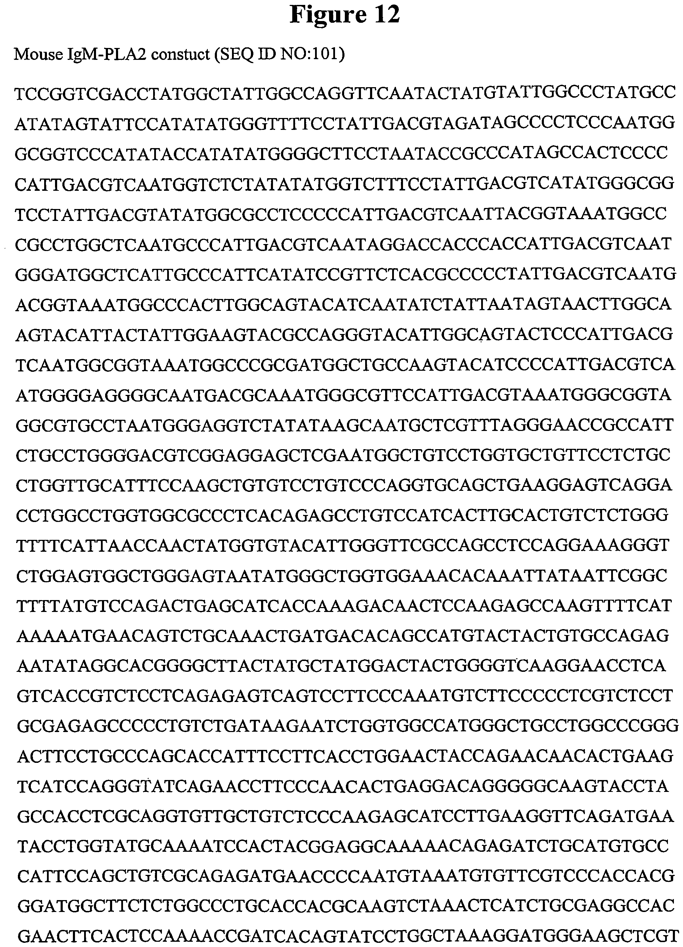

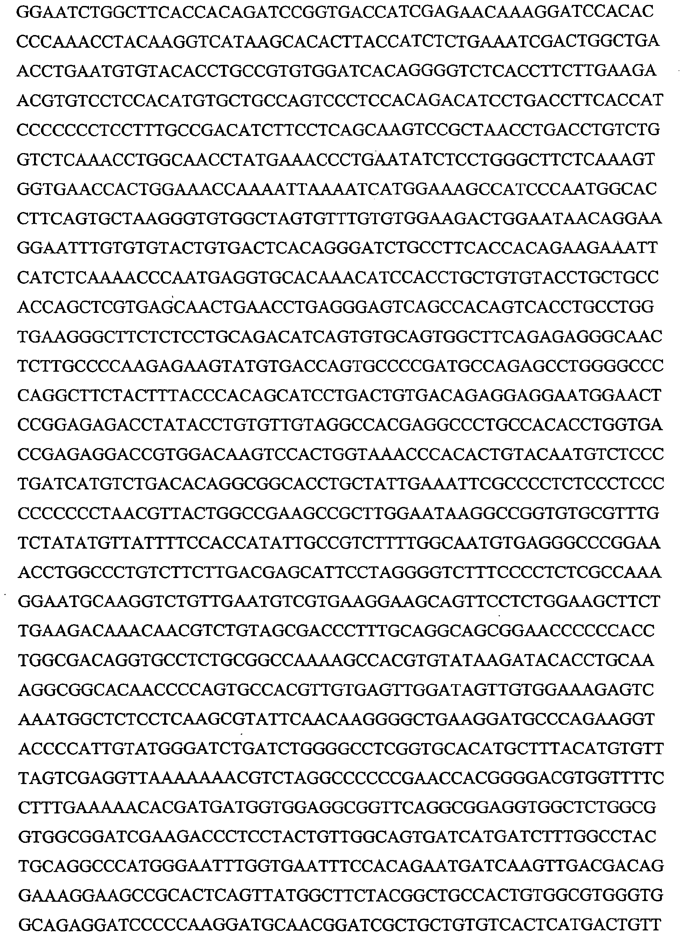

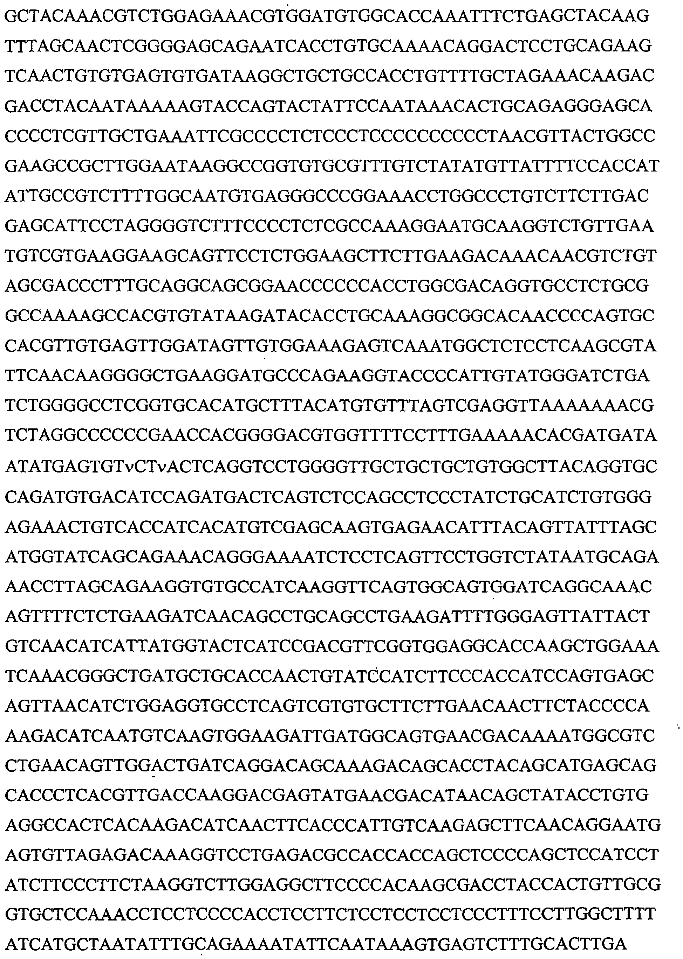

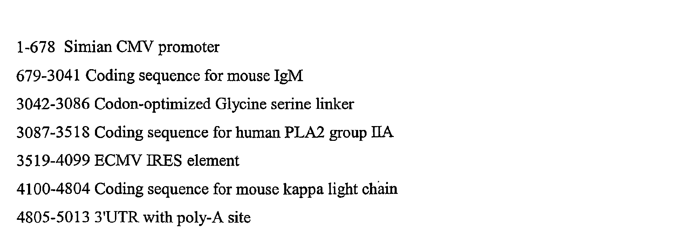

Figure 12 shows an exemplary Mouse IgM-PLA2 construct described herein (SEQ ID NO:101).

### DEFINITIONS

To facilitate an understanding of the present invention, a number of terms and phrases are defined below:

As used herein, the terms "biocide" or "biocides" refer to at least a portion of a naturally occurring or synthetic molecule (e.g., peptides) that directly kills or promotes the death and/or attenuation of (e.g., prevents growth and/or replication) of biological targets (e.g., bacteria, parasites, yeast, viruses, fungi, protozoans and the like). Examples of biocides include, but are not limited to, bactericides, viricides, fungicides, parasiticides, and the like.

As used herein, the terms "protein biocide" and "protein biocides" refer to at least a portion of a naturally occurring or synthetic peptide molecule that directly kills or promotes the death and/or attenuation of (*e.g.*, prevents growth and/or replication) of biological targets (*e.g.*, bacteria, parasites, yeast, viruses, fungi, protozoans and the like). Examples of biocides include, but are not limited to, bactericides, viricides, fungicides, parasiticides, and the like.

As used herein, the term "neutralization," "pathogen neutralization," "and spoilage organism neutralization" refer to destruction or inactivation (*e.g.*, loss of virulence) of a "pathogen" or "spoilage organism" (*e.g.*, bacterium, parasite, virus, fungus, mold, prion, and the like) thus preventing the pathogen's or spoilage organism's ability to initiate a disease state in a subject or cause degradation of a food product.

As used herein, the term "spoilage organism" refers to microorganisms (e.g., bacteria or fungi), which cause degradation of the nutritional or organoleptic quality of food and reduces its economic value and shelf life. Exemplary food spoilage microorganisms include, but are not limited to, Zygosaccharomyces bailii, Aspergillus niger, Saccharomyces cerivisiae, *Lactobacillus plantarum, Streptococcus faecalis, and Leuconostoc mesenteroides.*

As used herein, the term "microorganism targeting molecule" refers to any molecule (e.g., protein) that interacts with a microorganism. The microorganism targeting molecule may specifically interact with microorganisms at the exclusion of non- microorganism host cells. Preferred microorganism targeting molecules interact with broad classes of microorganism (e.g., all bacteria or all gram positive or negative bacteria). Described herein are also microorganism targeting molecules that interact with a specific species or sub-species of microorganism. The microorganism targeting molecules may interact with "Pathogen Associated Molecular Patterns (PAMPS)". The microorganism targeting molecules may be recognition molecules that are known to interact with or bind to PAMPS (*e.g.*, CD14,). The microorganism targeting molecules may be antibodies (*e.g.*, monoclonal antibodies directed towards PAMPS or monoclonal antibodies directed to specific organisms or serotype specific epitopes).

As used herein the term "biofilm" refers to an aggregation of microorganisms (e.g., bacteria) surrounded by an extracellular matrix or slime adherent on a surface *in vivo* or *ex vivo*, wherein the microorganisms adopt altered metabolic states.

As used herein, the term "host cell" refers to any eukaryotic cell (*e.g.*, mammalian cells, avian cells, amphibian cells, plant cells, fish cells, insect cells, yeast cells, and bacteria cells, and the like), whether located *in vitro* or *in vivo* (*e.g.,* in a transgenic organism).

As used herein, the term "cell culture" refers to any *in vitro* culture of cells. Included within this term are continuous cell lines (*e.g.*, with an immortal phenotype), primary cell cultures, finite cell lines (*e.g.*, non-transformed cells), and any other cell population maintained *in vitro,* including oocytes and embryos.

As used herein, the term "vector" refers to any genetic element, such as a plasmid, phage, transposon, cosmid, chromosome, retrovirus, virion, *etc.,* which is capable of replication when associated with the proper control elements and which can transfer gene sequences between cells. Thus, the term includes cloning and expression vehicles, as well as viral vectors.

As used herein, the term "multiplicity of infection" or "MOI" refers to the ratio of integrating vectors: host cells used during transfection or infection of host cells. For example, if 1,000,000 vectors are used to transfect 100,000 host cells, the multiplicity of infection is 10. The use of this term is not limited to events involving infection, but instead encompasses introduction of a vector into a host by methods such as lipofection, microinjection, calcium phosphate precipitation, and electroporation.

As used herein, the term "genome" refers to the genetic material (*e.g.*, chromosomes) of an organism or a host cell.

The term "nucleotide sequence of interest" refers to any nucleotide sequence (*e.g*., RNA or DNA), the manipulation of which may be deemed desirable for any reason (*e.g.*, treat disease, confer improved qualities, *etc*.), by one of ordinary skill in the art. Such nucleotide sequences include, but are not limited to, coding sequences, or portions thereof, of structural genes (*e.g.*, reporter genes, selection marker genes, oncogenes, drug resistance genes, growth factors, etc.), and non-coding regulatory sequences that do not encode an mRNA or protein product (e.g., promoter sequence, polyadenylation sequence, termination sequence, enhancer sequence, etc.).

The term "gene" refers to a nucleic acid (e.g., DNA or RNA) sequence that comprises coding sequences necessary for the production of a polypeptide or precursor (e.g., proinsulin). The polypeptide can be encoded by a full length coding sequence or by any portion of the coding sequence so long as the desired activity or functional properties (e.g., enzymatic activity, ligand binding, signal transduction, etc.) of the full-length or fragment are retained. The term also encompasses the coding region of a structural gene and includes sequences located adjacent to the coding region on both the 5' and 3' ends for a distance of about 1 kb or more on either end such that the gene corresponds to the length of the full-length mRNA. The sequences that are located 5' of the coding region and which are present on the mRNA are referred to as 5' untranslated sequences. The sequences that are located 3' or downstream of the coding region and which are present on the mRNA are referred to as 3' untranslated sequences. The term "gene" encompasses both cDNA and genomic forms of a gene. A genomic form or clone of a gene contains the coding region interrupted with non-coding sequences termed "introns" or "intervening regions" or "intervening sequences." Introns are segments of a gene that are transcribed into nuclear RNA (hnRNA); introns may contain regulatory elements such as enhancers. Introns are removed or "spliced out" from the nuclear or primary transcript; introns therefore are absent in the messenger RNA (mRNA) transcript. The mRNA functions during translation to specify the sequence or order of amino acids in a nascent polypeptide.

DNA molecules (*e.g.*, genes) are said to have "5' ends" and "3' ends" because mononucleotides are reacted to make oligonucleotides in a manner such that the 5' phosphate of one mononucleotide pentose ring is attached to the 3' oxygen of its neighbor in one direction via a phosphodiester linkage. Therefore, an end of an oligonucleotide is referred to as the "5' end" if its 5' phosphate is not linked to the 3' oxygen of a mononucleotide pentose ring. An end of an oligonucleotide is referred to as the "3' end" if its 3' oxygen is not linked to a 5' phosphate of another mononucleotide pentose ring. As used herein, a nucleic acid sequence, even if internal to a larger oligonucleotide, also may be said to have 5' and 3' ends. In either a linear or circular DNA molecule, discrete elements are referred to as being "upstream" or 5' of the "downstream" or 3' elements. This terminology reflects the fact that transcription proceeds in a 5' to 3' fashion along the DNA strand. The promoter and enhancer elements which direct the transcription of a linked gene are generally located 5' or upstream of the coding region. However, enhancer elements can exert their effect even when located 3' of the promoter element and the coding region. Transcription termination and polyadenylation signals are located 3' or downstream of the coding region.

As used herein, the term "exogenous gene" refers to a gene that is not naturally present in a host organism or cell, or is artificially introduced into a host organism or cell.

As used herein, the term "transgene" means a nucleic acid sequence (e.g., encoding one or more fusion protein polypeptides), which is introduced into the genome of a transgenic organism. A transgene can include one or more transcriptional regulatory sequences and other nucleic acid, such as introns, that may be necessary for optimal expression and secretion of a nucleic acid encoding the fusion protein. A transgene can include an enhancer sequence. A fusion protein sequence can be operatively linked to a tissue specific promoter, *e.g.*, mammary gland specific promoter sequence that results in the secretion of the protein in the milk of a transgenic mammal, a urine specific promoter, or an egg specific promoter.

As used herein, the term "transgenic cell" refers to a cell containing a transgene.

A "transgenic organism," as used herein, refers to a transgenic animal or plant.

As used herein, a "transgenic animal" is a non-human animal in which one or more, and preferably essentially all, of the cells of the animal contain a transgene introduced by way of human intervention, such as by transgenic techniques known in the art. The transgene can be introduced into the cell, directly or indirectly by introduction into a precursor of the cell, by way of deliberate genetic manipulation, such as by microinjection or by infection with a recombinant virus.

Mammals are defined herein as all animals, excluding humans, which have mammary glands and produce milk.

As used herein, a "dairy animal" refers to a milk producing non-human mammal that is larger than a laboratory rodent (*e.g.*, a mouse). The dairy animals may produce large volumes of milk and have long lactating periods (*e.g.*, cows or goats).

As used herein, the term "plant" refers to either a whole plant, a plant part, a plant cell, or a group of plant cells, including plants that are actively growing (e.g. in soil) and those that have been harvested. The class of plants used in methods of the invention is generally as broad as the class of higher plants amenable to transformation techniques, including both monocotyledonous and dicotyledonous plants. It includes plants of a variety of ploidy levels, including polyploid, diploid and haploid.

As used herein, a "transgenic plant" is a plant, preferably a multi-celled or higher plant, in which one or more, and preferably essentially all, of the cells of the plant contain a transgene introduced by way of human intervention, such as by transgenic techniques known in the art.

As used herein, the term "gene expression" refers to the process of converting genetic information encoded in a gene into RNA (*e.g.*, mRNA, rRNA, tRNA, or snRNA) through "transcription" of the gene (*i.e.*, via the enzymatic action of an RNA polymerase), and for protein encoding genes, into protein through "translation" of mRNA. Gene expression can be regulated at many stages in the process. "Up-regulation" or "activation" refers to regulation that increases the production of gene expression products (*i.e.*, RNA or protein), while "down-regulation" or "repression" refers to regulation that decrease production. Molecules (*e.g.*, transcription factors) that are involved in up-regulation or down-regulation are often called "activators" and "repressors," respectively.

As used herein, the term "protein of interest" refers to a protein encoded by a nucleic acid of interest.

As used herein, the term "native" (or wild type) when used in reference to a protein refers to proteins encoded by partially homologous nucleic acids so that the amino acid sequence of the proteins varies. As used herein, the term "variant" encompasses proteins encoded by homologous genes having both conservative and nonconservative amino acid substitutions that do not result in a change in protein function, as well as proteins encoded by homologous genes having amino acid substitutions that cause decreased (*e.g.*, null mutations) protein function or increased protein function.

The term "isolated" when used in relation to a nucleic acid, as in "an isolated oligonucleotide" refers to a nucleic acid sequence that is identified and separated from at least one contaminant nucleic acid with which it is ordinarily associated in its natural source. Isolated nucleic acids are nucleic acids present in a form or setting that is different from that in which they are found in nature. In contrast, non-isolated nucleic acids are nucleic acids such as DNA and RNA that are found in the state in which they exist in nature.

As used herein, the terms "nucleic acid molecule encoding," "DNA sequence encoding," "DNA encoding," "RNA sequence encoding," and "RNA encoding" refer to the order or sequence of deoxyribonucleotides or ribonucleotides along a strand of deoxyribonucleic acid or ribonucleic acid. The order of these deoxyribonucleotides or ribonucleotides determines the order of amino acids along the polypeptide (protein) chain translated from the mRNA. The DNA or RNA sequence thus codes for the amino acid sequence.

As used herein, the terms "complementary" or "complementarity" are used in reference to polynucleotides (*i.e.*, a sequence of nucleotides) related by the base-pairing rules. For example, for the sequence "A-G-T," is complementary to the sequence "T-C-A." Complementarity may be "partial," in which only some of the nucleic acids' bases are matched according to the base pairing rules. Or, there may be "complete" or "total" complementarity between the nucleic acids. The degree of complementarity between nucleic acid strands has significant effects on the efficiency and strength of hybridization between nucleic acid strands. This is of particular importance in amplification reactions, as well as detection methods that depend upon binding between nucleic acids.

The terms "homology" and "percent identity" when used in relation to nucleic acids refers to a degree of complementarity. There may be partial homology (*i.e.*, partial identity) or complete homology (*i.e*., complete identity). A partially complementary sequence is one that at least partially inhibits a completely complementary sequence from hybridizing to a target nucleic acid sequence and is referred to using the functional term "substantially homologous." The inhibition of hybridization of the completely complementary sequence to the target sequence may be examined using a hybridization assay (Southern or Northern blot, solution hybridization and the like) under conditions of low stringency. A substantially homologous sequence or probe (*i.e.*, an oligonucleotide which is capable of hybridizing to another oligonucleotide of interest) will compete for and inhibit the binding (*i.e*., the hybridization) of a completely homologous sequence to a target sequence under conditions of low stringency. This is not to say that conditions of low stringency are such that nonspecific binding is permitted; low stringency conditions require that the binding of two sequences to one another be a specific (*i.e.*, selective) interaction. The absence of nonspecific binding may be tested by the use of a second target which lacks even a partial degree of complementarity (*e.g.*, less than about 30% identity); in the absence of nonspecific binding the probe will not hybridize to the second non-complementary target.

The art knows well that numerous equivalent conditions may be employed to comprise low stringency conditions; factors such as the length and nature (DNA, RNA, base composition) of the probe and nature of the target (DNA, RNA, base composition, present in solution or immobilized, *etc.*) and the concentration of the salts and other components (*e.g.*, the presence or absence of formamide, dextran sulfate, polyethylene glycol) are considered and the hybridization solution may be varied to generate conditions of low stringency hybridization different from, but equivalent to, the above listed conditions. In addition, the art knows conditions that promote hybridization under conditions of high stringency (*e.g.*, increasing the temperature of the hybridization and/or wash steps, the use of formamide in the hybridization solution, *etc.*).

When used in reference to a double-stranded nucleic acid sequence such as a cDNA or genomic clone, the term "substantially homologous" refers to any probe that can hybridize to either or both strands of the double-stranded nucleic acid sequence under conditions of low stringency as described above.

When used in reference to a single-stranded nucleic acid sequence, the term "substantially homologous" refers to any probe that can hybridize (*i.e*., it is the complement of) the single-stranded nucleic acid sequence under conditions of low stringency as described above.

As used herein, the term "hybridization" is used in reference to the pairing of complementary nucleic acids. Hybridization and the strength of hybridization (*i.e.*, the strength of the association between the nucleic acids) is impacted by such factors as the degree of complementary between the nucleic acids, stringency of the conditions involved, the Tₘ of the formed hybrid, and the G:C ratio within the nucleic acids. A single molecule that contains pairing of complementary nucleic acids within its structure is said to be "self-hybridized."

As used herein, the term "Tₘ" is used in reference to the "melting temperature" of a nucleic acid. The melting temperature is the temperature at which a population of double-stranded nucleic acid molecules becomes half dissociated into single strands. The equation for calculating the Tₘ of nucleic acids is well known in the art. As indicated by standard references, a simple estimate of the Tₘ value may be calculated by the equation: Tₘ = 81.5 + 0.41 (% G + C), when a nucleic acid is in aqueous solution at 1 M NaCl *(See e.g.,* Anderson and Young, Quantitative Filter Hybridization, in Nucleic Acid Hybridization [1985]). Other references include more sophisticated computations that take structural as well as sequence characteristics into account for the calculation of Tₘ.

As used herein the term "stringency" is used in reference to the conditions of temperature, ionic strength, and the presence of other compounds such as organic solvents, under which nucleic acid hybridizations are conducted. With "high stringency" conditions, nucleic acid base pairing will occur only between nucleic acid fragments that have a high frequency of complementary base sequences. Thus, conditions of "weak" or "low" stringency are often required with nucleic acids that are derived from organisms that are genetically diverse, as the frequency of complementary sequences is usually less.

"High stringency conditions" when used in reference to nucleic acid hybridization comprise conditions equivalent to binding or hybridization at 42°C in a solution consisting of 5X SSPE (43.8 g/l NaCl, 6.9 g/l NaH₂PO₄ H₂O and 1.85 g/l EDTA, pH adjusted to 7.4 with NaOH), 0.5% SDS, 5X Denhardt's reagent and 100 µg/ml denatured salmon sperm DNA followed by washing in a solution comprising 0.1X SSPE, 1.0% SDS at 42°C when a probe of about 500 nucleotides in length is employed.

"Medium stringency conditions" when used in reference to nucleic acid hybridization comprise conditions equivalent to binding or hybridization at 42°C in a solution consisting of 5X SSPE (43.8 g/l NaCl, 6.9 g/l NaH₂PO H₂O and 1.85 g/l EDTA, pH adjusted to 7.4 with NaOH), 0.5% SDS, 5X Denhardt's reagent and 100 µg/ml denatured salmon sperm DNA followed by washing in a solution comprising 1.0X SSPE, 1.0% SDS at 42°C when a probe of about 500 nucleotides in length is employed.

"Low stringency conditions" comprise conditions equivalent to binding or hybridization at 42°C in a solution consisting of 5X SSPE (43.8 g/l NaCl, 6.9 g/l NaH₂PO₄ H₂O and 1.85 g/l EDTA, pH adjusted to 7.4 with NaOH), 0.1% SDS, 5X Denhardt's reagent [50X Denhardt's contains per 500 ml: 5 g Ficoll (Type 400, Pharamcia), 5 g BSA (Fraction V; Sigma)] and 100 µg/ml denatured salmon sperm DNA followed by washing in a solution comprising 5X SSPE, 0.1% SDS at 42°C when a probe of about 500 nucleotides in length is employed.

A gene may produce multiple RNA species that are generated by differential splicing of the primary RNA transcript. cDNAs that are splice variants of the same gene will contain regions of sequence identity or complete homology (representing the presence of the same exon or portion of the same exon on both cDNAs) and regions of complete non-identity (for example, representing the presence of exon "A" on cDNA 1 wherein cDNA 2 contains exon "B" instead). Because the two cDNAs contain regions of sequence identity they will both hybridize to a probe derived from the entire gene or portions of the gene containing sequences found on both cDNAs; the two splice variants are therefore substantially homologous to such a probe and to each other.

The terms "in operable combination," "in operable order," and "operably linked" as used herein refer to the linkage of nucleic acid sequences in such a manner that a nucleic acid molecule capable of directing the transcription of a given gene and/or the synthesis of a desired protein molecule is produced. The term also refers to the linkage of amino acid sequences in such a manner so that a functional protein is produced.

As used herein, the term "selectable marker" refers to a gene that encodes an enzymatic activity that confers the ability to grow in medium lacking what would otherwise be an essential nutrient (*e.g.*, the *HIS3* gene in yeast cells); in addition, a selectable marker may confer resistance to an antibiotic or drug upon the cell in which the selectable marker is expressed. Selectable markers may be "dominant"; a dominant selectable marker encoders an enzymatic activity that can be detected in any eukaryotic cell line. Examples of dominant selectable markers include, but are not limited to, the bacterial aminoglycoside 3' phosphotransferase gene (also referred to as the *neo* gene) that confers resistance to the drug G418 in mammalian cells, the bacterial hygromycin G phosphotransferase (*hyg*) gene that confers resistance to the antibiotic hygromycin and the bacterial xanthine-guanine phosphoribosyl transferase gene (also referred to as the gpt gene) that confers the ability to grow in the presence of mycophenolic acid. Other selectable markers are not dominant in that their use must be in conjunction with a cell line that lacks the relevant enzyme activity. Examples of non-dominant selectable markers include the thymidine kinase (*tk*) gene that is used in conjunction with *tk*⁻ cell lines, the CAD gene which is used in conjunction with CAD-deficient cells and the mammalian hypoxanthine-guanine phosphoribosyl transferase (*hprt*) gene which is used in conjunction with *hprt⁻* cell lines. A review of the use of selectable markers in mammalian cell lines is provided in Sambrook, J. et al., Molecular Cloning: A Laboratory Manual, 2nd ed., Cold Spring Harbor Laboratory Press, New York (1989) pp.16.9-16.15.

As used herein, the term "reporter gene" refers to a gene encoding a protein that may be assayed. Examples of reporter genes include, but are not limited to, luciferase (*See, e.g.,* deWet et al., Mol. Cell. Biol. 7:725 [1987] and US Pat Nos., 6,074,859; 5,976,796; 5,674,713; and 5,618,682; green fluorescent protein (*e.g.*, GenBank Accession Number U43284; a number of GFP variants are commercially available from CLONTECH Laboratories, Palo Alto, CA), chloramphenicol acetyltransferase, β-galactosidase, alkaline phosphatase, and horseradish peroxidase.

As used herein, the term "regulatory element" refers to a genetic element that controls some aspect of the expression of nucleic acid sequences. For example, a promoter is a regulatory element that facilitates the initiation of transcription of an operably linked coding region. Other regulatory elements are splicing signals, polyadenylation signals, termination signals, RNA export elements, internal ribosome entry sites, *etc.* (defined *infra*)*.*

Transcriptional control signals in eukaryotes comprise "promoter" and "enhancer" elements. Promoters and enhancers consist of short arrays of DNA sequences that interact specifically with cellular proteins involved in transcription (Maniatis et al., Science 236:1237 [1987]). Promoter and enhancer elements have been isolated from a variety of eukaryotic sources including genes in yeast, insect and mammalian cells, and viruses (analogous control elements, *i.e.,* promoters, are also found in prokaryotes). The selection of a particular promoter and enhancer depends on what cell type is to be used to express the protein of interest. Some eukaryotic promoters and enhancers have a broad host range while others are functional in a limited subset of cell types (for review *See e.g.,* Voss et al., Trends Biochem. Sci., 11:287 [1986]; and Maniatis *et al., supra*). For example, the SV40 early gene enhancer is very active in a wide variety of cell types from many mammalian species and has been widely used for the expression of proteins in mammalian cells (Dijkema et al., EMBO J. 4:761 [1985]). Two other examples of promoter/enhancer elements active in a broad range of mammalian cell types are those from the human elongation factor 1α gene (Uetsuki et al., J. Biol. Chem., 264:5791 [1989]; Kim et al., Gene 91:217 [1990]; and Mizushima and Nagata, Nuc. Acids. Res., 18:5322 [1990]) and the long terminal repeats of the Rous sarcoma virus (Gorman et al., Proc. Natl. Acad. Sci. USA 79:6777 [1982]) and the human cytomegalovirus (Boshart et al., Cell 41:521 [1985]). Inducible retroviral promoters may be utilized.

As used herein, the term "promoter/enhancer" denotes a segment of DNA which contains sequences capable of providing both promoter and enhancer functions (*i.e.*, the functions provided by a promoter element and an enhancer element, see above for a discussion of these functions). For example, the long terminal repeats of retroviruses contain both promoter and enhancer functions. The enhancer/promoter may be "endogenous" or "exogenous" or "heterologous." An "endogenous" enhancer/promoter is one that is naturally linked with a given gene in the genome. An "exogenous" or "heterologous" enhancer/promoter is one that is placed in juxtaposition to a gene by means of genetic manipulation (*i.e.*, molecular biological techniques such as cloning and recombination) such that transcription of that gene is directed by the linked enhancer/promoter.

Regulatory elements may be tissue specific or cell specific. The term "tissue specific" as it applies to a regulatory element refers to a regulatory element that is capable of directing selective expression of a nucleotide sequence of interest to a specific type of tissue (*e.g*., mammillary gland) in the relative absence of expression of the same nucleotide sequence(s) of interest in a different type of tissue (*e.g*., liver).

Tissue specificity of a regulatory element may be evaluated by, for example, operably linking a reporter gene to a promoter sequence (which is not tissue-specific) and to the regulatory element to generate a reporter construct, introducing the reporter construct into the genome of an animal such that the reporter construct is integrated into every tissue of the resulting transgenic animal, and detecting the expression of the reporter gene (*e.g.*, detecting mRNA, protein, or the activity of a protein encoded by the reporter gene) in different tissues of the transgenic animal. The detection of a greater level of expression of the reporter gene in one or more tissues relative to the level of expression of the reporter gene in other tissues shows that the regulatory element is "specific" for the tissues in which greater levels of expression are detected. Thus, the term "tissue-specific" (*e.g.*, liver-specific) as used herein is a relative term that does not require absolute specificity of expression. In other words, the term "tissue-specific" does not require that one tissue have extremely high levels of expression and another tissue have no expression. It is sufficient that expression is greater in one tissue than another. By contrast, "strict" or "absolute" tissue-specific expression is meant to indicate expression in a single tissue type (*e.g*., liver) with no detectable expression in other tissues.

The term "cell type specific" as applied to a regulatory element refers to a regulatory element which is capable of directing selective expression of a nucleotide sequence of interest in a specific type of cell in the relative absence of expression of the same nucleotide sequence of interest in a different type of cell within the same tissue (*e.g*., cells infected with retrovirus, and more particularly, cells infected with BLV or HTLV). The term "cell type specific" when applied to a regulatory element also means a regulatory element capable of promoting selective expression of a nucleotide sequence of interest in a region within a single tissue.

The cell type specificity of a regulatory element may be assessed using methods well known in the art (*e.g.*, immunohistochemical staining and/or Northern blot analysis). Briefly, for immunohistochemical staining, tissue sections are embedded in paraffin, and paraffin sections are reacted with a primary antibody specific for the polypeptide product encoded by the nucleotide sequence of interest whose expression is regulated by the regulatory element. A labeled (*e.g.*, peroxidase conjugated) secondary antibody specific for the primary antibody is allowed to bind to the sectioned tissue and specific binding detected (*e.g*., with avidin/biotin) by microscopy. Briefly, for Northern blot analysis, RNA is isolated from cells and electrophoresed on agarose gels to fractionate the RNA according to size followed by transfer of the RNA from the gel to a solid support (*e.g.*, nitrocellulose or a nylon membrane). The immobilized RNA is then probed with a labeled oligodeoxyribonucleotide probe or DNA probe to detect RNA species complementary to the probe used. Northern blots are a standard tool of molecular biologists.

The term "promoter," "promoter element," or "promoter sequence" as used herein, refers to a DNA sequence which when ligated to a nucleotide sequence of interest is capable of controlling the transcription of the nucleotide sequence of interest into mRNA. A promoter is typically, though not necessarily, located 5' (*i.e.,* upstream) of a nucleotide sequence of interest whose transcription into mRNA it controls, and provides a site for specific binding by RNA polymerase and other transcription factors for initiation of transcription.

Promoters may be constitutive or regulatable. The term "constitutive" when made in reference to a promoter means that the promoter is capable of directing transcription of an operably linked nucleic acid sequence in the absence of a stimulus (*e.g.*, heat shock, chemicals, *etc.*). In contrast, a "regulatable" promoter is one that is capable of directing a level of transcription of an operably linked nucleic acid sequence in the presence of a stimulus (*e.g.*, heat shock, chemicals, *etc*.), which is different from the level of transcription of the operably linked nucleic acid sequence in the absence of the stimulus.

The presence of "splicing signals" on an expression vector often results in higher levels of expression of the recombinant transcript. Splicing signals mediate the removal of introns from the primary RNA transcript and consist of a splice donor and acceptor site (Sambrook et al., Molecular Cloning: A Laboratory Manual, 2nd ed., Cold Spring Harbor Laboratory Press, New York [1989], pp. 16.7-16.8). A commonly used splice donor and acceptor site is the splice junction from the 16S RNA of SV40.

Efficient expression of recombinant DNA sequences in eukaryotic cells requires expression of signals directing the efficient termination and polyadenylation of the resulting transcript. Transcription termination signals are generally found downstream of the polyadenylation signal and are a few hundred nucleotides in length. The term "poly A site" or "poly A sequence" as used herein denotes a DNA sequence that directs both the termination and polyadenylation of the nascent RNA transcript. Efficient polyadenylation of the recombinant transcript is desirable as transcripts lacking a poly A tail are unstable and are rapidly degraded. The poly A signal utilized in an expression vector may be "heterologous" or "endogenous." An endogenous poly A signal is one that is found naturally at the 3' end of the coding region of a given gene in the genome. A heterologous poly A signal is one that is isolated from one gene and placed 3' of another gene. A commonly used heterologous poly A signal is the SV40 poly A signal. The SV40 poly A signal is contained on a 237 bp BamHI/BclI restriction fragment and directs both termination and polyadenylation (Sambrook, supra, at 16.6-16.7).

Eukaryotic expression vectors may also contain "viral replicons "or "viral origins of replication." Viral replicons are viral DNA sequences that allow for the extrachromosomal replication of a vector in a host cell expressing the appropriate replication factors. Vectors that contain either the SV40 or polyoma virus origin of replication replicate to high "copy number" (up to 104 copies/cell) in cells that express the appropriate viral T antigen. Vectors that contain the replicons from bovine papillomavirus or Epstein-Barr virus replicate extrachromosomally at "low copy number" (~100 copies/cell). However, it is not intended that expression vectors be limited to any particular viral origin of replication.

As used herein, the term "long terminal repeat" or "LTR" refers to transcriptional control elements located in or isolated from the U3 region 5' and 3' of a retroviral genome. As is known in the art, long terminal repeats may be used as control elements in retroviral vectors, or isolated from the retroviral genome and used to control expression from other types of vectors.

As used herein, the terms "RNA export element" or "Pre-mRNA Processing Enhancer (PPE)" refer to 3' and 5' cis-acting post-transcriptional regulatory elements that enhance export of RNA from the nucleus. "PPE" elements include, but are not limited to Mertz sequences (described in US 5,914,267 and 5,686,120, and woodchuck mRNA processing enhancer (WPRE; WO 99/14310,).

As used herein, the term "polycistronic" refers to an mRNA encoding more than one polypeptide chain (*See, e.g.,* WO 93/03143, WO 88/05486, and European Pat. No. 117058. Likewise, the term "arranged in polycistronic sequence" refers to the arrangement of genes encoding two different polypeptide chains in a single mRNA.

As used herein, the term "internal ribosome entry site" or "IRES" refers to a sequence located between polycistronic genes that permits the production of the expression product originating from the second gene by internal initiation of the translation of the dicistronic mRNA. Examples of internal ribosome entry sites include, but are not limited to, those derived from foot and mouth disease virus (FDV), encephalomyocarditis virus, poliovirus and RDV (Scheper et al., Biochem. 76: 801-809 [1994]; Meyer et al., J. Virol. 69: 2819-2824 [1995]; Jang et al., 1988, J. Virol. 62: 2636-2643 [1998]; Haller et al., J. Virol. 66: 5075-5086 [1995]). Vectors incorporating IRESs may be assembled as is known in the art. For example, a retroviral vector containing a polycistronic sequence may contain the following elements in operable association: nucleotide polylinker, gene of interest, an internal ribosome entry site and a mammalian selectable marker or another gene of interest. The polycistronic cassette is situated within the retroviral vector between the 5' LTR and the 3' LTR at a position such that transcription from the 5' LTR promoter transcribes the polycistronic message cassette. The transcription of the polycistronic message cassette may also be driven by an internal promoter (*e.g.*, cytomegalovirus promoter) or an inducible promoter (*e.g.*, the inducible promoters described herein), which may be preferable depending on the use. The polycistronic message cassette can further comprise a cDNA or genomic DNA (gDNA) sequence operatively associated within the polylinker. Any mammalian selectable marker can be utilized as the polycistronic message cassette mammalian selectable marker. Such mammalian selectable markers are well known to those of skill in the art and can include, but are not limited to, kanamycin/G418, hygromycin B or mycophenolic acid resistance markers.

As used herein, the term "retrovirus" refers to a retroviral particle which is capable of entering a cell (*i.e.,* the particle contains a membrane-associated protein such as an envelope protein or a viral G glycoprotein which can bind to the host cell surface and facilitate entry of the viral particle into the cytoplasm of the host cell) and integrating the retroviral genome (as a double-stranded provirus) into the genome of the host cell.

As used herein, the term "retroviral vector" refers to a retrovirus that has been modified to express a gene of interest. Retroviral vectors can be used to transfer genes efficiently into host cells by exploiting the viral infectious process. Foreign or heterologous genes cloned (*i.e*., inserted using molecular biological techniques) into the retroviral genome can be delivered efficiently to host cells that are susceptible to infection by the retrovirus. Through well-known genetic manipulations, the replicative capacity of the retroviral genome can be destroyed. The resulting replication-defective vectors can be used to introduce new genetic material to a cell but they are unable to replicate. A helper virus or packaging cell line can be used to permit vector particle assembly and egress from the cell. Such retroviral vectors comprise a replication-deficient retroviral genome containing a nucleic acid sequence encoding at least one gene of interest (*i.e.*, a polycistronic nucleic acid sequence can encode more than one gene of interest), a 5' retroviral long terminal repeat (5' LTR); and a 3' retroviral long terminal repeat (3' LTR).

The term "pseudotyped retroviral vector" refers to a retroviral vector containing a heterologous membrane protein. The term "membrane-associated protein" refers to a protein (*e.g.*, a viral envelope glycoprotein or the G proteins of viruses in the Rhabdoviridae family such as VSV, Piry, Chandipura and Mokola), which is associated with the membrane surrounding a viral particle; these membrane-associated proteins mediate the entry of the viral particle into the host cell. The membrane associated protein may bind to specific cell surface protein receptors, as is the case for retroviral envelope proteins or the membrane-associated protein may interact with a phospholipid component of the plasma membrane of the host cell, as is the case for the G proteins derived from members of the Rhabdoviridae family.

A "subject" is an animal such as vertebrate, preferably a mammal, more preferably a human or a bovine. Mammals, however, are understood to include, but are not limited to, murines, simians, humans, bovines, cervids, equines, porcines, canines, felines *etc*.).

An "effective amount" is an amount sufficient to effect beneficial or desired results. An effective amount can be administered in one or more administrations,

"Co-administration" refers to administration of more than one agent or therapy to a subject. Co-administration may be concurrent or, alternatively, the chemical compounds described herein may be administered in advance of or following the administration of the other agent(s). One skilled in the art can readily determine the appropriate dosage for co-administration. When co-administered with another therapeutic agent, both the agents may be used at lower dosages. Thus, co-administration is especially desirable where the claimed compounds are used to lower the requisite dosage of known toxic agents.

As used herein, the term "toxic" refers to any detrimental or harmful effects on a cell or tissue.

A "pharmaceutical composition" is intended to include the combination of an active agent with a carrier, inert or active, making the composition suitable for diagnostic or therapeutic use *in vivo, in vivo* or *ex vivo.*

As used herein, the term "pharmaceutically acceptable carrier" encompasses any of the standard pharmaceutical carriers, such as a phosphate buffered saline solution, water, and an emulsion, such as an oil/water or water/oil emulsion, and various types of wetting agents. The compositions also can include stabilizers and preservatives. For examples of carriers, stabilizers and adjuvants see Martin, Remington's Pharmaceutical Sciences, 15th Ed., Mack Publ. Co., Easton, PA (1975).

"Pharmaceutically acceptable salt" as used herein, relates to any pharmaceutically acceptable salt (acid or base) of a compound of the present invention, which, upon administration to a recipient, is capable of providing a compound of this invention or an active metabolite or residue thereof. As is known to those of skill in the art, "salts" of the compounds of the present invention may be derived from inorganic or organic acids and bases. Examples of acids include hydrochloric, hydrobromic, sulfuric, nitric, perchloric, fumaric, maleic, phosphoric, glycolic, lactic, salicylic, succinic, toluene-p-sulfonic, tartaric, acetic, citric, methanesulfonic, ethanesulfonic, formic, benzoic, malonic, naphthalene-2-sulfonic and benzenesulfonic acid. Other acids, such as oxalic, while not in themselves pharmaceutically acceptable, may be employed in the preparation of salts useful as intermediates in obtaining the compounds of the invention and their pharmaceutically acceptable acid.

As used herein, the term "nutraceutical," refers to a food substance or part of a food, which includes a fusion protein. Nutraceuticals can provide medical or health benefits, including the prevention, treatment, or cure of a disorder. The transgenic protein will often be present in the nutraceutical at concentration of at least 100 µg/kg, more preferably at least 1 mg/kg, most preferably at least 10 mg/kg. A nutraceutical can include the milk of a transgenic animal.

As used herein, the term "purified" or "to purify" refers to the removal of undesired components from a sample. As used herein, the term "substantially purified" refers to molecules, either nucleic or amino acid sequences, that are removed from their natural environment, isolated or separated, and are at least 60% free, preferably 75% free, and most preferably 90% free from other components with which they are naturally associated. An "isolated polynucleotide" is therefore a substantially purified polynucleotide.

The terms "bacteria" and "bacterium" refer to all prokaryotic organisms, including those within all of the phyla in the Kingdom Procaryotae. It is intended that the term encompass all microorganisms considered to be bacteria including Mycoplasma, Chlamydia, Actinomyces, Streptomyces, and Rickettsia. All forms of bacteria are included within this definition including cocci, bacilli, spirochetes, spheroplasts, protoplasts, *etc*. Also included within this term are prokaryotic organisms that are gram negative or gram positive. "Gram negative" and "gram positive" refer to staining patterns with the Gram-staining process that is well known in the art. *(See e.g.,* Finegold and Martin, Diagnostic Microbiology, 6th Ed., CV Mosby St. Louis, pp. 13-15 [1982]). "Gram positive bacteria" are bacteria that retain the primary dye used in the Gram stain, causing the stained cells to appear dark blue to purple under the microscope. "Gram negative bacteria" do not retain the primary dye used in the Gram stain, but are stained by the counterstain. Thus, gram negative bacteria appear red. In some embodiments, the bacteria are those capable of causing disease (pathogens) and those that cause product degradation or spoilage.

As used herein, the term "antigen binding protein" refers to proteins that bind to a specific antigen. "Antigen binding proteins" include, but are not limited to, immunoglobulins, including polyclonal, monoclonal, chimeric, single chain, and humanized antibodies, Fab fragments, F(ab')2 fragments, and Fab expression libraries. Various procedures known in the art are used for the production of polyclonal antibodies. For the production of antibody, various host animals can be immunized by injection with the peptide corresponding to the desired epitope including but not limited to rabbits, mice, rats, sheep, goats, *etc*. The peptide may be conjugated to an immunogenic carrier (*e.g.*, diphtheria toxoid, bovine serum albumin (BSA), or keyhole limpet hemocyanin (KLH)). Various adjuvants are used to increase the immunological response, depending on the host species, including but not limited to Freund's (complete and incomplete), mineral gels such as aluminum hydroxide, surface active substances such as lysolecithin, pluronic polyols, polyanions, peptides, oil emulsions, keyhole limpet hemocyanins, dinitrophenol, and potentially useful human adjuvants such as BCG (Bacille Calmette-Guerin) and Corynebacterium parvum.

For preparation of monoclonal antibodies, any technique that provides for the production of antibody molecules by continuous cell lines in culture may be used (*See e.g*., Harlow and Lane, Antibodies: A Laboratory Manual, Cold Spring Harbor Laboratory Press, Cold Spring Harbor, NY). These include, but are not limited to, the hybridoma technique originally developed by Köhler and Milstein (Köhler and Milstein, Nature, 256:495-497 [1975]), as well as the trioma technique, the human B-cell hybridoma technique (See e.g., Kozbor et al., Immunol. Today, 4:72 [1983]), and the EBV-hybridoma technique to produce human monoclonal antibodies (Cole et al., in Monoclonal Antibodies and Cancer Therapy, Alan R. Liss, Inc., pp. 77-96 [1985]). Suitable monoclonal antibodies, including recombinant chimeric monoclonal antibodies and chimeric monoclonal antibody fusion proteins are prepared as described herein.

Techniques described for the production of single chain antibodies (US 4,946,778 ) can be adapted to produce specific single chain antibodies as desired. Described herein is the use of the techniques known in the art for the construction of Fab expression libraries (Huse et al., Science, 246:1275-1281 [1989]) to allow rapid and easy identification of monoclonal Fab fragments with the desired specificity. Monoclonal antibodies can be generated using the ABL-MYC method (See e.g., U.S. Patent 5,705,150 and 5,244,656,) (Neoclone, Madison, WI). ABL-MYC is a recombinant retrovirus that constitutively expresses v-abl and c-myc oncogenes. When used to infect antigen-activated splenocytes, this retroviral system rapidly induces antigen-specific plasmacytomas. ABL-MYC targets antigen-stimulated (Ag-stimulated) B-cells for transformation.

Antibody fragments that contain the idiotype (antigen binding region) of the antibody molecule can be generated by known techniques. For example, such fragments include but are not limited to: the F(ab')2 fragment that can be produced by pepsin digestion of an antibody molecule; the Fab' fragments that can be generated by reducing the disulfide bridges of an F(ab')2 fragment, and the Fab fragments that can be generated by treating an antibody molecule with papain and a reducing agent

Genes encoding antigen-binding proteins can be isolated by methods known in the art. In the production of antibodies, screening for the desired antibody can be accomplished by techniques known in the art (*e.g.*, radioimmunoassay, ELISA (enzyme-linked immunosorbant assay), "sandwich" immunoassays, immunoradiometric assays, gel diffusion precipitin reactions, immunodiffusion assays, *in situ* immunoassays (using colloidal gold, enzyme or radioisotope labels, for example), Western Blots, precipitation reactions, agglutination assays (*e.g.*, gel agglutination assays, hemagglutination assays, *etc*.), complement fixation assays, immunofluorescence assays, protein A assays, and immunoelectrophoresis assays, *etc*.) *etc.*

### GENERAL DESCRIPTION OF THE INVENTION

The present invention relates to compositions and methods for treating and/or preventing illnesses in animals caused by pathogens. More particularly, the present invention relates to therapeutic and prophylactic compositions directed to combating bacterial, parasitical, and fungal infections in humans and other animals (*e.g.*, feedlot and domestic animals such as cows, chickens, turkeys, pigs, and sheep).

Described herein are fusion proteins comprising microorganism targeting molecules (e.g., innate immune system receptors) directed against bacterial, parasitic, and fungal pathogens and methods of using and creating these molecules. The antibodies can be chimeras (*e.g.*, murine-bovine).

Described herein are chimeric monoclonal antibodies directed against foodborne bacterial, protozoan and parasitic pathogens. However, the bacterial pathogens need not be foodborne (*e.g*., gastrointestinal). Therapeutic compositions and methods are described herein to combat other bacterial infections via other possible routes of transmission (*e.g*., respiratory, salivary, fecal-oral, skin-to-skin, bloodborne, genital, urinary, eye-to-eye, zoonotic, *etc*.). Moreover, described herein are chimeric monoclonal antibodies against viruses, prions, fungal, protozoan and other parasitic and pathogenic sources of illness.

Further described herein are chimeric recombinant monoclonal antibody fusion proteins. The fusion proteins may comprise one or more portions of an immunoglobulin and a portion of a biocide molecule, such as bactericides, viricides, fungicides, parasiticides, and the like. Described herein are antibody biocide fusion proteins, wherein the biocide component comprises a bactericidal enzyme such as human lysozyme, phospholipase A2 (groups I, II, V, X, and XII), lactoferrin, lactoperoxidase, and bacterial permeability increasing protein. Described herein are fusion proteins comprising immune system complement proteins including cytokines such as the interferons (*e.g*., IFN-α, IFN-β, and IFN-γ) and the tumor necrosis factors (*e.g.*, TNF-α, and TNF-β) and defensins. The antibody portion of these fusion proteins may bind specifically to a foodborne bacterial pathogen (*e.g., E. coli* 0157:H7, *Listeria monocytogenes, Campylobacter jejuni,* and the like).

Described herein are compositions comprising fusion proteins in an edible carrier such as whey protein. Preferred methods of using these compositions include, but are not limited to, food additives for human and animal (*e.g.*, bovines) consumption, carcass decontaminating compounds used during processing and finishing feedlot animal (e.g., bovine) carcasses and poultry, as well as pharmaceutical compositions for both human and veterinary medicine.

Suitable food additive formulations of the compositions described herein include, but are not limited to, compositions directly applied to food products such as processed meat slices and dairy products in the form of sprays, powders, injected solutions, coatings, gels, rinses, dips, films (*e.g*., bonded), extrusions, among other known formulations.

Further described herein are compositions (*e.g*., rinses, sprays, and the like) for sanitizing food-processing, medical, military or household equipment. For example, described herein are compositions for disinfecting meat-processing equipment. Described herein is that a number of food (*e.g.*, meat) processors will benefit from using the compositions and methods of the present invention in their operations. Compositions described herein may be provided to the entire range of meat processing operations from the largest commercial slaughterhouses to individual consumers.

Those skilled in the art will appreciate that the compositions disclosed herein can be readily formulated to include additional compounds common in the pharmaceutical arts such as, excipients, extenders, preservatives, and bulking agents depending on the intended use of a composition. Furthermore, ingestible formulations of these compositions may also comprise any material approved by the United States Department of Agriculture (USDA) for incorporation into food products such as substances that are generally recognized as safe (GRAS) including, food additives, flavorings, colorings, vitamins, minerals, and phytonutrients. The term phytonutrients as used herein, refers to organic compounds isolated from plants having biological effects including, but not limited to, compounds from the following classes of molecules: isoflavonoids, oligomeric proanthcyanidins, indol-3-carbinol, sulforaphone, fibrous ligands, plant phytosterols, ferulic acid, anthocyanocides, triterpenes, omega 3/6 fatty acids, polyacetylene, quinones, terpenes, cathechins, gallates, and quercitin.

The fusion proteins described herein may be purified from the lactations of transgenic non-human mammals such as, cows, pigs, sheep, and goats.

Further described herein are novel genetic constructs and methods of producing transgenic animals that express the compositions described herein in their lactation. Also described herein are methods of inducing transgenic animals (*e.g.*, bovines) to lactate upon maturation.

Described herein are methods of stably transfecting cell lines (*e.g*., mammalian, plant, insect, and amphibian) with encoding the fusion proteins disclosed herein. The constructs described herein may allow complex multicistronic gene constructs to be stably inserted into cells (*e.g.*, mammalian, bacteria, fungal cells, plant, *etc*). The production of fusion proteins in mammalian cell lines (or in transgenic mammals) allows for their proper assembly and processing. Another method described herein is protein production in mammalian tissue culture bioreactors.

Monoclonal antibodies are typically produced in mammalian cells to ensure correct processing, however mammalian tissue culture bioreactors are often expensive to operate thus placing products beyond mass applications. The ability to manufacture monoclonals in the milk of transgenic animals (*e.g.*, bovines) is contemplated to expand the scope of monoclonal antibodies typically from individual medicine to applications for large populations. Production of the disclosed compositions in the milk of transgenic mammals (*e.g.*, bovines) provides large quantities for economical distribution to food safety and processing operations. For instance, at reasonable expression levels of about one gram per liter of milk, a herd of 100 transgenic cows will produce about a metric ton of recombinant protein per year. This enables production of recombinant monoclonals at 100 fold less cost than in cell culture bioreactors. Described herein are methods of creating transgenic bovines that produce the compositions described herein in their lactation. Also described herein are methods of isolating and purifying the compositions described herein from the lactation of milk producing herd animals (*e.g.*, cows, sheep, and goats and the like).

Further described herein is a fusion protein enriched colostrum, or colostrum like products, for use as milk substitutes and nutritional supplements for nursing mammals and in particular for nursing feedlot animals. These compositions may comprise the microorganism targeting molecule fusion proteins described herein. Introducing these compositions to nursing feedlot animals will reduce the colonization of the animal's gastrointestinal tract by pathogenic organisms such as *E*. *coli* O157:H7 and *Listeria monocytogenes* and *Cryptosporidium parvum.* Furthermore, the compositions may be added to feeds to control diseases such as coccidiosis, which are common in both cattle and chicken feeding operations. In particular, providing a fusion protein enriched milk replacer or colostrum supplement reduces the load of *E*. *coli* O157:H7 in the gastrointestinal tract of the neonate and specifically places the targeted pathogenic organisms at a competitive disadvantage in relation to normal gastrointestinal flora. Inducing a protective immune response in animals fed the preset fusion protein enriched colostrums and colostrum-like compositions is described herein. An immune response in animals feed the present compositions may be induced.

Described herein are compositions and methods directed against foodborne pathogens such as, but not limited to, *E*. *coli* O157:H7, *Listeria monocytogenes, Campylobacter jejuni, Clostridium botulinum, Clostridium perfringens, Staphylococcus aureus, Staphylococcus epidermidis, Staphylococcus pneumoniae, Staphylococcus saprophyticus, Staphylococcus mutans, Shigella dysenteriae, Salmonella typhi, Salmonella paratyphi, Salmonella enteritidis, Cryptosporidium parvum,* fungi, and the like. Further described herein are composition and methods directed against food spoilage organisms such as, but not limited to, bacteria (e.g., *Lactobacillus, Leuconostoc, Pediococcus,* and *Streptococcus* and fungi (e.g., *Monilia, Trichoderma, Crinipellis, Moniliophthora, Phytophthora, Botrytis, and Fusarium).*

Also described herein are compositions and methods directed against protozoans, particularly, apicomplexan protozoans including, but not limited to coccidian, cryptosporidian, toxoplasman, malarian and trypanosomatid protozoans.

The compositions of the present invention comprise CD14, a biocide molecule (or portion thereof) such as, a bactericidal enzyme, (e.g., lysozyme), and a linker that connects CD14 and the biocide molecule. The compositions may further comprise a signaling molecule or sequence that predictably directs the composition to an intracellular or extracellular location.

Described herein are broad spectrum antimicrobials. Broad spectrum antimicrobials find use as a preventative tool where the identity of possible food contaminants is unknown, and new organisms can emerge as serious threats. Broad spectrum antimicrobials are also well suited for use in medicine and biodefense in confronting an infection of unknown etiology.

Broad spectrum antimicrobials take advantage of the innate immune system, which provides an important front line defense through receptors on specialized cells (e.g., macrophages, neutrophils) that are capable of binding the vast majority of microbes to which these body surfaces are exposed. The recognition molecule CD14, that recognizes and binds to Pathogen Associated Molecular Patterns (PAMPs) common to many organisms is used as the targeting portion of fusion proteins of the present invention.

Furthermore, broadly reactive monoclonal antibodies that bind to PAMPS can be used as the targeting portion of the fusion proteins. Biocidal enzymes can be delivered in high concentrations to the surface of bacteria by expressing the two components, a microorganism targeting molecule and a biocidal payload as a fusion protein.

IgM (*e.g.*, for increased avidity of binding to repetitive PAMPs) and secretory IgA (*e.g.*, for greater stability in harsh environments) can be used and instead of attaching the biocide directly to the targeting molecule, it is attached to the J chain that is used to assemble both pentameric IgM and dimeric IgA. By using components of the innate immune system antimicrobials can be provided that function effectively ex vivo (*e.g.*, in food safety settings), as well as in vivo (e.g., in clinical medicine and veterinary medicine), which can confront a broad range of bacteria through the broad affinity of the innate recognition. Furthermore, because the recognition targets bacterial features that are essential to bacterial invasion and attachment, resistance is very unlikely to occur. Described herein is a novel class of antimicrobials that find use in a variety of settings.

### I. Immunoglobulins

Immunoglobulins (antibodies) are proteins generated by the immune system to provide a specific molecule capable of complexing with an invading molecule commonly referred to as an antigen. Natural antibodies have two identical antigen-binding sites, both of which are specific to a particular antigen. The antibody molecule recognizes the antigen by complexing its antigen-binding sites with areas of the antigen termed epitopes. The epitopes fit into the conformational architecture of the antigen-binding sites of the antibody, enabling the antibody to bind to the antigen.

The immunoglobulin molecule is composed of two identical heavy and two identical light polypeptide chains, held together by interchain disulfide bonds. Each individual light and heavy chain folds into regions of about 110 amino acids, assuming a conserved three-dimensional conformation. The light chain comprises one variable region (termed V_{L}) and one constant region (C_{L}), while the heavy chain comprises one variable region (V_{H}) and three constant regions (C_{H}1, C_{H}2 and C_{H}3). Pairs of regions associate to form discrete structures. In particular, the light and heavy chain variable regions, V_{L} and V_{H}, associate to form an "F_{V} " area that contains the antigen-binding site.

The variable regions of both heavy and light chains show considerable variability in structure and amino acid composition from one antibody molecule to another, whereas the constant regions show little variability. Each antibody recognizes and binds an antigen through the binding site defined by the association of the heavy and light chain, variable regions into an F_{V} area. The light-chain variable region V_{L} and the heavy-chain variable region V_{H} of a particular antibody molecule have specific amino acid sequences that allow the antigen-binding site to assume a conformation that binds to the antigen epitope recognized by that particular antibody.

Within the variable regions are found regions in which the amino acid sequence is extremely variable from one antibody to another. Three of these so-called "hypervariable" regions or "complementarity-determining regions" (CDR's) are found in each of the light and heavy chains. The three CDRs from a light chain and the three CDRs from a corresponding heavy chain form the antigen-binding site.

Cleavage of naturally occurring antibody molecules with the proteolytic enzyme papain generates fragments that retain their antigen-binding site. These fragments, commonly known as Fab's (for Fragment, antigen binding site) are composed of the C_{L}, V_{L}, C_{H}1 and V_{H} regions of the antibody. In the Fab the light chain and the fragment of the heavy chain are covalently linked by a disulfide linkage.

Monoclonal antibodies against target antigens (*e.g.*, a cell surface protein, such as receptors) are produced by a variety of techniques including conventional monoclonal antibody methodologies such as the somatic cell hybridization techniques of Kohler and Milstein, Nature, 256:495 (1975). Although, somatic cell hybridization procedures can be preferred, other techniques for producing monoclonal antibodies are contemplated as well (*e.g.*, viral or oncogenic transformation of B lymphocytes).

The preferred animal system for preparing hybridomas is the murine system. Hybridoma production in the mouse is a well-established procedure. Immunization protocols and techniques for isolation of immunized splenocytes for fusion are known in the art. Fusion partners (*e.g*., murine myeloma cells) and fusion procedures are also known.

Human monoclonal antibodies (mAbs) directed against human proteins can be generated using transgenic mice carrying the complete human immune system rather than- the mouse system. Splenocytes from the transgenic mice are immunized with the antigen of interest, which are used to produce hybridomas that secrete human mAbs with specific affinities for epitopes from a human protein. (*See e.g*., Wood et al., WO 91/00906, Kucherlapati et al., WO 91/10741; Lonberg et al., WO 92/03918; Kay et al., WO 92/03917; N. Lonberg et al., Nature, 368:856-859 [1994]; L.L. Green et al., Nature Genet., 7:13-21 [1994]; S.L. Morrison et al., Proc. Nat. Acad. Sci. USA, 81:6851-6855 [1994]; Bruggeman et al., Immunol., 7:33-40 [1993]; Tuaillon et al., Proc. Nat. Acad. Sci. USA, 90:3720-3724 [1993]; and Bruggeman et al. Eur. J. Immunol., 21:1323-1326 [1991]).

Monoclonal antibodies can also be generated by other methods known to those skilled in the art of recombinant DNA technology. An alternative method, referred to as the "combinatorial antibody display" method, has been developed to identify and isolate antibody fragments having a particular antigen specificity, and can be utilized to produce monoclonal antibodies. *(See e.g.,* Sastry et al., Proc. Nat. Acad. Sci. USA, 86:5728 [1989]; Huse et al., Science, 246:1275 [1989]; and Orlandi et al., Proc. Nat. Acad. Sci. USA, 86:3833 [1989]). After immunizing an animal with an immunogen as described above, the antibody repertoire of the resulting B-cell pool is cloned. Methods are generally known for obtaining the DNA sequence of the variable regions of a diverse population of immunoglobulin molecules by using a mixture of oligomer primers and the PCR. For instance, mixed oligonucleotide primers corresponding to the 5' leader (signal peptide) sequences and/or framework 1 (FR1) sequences, as well as primer to a conserved 3' constant region primer can be used for PCR amplification of the heavy and light chain variable regions from a number of murine antibodies. (*See e.g.,* Larrick et al., Biotechniques, 11:152-156 [1991]). A similar strategy can also been used to amplify human heavy and light chain variable regions from human antibodies *(See e.g.,* Larrick et al., Methods: Companion to Methods in Enzymology, 2:106-110 [1991]).

RNA can be isolated from B lymphocytes, for example, peripheral blood cells, bone marrow, or spleen preparations, using standard protocols (*e.g.*, US 4,683,292; Orlandi, et al., Proc. Nat. Acad. Sci. USA, 86:3833-3837 [1989]; Sastry et al., Proc. Nat. Acad. Sci. USA, 86:5728-5732 [1989]; and Huse et al., Science, 246:1275 [1989]). First strand cDNA is synthesized using primers specific for the constant region of the heavy chain(s) and each of the κ and λ light chains, as well as primers for the signal sequence. Using variable region PCR primers, the variable regions of both heavy and light chains are amplified, each alone or in combination, and ligated into appropriate vectors for further manipulation ingenerating the display packages. Oligonucleotide primers useful in amplification protocols may be unique or degenerate or incorporate inosine at degenerate positions. Restriction endonuclease recognition sequences may also be incorporated into the primers to allow for the cloning of the amplified fragment into a vector in a predetermined reading frame for expression.

The V-gene library cloned from the immunization-derived antibody repertoire can be expressed by a population of display packages, preferably derived from filamentous phage, to form an antibody display library. Ideally, the display package comprises a system that allows the sampling of very large variegated antibody display libraries, rapid sorting after each affinity separation round, and easy isolation of the antibody gene from purified display packages. In addition to commercially available kits for generating phage display libraries, examples of methods and reagents particularly amenable for use in generating a variegated antibody display library can be found in, for example, US 5,223,409; WO 92/18619; WO 91/17271; WO 92/20791; WO 92/15679; WO 93/01288; WO 92/01047; WO 92/09690; WO 90/02809; Fuchs et al., Biol. Technology, 9:1370-1372 [1991]; Hay et al., Hum. Antibod. Hybridomas, 3:81-85 [1992]; Huse et al., Science, 46:1275-1281 [1989]; Hawkins et al., J. Mol. Biol., 226:889-896 [1992]; Clackson et al., Nature, 352:624-628 [1991]; Gram et al., Proc. Nat. Acad. Sci. USA, 89:3576-3580 [1992]; Garrad et al., Bio/Technolog, 2:1373-1377 [1991]; Hoogenboom et al., Nuc. Acid Res., 19:4133-4137 [1991]; and Barbas et al., Proc. Nat. Acad. Sci. USA, 88:7978 [1991]. The V region domains of heavy and light chains can be expressed on the same polypeptide, joined by a flexible linker to form a single-chain Fv fragment, and the scFV gene subsequently cloned into the desired expression vector or phage genome.

As generally described in McCafferty et al., Nature, 348:552-554 (1990), complete V_{H} and V_{L} domains of an antibody, joined by a flexible linker (*e.g.*, (Gly₄-Ser)₃) can be used to produce a single chain antibody which can render the display package separable based on antigen affinity. Isolated scFV antibodies immunoreactive with the antigen can subsequently be formulated into a pharmaceutical preparation for use in the subject method.

Once displayed on the surface of a display package (*e.g.*, filamentous phage), the antibody library is screened with the target antigen, or peptide fragment thereof, to identify and isolate packages that express an antibody having specificity for the target antigen. Nucleic acid encoding the selected antibody can be recovered from the display package (e.g., from the phage genome) and subcloned into other expression vectors by standard recombinant DNA techniques.

Specific antibody molecules with high affinities for a surface protein can be made according to methods known to those in the art, e.g., methods involving screening of libraries US 5,233,409 and US 5,403,484. Further, the methods of these libraries can be used in screens to obtain binding determinants that are mimetics of the structural determinants of antibodies.

In particular, the Fv binding surface of a particular antibody molecule interacts with its target ligand according to principles of protein-protein interactions, hence sequence data for V_{H} and V_{L} (the latter of which may be of the κ or λ chain type) is the basis for protein engineering techniques known to those with skill in the art. Details of the protein surface that comprises the binding determinants can be obtained from antibody sequence in formation, by a modeling procedure using previously determined three-dimensional structures from other antibodies obtained from NMR studies or crytallographic data.

A variegated peptide library can be expressed by a population of display packages to form a peptide display library. Ideally, the display package comprises a system that allows the sampling of very large variegated peptide display libraries, rapid sorting after each affinity separation round, and easy isolation of the peptide-encoding gene from purified display packages. Peptide display libraries can be in, e.g., prokaryotic organisms and viruses, which can be amplified quickly, are relatively easy to manipulate, and which allows the creation of large number of clones. Preferred display packages include, for example, vegetative bacterial cells, bacterial spores, and most preferably, bacterial viruses (especially DNA viruses). However, also described herein is the use of eukaryotic cells, including yeast and their spores, as potential display packages. Phage display libraries are known in the art.

Other techniques include affinity chromatography with an appropriate "receptor," *e.g.*, a target antigen, followed by identification of the isolated binding agents or ligands by conventional techniques (*e.g.*, mass spectrometry and NMR). Preferably, the soluble receptor is conjugated to a label (*e.g.*, fluorophores, colorimetric enzymes, radioisotopes, or luminescent compounds) that can be detected to indicate ligand binding. Alternatively, immobilized compounds can be selectively released and allowed to diffuse through a membrane to interact with a receptor.

Combinatorial libraries of compounds can also be synthesized with "tags" to encode the identity of each member of the library. (*See e.g.,* W.C. Still et al., WO 94/08051). In general, this method features the use of inert but readily detectable tags that are attached to the solid support or to the compounds. When an active compound is detected, the identity of the compound is determined by identification of the unique accompanying tag. This tagging method permits the synthesis of large libraries of compounds that can be identified at very low levels among to total set of all compounds in the library.

The term modified antibody is also intended to include antibodies, such as monoclonal antibodies, chimeric antibodies, and humanized antibodies which have been modified by, for example, deleting, adding, or substituting portions of the antibody. For example, an antibody can be modified by deleting the hinge region, thus generating a monovalent antibody.

Chimeric mouse-human monoclonal antibodies can be produced by recombinant DNA techniques known in the art. For example, a gene encoding the Fc constant region of a murine (or other species) monoclonal antibody molecule is digested with restriction enzymes to remove the region encoding the murine Fc, and the equivalent portion of a gene encoding a human Fc constant region is substituted. (*See e.g.*, Robinson et al., PCT/US86/02269; European Patent Application 184,187; European Patent Application 171,496; European Patent Application 173,494; WO 86/01533; US 4,816,567; European Patent Application 125,023; Better et al., Science, 240:1041-1043 [1988]; Liu et al., Proc. Nat. Acad. Sci. USA, 84:3439-3443 [1987]; Liu et al., J. Immunol., 139:3521-3526 [1987]; Sun et al., Proc. Nat. Acad. Sci. USA, 84:214-218 [1987]; Nishimura et al., Canc. Res., 47:999-1005 [1987]; Wood et al., Nature, 314:446-449 [1985]; and Shaw et al., J. Natl. Cancer Inst., 80:1553-1559 [1988]).

The chimeric antibody can be further humanized by replacing sequences of the Fv variable region that are not directly involved in antigen binding with equivalent sequences from human Fv variable regions. General reviews of humanized chimeric antibodies are provided by S.L. Morrison, Science, 229:1202-1207 (1985) and by Oi et al., Bio. Techniques, 4:214 (1986). Those methods include isolating, manipulating, and expressing the nucleic acid sequences that encode all or part of immunoglobulin Fv variable regions from at least one of a heavy or light chain. Sources of such nucleic acid are well known to those skilled in the art and, for example, may be obtained from 7E3, an anti-GPII_{b}IIIₐ, antibody producing hybridoma. The recombinant DNA encoding the chimeric antibody, or fragment thereof, can then be cloned into an appropriate expression vector.

Suitable humanized antibodies can alternatively be produced by CDR substitution (*e.g*., US 5,225,539; Jones et al., Nature, 321:552-525 [1986]; Verhoeyan et al., Science, 239:1534 [1988]; and Beidler et al., J. Immunol., 141:4053 [1988]). All of the CDRs of a particular human antibody may be replaced with at least a portion of a non-human CDR or only some of the CDRs may be replaced with non-human CDRs. It is only necessary to replace the number of CDRs required for binding of the humanized antibody to the Fc receptor.

An antibody can be humanized by any method that is capable of replacing at least a portion of a CDR of a human antibody with a CDR derived from a non-human antibody. The human CDRs may be replaced with non-human CDRs; using oligonucleotide site-directed mutagenesis.

Also described herein are chimeric and humanized antibodies in which specific amino acids have been substituted, deleted or added. In particular, preferred humanized antibodies have amino acid substitutions in the framework region, such as to improve binding to the antigen. For example, in a humanized antibody having mouse CDRs, amino acids located in the human framework region can be replaced with the amino acids located at the corresponding positions in the mouse antibody. Such substitutions are known to improve binding of humanized antibodies to the antigen in some instances.

The fusion proteins may include a monoclonal antibody subunit (*e.g.*, a human, murine, or bovine), or a fragment thereof, (*e.g.*, an antigen binding fragment thereof). The monoclonal antibody subunit or antigen binding fragment thereof can be a single chain polypeptide, a dimer of a heavy chain and a light chain, a tetramer of two heavy and two light chains, or a pentamer (*e.g.*, IgM). IgM is a pentamer of five monomer units held together by disulfide bonds linking their carboxyl-terminal (Cµ4/Cµ4) domains and Cµ3/Cµ3 domains. The pentameric structure of IgM provides 10 antigen-binding sites, thus serum IgM has a higher valency than other types of antibody isotypes. With its high valency, pentameric IgM is more efficient than other antibody isotypes at binding multidimensional antigens (*e.g.*, viral particles and red blood cells. However, due to its large pentameric structure, IgM does not diffuse well and is usually found in low concentrations in intercellular tissue fluids. The J chain of IgM allows the molecule to bind to receptors on secretary cells, which transport the molecule across epithelial linings to the external secretions that bathe the mucosal surfaces. One may take advantage of the low diffusion rate ofpentameric IgM to help concentrate the fusion proteins of present invention at a site of interest. Monoclonal IgM, and fusion and chimeric proteins thereof, may be directed to destroying *Cryptosporidium parvum* and other types of parasitic pathogens.

An IgA can be utilized to make a directed biocide. IgA's are preferably produced using either one, two or three constructs. IgA made by use of two or three retrovector constructs. For example, a retroviral construct can be produced in which the J-chain expression is driven by the long terminal repeat (LTR) promoter, and expression of a heavy chain and light chain separated by an IRES sequence is driven by an internal promoter. In another example, the heavy chain and light chain are provided in one vector and the J chain is provided in another vector. In another example, a third construct expressing the secretory component truncated form from poly IgR is provided.

Secretion of a directed biocide can be enhanced by transfecting a cell producing a directed biocide with a vector (e.g., a retroviral vector) that expressed secretory component. See U.S. Pat. No. 6,300,104; Koteswarra and Morrison, Proc. Natl. Acad. Sci. USA 94:6364-68 (1997).

The monoclonal antibody can be a murine antibody or a fragment thereof. The monoclonal antibody can be a bovine antibody or a fragment thereof. For example, the murine antibody can be produced by a hybridoma that includes a B cell obtained from a transgenic mouse having a genome comprising a heavy chain transgene and a light chain transgene fused to an immortalized cell. The antibodies can be of various isotypes, including, but not limited to: IgG (*e.g.*, IgG1, IgG2, IgG2a, IgG2b, IgG2c, IgG3, IgG4); IgM; IgA1; IgA2; IgA_{sec}; IgD; and IgE. The antibody can be an IgG isotype. The antibody can be an IgM isotype. The antibodies can be full-length (*e.g.*, an IgG1, IgG2, IgG3, or IgG4 antibody) or can include only an antigen-binding portion (*e.g.*, a Fab, F(ab')₂, Fv or a single chain Fv fragment).

The immunoglobulin subunit of the fusion proteins can be a recombinant antibody (*e.g.*, a chimeric or a humanized antibody), a subunit, or an antigen binding fragment thereof (*e.g.*, has a variable region, or at least a complementarity determining region (CDRs)).

The immunoglobulin subunit of the fusion protein may be monovalent (*e.g.*, includes one pair of heavy and light chains, or antigen binding portions thereof). The immunoglobulin subunit of the fusion protein may be a divalent (*e.g.*, includes two pairs of heavy and light chains, or antigen binding portions thereof). The transgenic fusion proteins may include an immunoglobulin heavy chain or a fragment thereof (*e.g.*, an antigen binding fragment thereof).

The fusion proteins and/or or recombinant antibodies may comprise an immunoglobulin having only heavy chains such as the HCAbs found in certain *Camelidae* (*e.g*., camels, dromedaries, and Ilamas) species, spotted ratfish, and nurse shark. There may be differences between conventional antibodies and HCAbs in both the V_{H} and C_{H} regions. For instance, as reported by Muyldermans *et al.* and Nguyen *et al.,* the sequences of HCAbs variable domains (V_{H}H) differ significantly from those of conventional antibodies (V_{H}). (S. Muyldermans et al., Protein, Eng., 7:1129-1135 [1994]; V.K. Nguyen et al., J. Mol. Biol., 275:413-418 [1998]; and V.K. Nguyen et al., Immunogenetics DOI 10.1007/s00251-002-0433-0 [2002]). Additionally, HCAbs lack the first domain of the constant region (C_{H}); the matured V_{H}H-DJ is directly joined to the hinge region. Separate sets of V and C genes encode conventional antibodies and HCAbs, however, conventional antibodies and HCAbs have some common D genes and appear to have identical J_{H} regions. (V.K. Nguyen et al., EMBO J., 19:921-930 [2000]; and V.K. Nguyen et al., Adv. Immunol., 79:261-296 [2001]).

IgM may be used as the microorganism targeting molecule. IgMs bind with multiple epitopes, effectively enhancing the avidity of the binding. The genes for both SP-D and MBL of these molecules have been sequenced and both have been produced as recombinant molecules in full or truncated forms (Shrive et al., J Mol Biol 2003; 331:509-23; Arora et al., J Biol Chem 2001; 276:43087-94).

The microorganism targeting molecules may be monoclonal antibodies that target PAMPs. The monoclonal antibodies may utilize the multimeric structure of IgM and IgA. The repetitive structure of many PAMPs allows antibodies to bind to two identical epitopes on one molecule or on separate molecules (cross-linking) on the bacterial surface. This type of interaction results in an overall higher binding energy per antibody molecule than the engagement of one single arm of the immunoglobulin in the binding; for the antibody to detach, both binding sites would have to be released at once. The avidity is proportionally higher for IgA, which is a dimer, bearing a total of 4 binding sites, or IgM which usually is a pentamer, having 10 binding sites.

Packing plants are harsh environments; secretory IgA is adapted to function in the gastrointestinal tract and its dimeric configuration is supported by a portion of the secretory component that assists its membrane transport. Thus IgM and IgA may offer advantages as targeting molecules over IgG. The fact that they are linked by a J chain, and in the case of IgA coexpressed with secretory component, may allow for attaching the payload biocide to these auxiliary chains instead of to the Fc portion of the immunoglobulin itself. Accordingly, the immunoglobulin J-chain can be used as a microorganism targetin molecule.

A system of hybridoma-like antibody preparation, developed by Neoclone (Madison, WI), can be used in the production of monoclonal antibodies. Splenocytes from immunized mice are immortalized using a retrovector-mediated introduction of the abl-myc genes. On reintroduction into recipient mice one dominant immortalized B cell clone (plasmacytoma) outgrows all others and produces a monoclonal antibody in the ascitic fluid. The B cell clone can be harvested with the ascitic fluid that contains high concentration of monoclonal antibody. This process can be completed in 8-10 weeks.

### II. Innate System Receptors

Innate immunity receptors can be used as microorganism targeting molecules due to their high affinity of interaction with a multitude of microorganisms. These receptors are all highly conserved structures across species, even across classes (Ezekowitz RAB and Hoffmann JA. Innate Immunity. Totowa, NJ: HUmana Press, 2003). Many of these evolutionarily ancient receptors are found in Invertebrae (Aderem A and Ulevitch RJ. Nature 2000; 406:782-7). Preferred microorganism targeting molecules are those that exist as soluble molecules in circulation.

In the present invention, the microorganism targeting molecule is CD 14, found on monocytes/macrophages and neutrophils (Haziot et al., J Immunol 1988; 141:547-52). This molecule exists as both a membrane-bound form, with a GPI anchor, and as a soluble form. Both versions of CD14 bind LPS with high affinity. The reported dissociation constant for LPS binding to CD14 is K_{D} 3-7 x 10⁻⁸ M (Tobias et al., J Biol Chem 1995; 270:10482-8). Haziot and co-workers have produced recombinant soluble human CD14 and have used it to study the binding to LPS (Haziot et al., J Immunol 1995; 154:6529-32), showing that soluble CD14 binds LPS under various conditions. LPS is one of the main components of the cell wall of Gram-negative bacteria. CD14 also binds to peptidoglycan structures with high affinity (Kd=25nM) (Dziarski, Cell Mol Life Sci 2003; 60:1793-804; Dziarski et al., Chem Immunol 2000; 74:83-107; Dziarski et al., Infect Immun 2000; 68:5254-60.). Peptidoglycan (PGN) structures make up a substantial portion of the cell wall of Gram-negative and Gram-positive bacteria. In addition, CD14 binds to lipoteichoic acid on Gram-positive bacteria, lipoarabinomannan ofmycobacteriae, lipoproteins from spirochetes and mycobacteriae, and others (Dziarski, Cell Mol Life Sci 2003; 60:1793-804). CD14 is a highly versatile receptor that interacts with an impressive variety of different bacteria.

The microorganism targeting molecule may be the LPS-binding protein (LBP) (Tobias et al., J Exp Med 1986; 164:777-93). LBP is an acute phase protein that is released into circulation upon a bacterial infection with Gram-negative bacteria or exposure to LPS. LBP can bind circulating or bound LPS, and through this interaction inhibit LPS-related septic shock (Lamping et al., J Clin Invest 1998; 101:2065-71). LBP can inhibit LPS-induced signaling by blocking LPS transfer from CD14 to Toll like receptor 4 (Thompson et al., J Biol Chem 2003; 278:28367-71). In addition, LBP is capable of removing LPS from mCD14 with high efficiency, indicating that LBP binds LPS more strongly than CD14. Separate studies aimed at comparing binding efficiencies between LPS, LBP, and CD14 have confirmed that LBP binds LPS with a roughly ten-fold higher affinity K_{D} 3.5 x 10⁻⁹ M (Tobias et al., J Biol Chem 1995; 270:10482-8) than CD14. LBP has been cloned, sequenced and expressed by various groups (Schumann et al., Science 1990; 249:1429-31; Theofan et al., J Immunol 1994; 152:3623-9; Thompson et al., J Biol Chem 2003; 278:28367-71).

The microorganism targeting molecule may be a member of the collectins, also called defense collagens (Van De Wetering et al., Eur J Biochem 2004; 271:1229-49). Surfactant protein D (SP-D) has been shown to interact with rough and smooth LPS on bacterial surfaces (Clark et al., Microbes Infect 2000; 2:273-8; Lawson and Reid, Immunol Rev 2000; 173:66-78) and therefore can target Gram-negative microorganisms. Mannan-binding lectin (MBL), from this family, which is known to target peptidoglycans (and hence Gram-positive bacteria) (Lu et al., Biochim Biophys Acta 2002; 1572:387-400), can be utilized. Collectins assemble into multimers, effectively multiplying the number of binding sites per complex available for interaction with the microorganism's repetitive surface.

The Toll receptor family can be used as pathogen targeting molecules; this group of cell bound receptors functions singly or in concert with other innate immune system receptors (Ezekowitz and Hoffmann, Innate Immunity. Totowa, NJ: HUmana Press, 2003; Janeway and Medzhitov. Annu Rev Immunol 2002; 20:197-216; Medzhitov and Janeway, Trends Microbiol 2000; 8:452-6. Toll like receptors (TLRs) comprise a family of cell surface receptors that are related to the *Drosophila* Toll protein, a molecule involved in defense against fungal infection in the fly (Aderem and Ulevitch, Nature, 406:785-787 [2000]). Ten mammalian TLRs have been identified (Aderem and Ulevitch, *Supra*). Two members of the family, TLR2 and TLR4, have been better characterized and shown to mediate the response to multiple bacterial cell-wall components including lipopolysaccharide (LPS), lipopeptides, peptidoglycans (PGN) and lipoteichoic acid (LTA) (Yang et al., Nature, 395:284-288 [1998]; Poltorak et al., Science, 282:2085-2088 [1998]; Aliprantis et al., Science, 285:736-739 [1999]; Chow et al., J. Biol. Chem., 274:10689-10692 [2000]; and Schwandner et al., J. Biol. Chem., 274: 17406-17409 [2000]). Mammalian TLRs have multiple leucine-rich repeats in the ectodomain and an intracellular Toll-IL1 receptor (TIR) domain that mediates a signaling cascade to the nucleus (Aderem and Ulevitch, *Supra*). Stimulation of TLR2 and TLR4 leads to the recruitment of the adaptor molecule MyD88 and the serine kinase IL-1R-associated kinase (IRAK), two signaling components that together with TRAF-6 mediate activation of NF-κB (Aderem and Ulevitch, *Supra*).

### III. Linkers

The transgenic fusion proteins described herein comprise a targeting molecule connected to a biocide molecule by a linker. The targeting molecule can be linked via a peptide linker or is directly fused (*e.g.*, covalently bonded) to the biocide molecule. The transgenic fusion proteins can assemble into dimeric, trimeric, tetrameric, pentameric, hexameric or higher polymeric complexes.

Described herein are retroviral constructs that encode in operable configuration an immunoglobulin (or portion thereof), a biocide molecule (or portion thereof), and a linker group that connects the immunoglobulin and the biocide. The linker group may comprise one amino acid moiety (*e.g.*, Xₙ; wherein X is any amino acid or amino acid derivative; and n =1). The linker group may comprise at least one amino acid moiety (*e.g.*, Xₙ; wherein X is any amino acid or amino acid derivative; and n ≥ 2). Similarly, The linker group may comprise two or more repeating amino acids (*e.g.*, XₙY_{z}; wherein X and Y are any amino acid or amino acid derivative; and n ≥ 1 and z ≥ 1). The linker group may comprise two or more repeating amino acids that form a repeating unit (*e.g.*, (XₙY_{z})ᵣ; wherein r ≥ 1). The present invention is not intended to be limited, however, to the aforementioned linker groups. Those skilled in the art will appreciate that a number of other linker group configurations and compositions find use in certain embodiments of the present invention.

In particularly, the linker group described herein has one or more of the following characteristics: 1) sufficient length and flexibility to allow for the rotation of the targeting molecule and the biocide molecule (*e.g.*, lysozyme) relative to one another; 2) a flexible extended conformation; 3) a propensity for developing ordered secondary or tertiary structures that interact with functional components; 4) nonreactive with the functional components of the construct (*e.g.*, minimal hydrophobic or charged character to react with the functional protein domains); 5) sufficient resistant to degradation (*e.g.*, digestion by proteases); and 6) allows the fusion protein to form a complex (*e.g.*, a di-, tri-, tetra-, penta-, or higher multimeric complex) while retaining biological (*e.g.*, biocidal) activity. The linker sequence should separate the target molecule and the biocide molecule of the fusion protein by a distance sufficient to ensure that each component properly folds into its secondary and tertiary structures.

In preferred embodiments, the peptide linker is from 2 to 500, more preferably from 50 to 100, and even more preferably, from 10 to 30 amino acids long. A polypeptide linker sequence of about 20 amino acids provides a suitable separation of functional protein domains. For example the peptide linker may be between 17 to 20 amino acids in length.

The present invention further contemplates peptide linkers comprised of the following amino acids: Gly, Ser, Asn, Thr or Ala. Typical surface amino acids in flexible protein regions include Gly, Ser, and Asn. Various amino acid sequence permutations of Gly, Ser, and optionally Asn, may provide suitable linker sequences. The peptide linkers may comprise further uncharged polar amino acids (*e.g.*, Gln, or Tyr) and/or nonpolar amino acids (*e.g.*, Val, Leu, Ileu, Pro, Phe, Met, Trp, Cys).

In some preferred embodiments, the peptide linker comprises one (or more) Gly-Ser elements. Fore example, in some of these embodiments, the peptide linker has the formula (Serₙ-Glyₓ)_{y}, wherein n and x ≥ 1, and y ≥ 1. In some preferred embodiments, the peptide linker has the formula (Ser-Gly₄)_{y}, wherein y = 1, 2, 3, 4, 5, 6, 7, 8 or more. The peptide linker may include a sequence having the formula (Ser-Gly₄)₃. The peptide linker may comprise a sequence of the formula ((Ser-Gly₄)₃-Ser-Pro). Other peptide linker sequences are described herein, including, but not limited to, Gly₄SerGly₅Ser, and ((Ser₄-Gly)₃-Ser-Pro).

The target molecule and the biocidal molecule comprising the fusion protein may be fused directly without a linker sequence. Linker sequences may be unnecessary where the fusion protein components have non-essential N-or C-terminal amino acid regions that separate functional domains and prevent steric interference.

### IV. Biocides

Described herein are novel fusion proteins. The recombinant fusion proteins may comprise one or more biocide molecules (*e.g.*, a bactericidal enzyme) attached to the antibody portion of the construct via a linking group. The specificity of the monoclonal antibody portion of the construct targets the biocide molecule to a pathogen such as, for example, *E. coli* O157:H7 *Listeria monocytogenes*, *Campylobacter jejuni*, or *Cryptosporidium parvum.*

One benefit of the specific targeting ability of the fusion protein construct is that it allows for relative accumulation of biocide at locations where the targeted pathogens are challenging the animal. Increasing the local concentration of biocide relative to the targeted pathogens enhances the biocidal activity of the fusion protein construct. In particular, directing the biocide (*e.g.*, lysozyme, PLA2, and the like) to the immediate vicinity of the pathogen (*e.g.*, a bacterium) via the antibody portion of the construct may effectively increase the biocide's local concentration, thus providing a significantly greater biocidal (*e.g.*, bactericidal) effect than administering biocide alone (parasiticidal compounds). For example in the case of lysozyme, the affinity constant (Kₘ) of lysozyme for its substrate is approximately 10⁻³ M, while that of phospholipase A2 is approximately 10⁻⁴ M. However, the K_{d} of a monoclonal antibody is usually in the range of 10⁻⁸ M to 10⁻¹¹ M, thus antibodies have about 5 orders of magnitude higher affinity for their substrates than do biocidal molecules alone. Therefore, monoclonal antibodies (or portions thereof can be used to specifically direct biocide molecules to a target by taking advantage of the antibody's very high affinity for target pathogens. Additionally, directing the fusion protein constructs to target pathogens also reduces the possible deleterious effects to the animal caused by systemic administration of the biocidal molecules.

The directed biocidal approach described herein may use a monoclonal antibody to direct a naturally occurring bactericidal enzyme to the target pathogen. The bactericidal enzyme(s) may be components of the innate immune system. One such preferred bactericidal enzyme is lysozyme.

Lysozyme is naturally present in mammalian tissues and in secretions such as tears and mucus. Lysozyme is also found in many foods including, egg whites, cow milk, and human colostrum. The enzyme is widely reported to have antibacterial properties. Lysozyme is a glycosidase that targets the polysaccharides of many bacterial cell walls rendering them more susceptible to osmotic lysis. Lysozyme is a 1,4-β-N-acetylmurmidase that cleaves the glycosidic bond between C-1 of N-acetylmuramic acid and C-4 of N-acetylglucosamine of the peptidoglycan layer present in many bacterial cell walls (*See e.g.,* M. Schindler et al., Biochemistry, 16(3):423-431 [1977]). While it is not clear whether this cleavage contributes to the bactericidal action of lysozyme (K. During et al., FEBS Lett., 449(2-3):93-100 [1999]; and H.R. Ibrahim et al., FEBS Lett., 506(1):27-32 [2001]), it is widely accepted that lysozyme plays an important role in defense against bacterial infection. Lysozyme has also been shown to bind to the lipid A portion of bacterial endotoxin. This interaction prevents the endotoxin from inducing the release of inflammatory components by lymphocytes and macrophages (*See e.g.,* B. Reusens-Billen et al., Diabetes Res. Clin. Pract., 23(2):85-94 [1994]; K. Takada et al., Infect. Immun., 62(4):1171-1175 [1994]; and K. Takada et al., Circ. Shock, 44(4):169-174 [1994]).

Other proteins that form part of the innate immune system, and especially those secreted by the intestinal Paneth cells, are contemplated for targeting the structural integrity of sporozoites. For example, phopholipase A2 (PLA2) is another naturally occurring bactericidal enzyme contemplated for use in certain embodiments of the present invention. Secretory type II phospholipase A2 (sPLA(2)-IIA) is a 14 kD enzyme synthesized in a number of gland cells, including Paneth cells of intestinal mucosa, prostate gland cells, and lacrimal glands. It is present in cellular secretions on mucosal surfaces including intestinal mucus, seminal plasma, and tears (X.D. Qu and R.I. Lehrer, Infect. Immun., 66:2791-2797 [1998]; and X.D. Qu et al., Infect. Immun., 64:5161-5165 [1996]). Evidence suggests that phopholipase A2 has an important antibacterial role in addition to its inflammatory mediating role *(See e.g.,* A.G. Buckland and D.C. Wilton, Biochim. Biophys. Acta, 1488(1-2):71-82 [2000]). Elevated amounts of phospholipase A2 is found in patients with acute bacterial diseases (J.O. Gronoos et al., J. Infect. Dis., 185:1767-1772 [2002]). The enzyme appears to effective in controlling *E coli.* infections when expressed in transgenic mice (*See e.g.*, V.J. Laine et al., Infect. Immun., 68(1):87-92 [2000]). While the present invention is not limited to any mechanisms, PLA2 appears to hydrolyze membrane phospholipids, thus destroying the membranes of invading microbes. PLA2 serves as a critical component of the innate immune system, functioning in combination with lysozyme and the defensins to provide an effective barrier to invasion by a diverse range of organisms.

Mammalian cells are generally highly resistant to sPLA(2) IIA (R.S. Koduri et al., J. Biol. Chem., 273:32142-32153 [1998]). The substrate specificity of the different members of the PLA2 family may be related to the differences in interfacial binding characteristics to charge-neutral phosphotidyl choline (PC) versus anionic phospholipids. Indeed, sPLA(2) family members sPLA2-V and -X bind efficiently and hydrolyze PC vesicles *in vitro* whereas the vesicles are a poor binding substrate for -IIA. Plasma membranes with a high PC content would therefore be stable in the presence of sPLA(2)-IIA. The composition of the phospholipids on the surface of the organism therefore contributes to the susceptibility of the organism to the action of sPLA2. Some parasitic eukaryotic organisms may evade the innate immune system by not stimulating the cells of the immune system to release biocidal enzymes and defensins (*e.g*., *G. lamblia* and *C. albicans* appear not to stimulate Paneth cells). However, one recent report suggests that *Plasmodium* is susceptible to sPLA2 (Type III, from bee venom). Type III sPLA2 has an activity that is similar to the type IIA enzyme, but is a slightly larger molecule having N- and C-terminal extensions. Systemically, sPLA(2)-IIA has a role in generalized inflammatory responses. In acute inflammation, the levels of the enzyme are elevated many hundreds of fold, however, it appears to have no adverse effect at epithelial surfaces. *In vitro,* sPLA(2) apparently has no deleterious effect on various types of cultured mammalian cells. Healthy transgenic mice chronically over-expressing sPLA(2)-IIA have been produced and exhibit an elevated resistance to infection by gram positive organisms (V.J. Laine et al., J. Immunol., 162:7402-7408 [1999]; and V.J. Laine et al., Infect, Immun., 68:87-92 [2000]).

A number of inhibitors have been identified that have activity against *C. parvum* by targeting the parasite's metabolic pathways. These include, but are not limited to, metalloprotease inhibitors (P.C. Okhuysen et al., Antimicrob. Agents Chemother., 40:2781-2784 [1996]) and serine protease antagonists (J.R. Forney et al., J. Parasitol., 82:638-640 [1996]). Other enzymes essential to *C. parvum* infectivity provide useful inhibitor targets. These include, for example, phosphoinositide 3-kinase (J.R. Forney et al., Infect. Immun., 67:844-852 [1999]) and cysteine proteinase (M.V. Nesterenko et al., Microbios., 83:77-88 [1995]).

Other naturally occurring bactericidal molecules (*e.g.*, enzymes) contemplated for use in certain embodiments of the present invention, include, but are not limited to, lactoferrin, lactoperoxidase, bacterial permeability increasing protein (BPI), and Aprotinin. *(See e.g.,* B.A. Mannion et al., J. Clin. Invest., 85(3):853-860 [1990]; A. Pellegrini et al., Biochem. Biophys. Res. Commun., 222(2):559-565 [1996]; and P. Prohinar et al., Mol. Microbiol., 43(6):1493-1504 [2002]).

The biocide component of the fusion protein may comprise an antimicrobial polypeptide (*See e.g.,* Antimicrobial Peptide Protocols, ed. W. M. Shafer, Humana Press, Totowa, NJ [1997]) or a pore forming agent. The antimicrobial peptide or pore forming agent may be a compound or peptide selected from the following: magainin (*e.g.*, magainin I, magainin II, xenopsin, xenopsin precursor fragment, caerulein precursor fragment), magainin I and II analogs (PGLa, magainin A, magainin G, pexiganin, Z-12, pexigainin acetate, D35, MSI-78A, MG0 [K10E, K11E, F12W-magainin 2], MG2+ [K10E, F12W-magainin-2], MG4+ [F12W-magainin 2], MG6+ [f12W, E19Q-magainin 2 amide], MSI-238, reversed magainin II analogs [*e.g*., 53D, 87-ISM, and A87-ISM], Ala-magainin II amide, magainin II amide), cecropin P1, cecropin A, cecropin B, indolicidin, nisin, ranalexin, lactoferricin B, poly-L-lysine, cecropin A (1-8)-magainin II (1-12), cecropin A (1-8)-melittin (1-12), CA(1-13)-MA(1-13), CA(1-13)-ME(1-13), gramicidin, gramicidin A, gramicidin D, gramicidin S, alamethicin, protegrin, histatin, dermaseptin, lentivirus amphipathic peptide or analog, parasin I, lycotoxin I or II, globomycin, gramicidin S, surfactin, ralinomycin, valinomycin, polymyxin B, PM2 [ (+/-) 1-(4-aminobutyl)-6-benzylindane], PM2c [ (+/-) -6-benzyl-1-(3-carboxypropyl)indane], PM3 [(+/-)1-benzyl-6-(4-aminobutyl)indane], tachyplesin, buforin I or II, misgurin, melittin, PR-39, PR-26, 9-phenylnonylamine, (KLAKKLA)n, (KLAKLAK)n, where n = 1, 2, or 3, (KALKALK)3, KLGKKLG)n, and KAAKKAA)n, wherein N =1, 2, or 3, paradaxin, Bac 5, Bac 7, ceratoxin, mdelin 1 and 5, bombin-like peptides, PGQ, cathelicidin, HD-5, Oabac5alpha, ChBac5, SMAP-29, Bac7.5, lactoferrin, granulysin, thionin, hevein and knottin-like peptides, MPG1, IbAMP, snakin, lipid transfer proteins, and plant defensins. Exemplary sequences for the above compounds are provided in Table 1. The antimicrobial peptides may be synthesized from L-amino acids, or may be synthesized from or comprise D-amino acids.

| **Table 1 Antimicrobial Peptides** | | | |
|---|---|---|---|
| **SEQ ID NO:** | **Name** | **Organism** | **Sequence** |
| 1 | lingual antimicrobial peptide precursor (Magainin) | *Bos taurus* | MRLHHLLLALLFLVLSAGSGFTQGVR NSQSCRRNKGICVP IRCPGSMRQIGTCLGAQVKCCRRK |
| 2 | antimicrobial peptide PGQ | *Xenopus laevis* | GVLSNVIGYLKKLGTGALNAVLKQ |
| 3 | Xenopsin | *Xenopus laevis* | MYKGIFLCVLLAVICANSLATPSSDA DEDNDEVERYVRGW ASKIGQTLGKIAKVGLKELIQPKREA MLRSAEAQGKRPWIL |
| 4 | magainin precursor | *Xenopus laevis* | MFKGLFICSLIAVICANALPQPEASAD EDMDEREVRGIGKFLHSAGKFGKAF VGEIMKSKRDAEAVGPEAFADEDLD EREVRGIGKFLHSAKKFGKAFVGEIM NSKRDAEAVGPEAFADEDLDEREVR GIGKFLHSAKKFGKAFVGEIMNSKRD AEAVGPEAFADEDLDEREVRGIGKFL HSAKKFGKAFVGEIMNSKRDAEAVG PEAFADEDFDEREVRGIGKFLHSAKK FGKAFVGEIMNSKRDAEAVGPEAFA DEDLDEREVRGIGKFLHSAKKFGK AFVGEIMNSKRDAEAVDDRRWVE |
| 5 | tachyplesin I | *Tachypleus gigas* | KWCFRVCYRGICYRRCR |
| 6 | tachyplesin II | *Tachypleus gigas* | RWCFRVCYRGICYRKCR |
| 7 | buforin I | *Bufo bufo gagarizans* | MSGRGKQGGKVRAKAKTRSSRAGL QFPVGRVHRLLRKGNYAQRVGAGA PVYLAAVLEYLTAEILELAGNAARD NKKTRIIPRHLQLAVRNDEELNKLLG GVTIAQGGVLPNIQAVLLPKT ESSKPAKSK |
| 8 | buforin II | *Bufo bufo gagarizans* | TRSSRAGLQFPVGRVHRLLRK |
| 9 | cecropin A | *Bombyx mori* | MNFVRILSFVFALVLALGAVSAAPEP RWKLFKKIEKVGRNVRDGLIKAGPAI AVIGQAKSLGK |
| 10 | cecropin B | *Bombyx mori* | MNFAKILSFVFALVLALSMTSAAPEP RWKIFKKIEKMGRN IRDGIVKAGPAIEVLGSAKAIGK |
| 11 | cecropin C | *Drosophila melanogaster* | MNFYKIFVFVALILAISIGQSEAGWLK KLGKRIERIGQHT RDATIQGLGIAQQAANVAATARG |
| 12 | cecropin P1 | *Sus scrofa* | SWLSKTAKKLENSAKKRISEGIAIAIQ GGPR |
| 13 | indolicidin | *Bos taurus* | ILPWKWPWWPWRR |
| 14 | nisin | *Lactococcus lactis* | ITSISLCTPGCKTGALMGCNMKTATC HCSIHVSK |
| 15 | ranalexin | *Rana catesbeiana* | FLGGLIKIVPAMICAVTKKC |
| 16 | lactoferricin B | *Bos taurus* | FKCRRWQWRMKKLGAPSITCVRRAF |
| 17 | protegrin-1 | *Sus scrofa* | RGGRLCYCRRRFCVCVGRX |
| 18 | protegrin-2 | *Sus scrofa* | GGRLCYCRRRFCICVG |
| 19 | histatin precursor | *Homo sapiens* | MKFFVFALILALMLSMTGADSHAKR HHGYKRKFHEKHHSHRGYRSNYLY DN |
| 20 | histatin 1 | *Macaca fascicularis* | DSHEERHHGRHGHHKYGRKFHEKH HSHRGYRSNYLYDN |
| 21 | dermaseptin | *Phyllomedus a sauvagei* | ALWKTMLKKLGTMALHAGKAALGA AADTISQTQ |
| 22 | dermaseptin 2 | *Phyllomedus a sauvagei* | ALWFTMLKKLGTMALHAGKAALGA AANTISQGTQ |
| 23 | dermaseptin 3 | *Phyllomedus a sauvagei* | ALWKNMLKGIGKLAGKAALGAVKK LVGAES |
| 24 | misgurin | *Misgurnus anguillicaud atus* | RQRVEELSKFSKKGAAARRRK |
| 25 | melittin | *Apis mellifera* | GIGAVLKVLTTGLPALISWISRKKRQ Q |
| 26 | pardaxin-1 | *Pardachirus pavoninus* | GFFALIPKIISSPLFKTLLSAVGSALSSS GEQE |
| 27 | pardaxin-2 | *Pardachirus pavoninus* | GFFALIPKIISSPIFKTLLSAVGSALSSS GGQE |
| 28 | bactenecin 5 precursor | *Bos taurus* | METQRASLSLGRCSLWLLLLGLVLPS ASAQALSYREAVLR AVDQFNERSSEANLYRLLELDPTPND DLDPGTRKPVSFRV KETDCPRTSQQPLEQCDFKENGLVK QCVGTVTLDPSNDQFDINCNELQSVR FRPPIRRPPIRPPFYPPFRPPIRPPIFPPIR PPFRPPLGPFPGRR |
| 29 | bactenecin precursor | *Bos taurus* | METPRASLSLGRWSLWLLLLGLALPS ASAQALSYREAVLR AVDQLNEQSSEPNIYRLLELDQPPQD DEDPDSPKRVSFRVKETVCSRTTQQP PEQCDFKENGLLKRCEGTVTLDQVR GNFDITCNNHQSIRITKQPWAPPQAA RLCRIVVIRVCR |
| 30 | ceratotoxin A | *Ceratitis capitata* | SIGSALKKALPVAKKIGKIALPIAKAA LP |
| 31 | ceratotoxin B | *Ceratitis capitata* | SIGSAFKKALPVAKKIGKAALPIAKA ALP |
| 32 | cathelicidin antimicrobial peptide | *Homo sapiens* | MKTQRNGHSLGRWSLVLLLLGLVMP LAIIAQVLSYKEAVL RAIDGINQRSSDANLYRLLDLDPRPT MDGDPDTPKPVSFT VKETVCPRTTQQSPEDCDFKKDGLV . KRCMGTVTLNQARGSFDISCDKDNK RFALLGDFFRKSKEKIGKEFKRIVQRI KDFLRNLVPRTES |
| 33 | myeloid cathelicidin 3 | *Equus caballus* | METQRNTRCLGRWSPLLLLLGLVIPP ATTQALSYKEAVLRAVDGLNQRSSD ENLYRLLELDPLPKGDKDSDTPKPVS FMVKETVCPRIMKQTPEQCDFKENG LVKQCVGTVILDPVKDYFDASCDEP QRVKRFHSVGSLIQRHQQMIRDKSEA TRHGIRIITRPKLLLAS |
| 34 | myeloid antimicrobial peptide BMAP-28 | *Bos taurus* | METQRASLSLGRWSLWLLLLGLALP SASAQALSYREAVLR AVDQLNEKSSEANLYRLLELDPPPKE DDENPNIPKPVSFRVKETVCPRTSQQ SPEQCDFKENGLLKECVGTVTLDQV GSNFDITCAVPQSVGGLRSLGRKILR AWKKYGPIIVPIIRIG |
| 35 | myeloid cathelicidin 1 | *Equus caballus* | METQRNTRCLGRWSPLLLLLGLVIPP ATTQALSYKEAVLR AVDGLNQRSSDENLYRLLELDPLPKG DKDSDTPKPVSFMVKETVCPRIMKQ TPEQCDFKENGLVKQCVGTVILGPV KDHFDVSCGEPQRVKRFGRLAKSFL RMRILLPRRKILLAS |
| 36 | SMAP 29 | *Ovis aries* | METQRASLSLGRCSLWLLLLGLALPS ASAQVLSYREAVLRAADQLNEKSSE ANLYRLLELDPPPKQDDENSNIPKPV SFRVKETVCPRTSQQPAEQCDFKENG LLKECVGTVTLDQVRNNFDITCAEPQ SVRGLRRLGRKIAHGVKKYGPTVLRI IRIAG |
| 37 | BNP-1 | *Bos taurus* | RLCRIVVIRVCR |
| 38 | HNP-1 | *Homo sapiens* | ACYCRIP.ACIAGERRYGTCIYQGRLW AFCC |
| 39 | HNP-2 | *Homo sapiens* | CYCRIPACIAGERRYGTCIYQGRLWA FCC |
| 40 | HOP-3 | *Homo sapiens* | DCYCRIPACIAGERRYGTCIYQGRLW AFCC |
| 41 | HNP-4 | *Homo sapiens* | VCSCRLVFCRRTELRVGNCLIGGVSF TYCCTRV |
| 42 | NP-1 | *Oryctolagus cuniculus* | VVCACRRALCLPRERRAGFCRIRGRI HPLCCRR |
| 43 | NP-2 | *Oryctolagus cuniculus* | VVCACRRALCLPLERRAGFCRIRGRI HPLCCRR |
| 44 | NP-3A | *Oryctolagus cuniculus* | GICACRRRFCPNSERFSGYCRVNGAR YVRCCSRR |
| 45 | NP-3B | *Oryctolagus cuniculus* | GRCVCRKQLLCSYRERRIGDCKIRGV RFPFCCPR |
| 46 | NP-4 | *Oryctolagus cuniculus* | VSCTCRRFSCGFGERASGSCTVNGG VRHTLCCRR |
| 47 | NP-5 | *Oryctolagus cuniculus* | VFCTCRGFLCGSGERASGSCTINGVR HTLCCRR |
| 48 | RatNP-1 | *Rattus norvegicus* | VTCYCRRTRCGFRERLSGACGYRGRI YRLCCR |
| 49 | Rat-NP-3 | *Rattus norvegicus* | CSCRYSSCRFGERLLSGACRLNGRIY RLCC |
| 50 | Rat-NP-4 | *Rattus norvegicus* | ACTCRIGACVSGERLTGACGLNGRIY RLCCR |
| 51 | GPNP | Guinea pig | RRCICTTRTCRFPYRRLGTCIFQNRVY TFCC |
| 52 | beta defensin-3 | *Homo sapiens* | MRIHYLLFALLFLFLVPVPGHGGIINT LQKYYCRVRGGRC AVLSCLPKEEQIGKCSTRGRKCCRRK K |
| 53 | theta defensin-1 | *Macaca mulatta* | RCICTRGFCRCLCRRGVC |
| 54 | defensin CUA1 | *Helianthus annuus* | MKSSMKMFAALLLVVMCLLANEMG GPLVVEARTCESQSHKFKGTCLSDTN CANVCHSERFSGGKCRGFRRRCFCT THC |
| 55 | defensin SD2 | *Helianthus annuus* | MKSSMKMFAALLLVVMCLLANEMG GPLVVEARTCESQSHKFKGTCLSDTN CANVCHSERFSGGKCRGFRRRCFCT THC |
| 56 | neutrophil defensin 2 | *Macaca mulatta* | ACYCRIPACLAGERRYGTCFYMGRV WAFCC |
| 57 | 4 KDA defensin | *Androctonus australis hector* | GFGCPFNQGACHRHCRSIRRRGGYC AGLFKQTCTCYR |
| 58 | *defensins* | *Mytilus galloprovinci alis* | GFGCPNNYQCHRHCKSIPGRCGGYC GGXHRLRCTCYRC |
| 59 | defensin AMP1 | *Heuchera sanguinea* | DGVKLCDVPSGTWSGHCGSSSKCSQ QCKDREHFAYGGACH YQFPSVKCFCKRQC |
| 60 | defensin AMP1 | *Clitoria ternatea* | NLCERASLTWTGNCGNTGHCDTQCR NWESAKHGACHKRGN WKCFCYFNC |
| 61 | cysteine-rich cryptdin-1 homolog | *Mus musculus* | MKKLVLLFALVLLAFQVQADSIQNT DEETKTEEQPGEKDQAVSVSFGDPQ GSALQDAALGWGRRCPQCPRCPSCP SCPRC PRCPRCKCNPK |
| 62 | beta-defensin-9 | *Bos taurus* | QGVRNFVTCRINRGFCVPIRCPGHRR QIGTCLGPQIKCCR |
| 63 | beta-defensin-7 | *Bos taurus* | QGVRNFVTCRINRGFCVPIRCPGHRR QIGTCLGPRIKCCR |
| 64 | beta-defensin-6 | *Bos taurus* | QGVRNHVTCRIYGGFCVPIRCPGRTR QIGTCFGRPVKCCRRW |
| 65 | beta-defensin-5 | *Bos taurus* | QVVRNPQSCRWNMGVCIPISCPGNM RQIGTCFGPRVPCCR |
| 66 | beta-defensin-4 | *Bos taurus* | QRVRNPQSCRWNMGVCIPFLCRVG MRQIGTCFGPRVPCCRR |
| 67 | beta-defensin-3 | *Bos taurus* | QGVRNHVTCRINRGFCVPIRCPGRTR QIGTCFGPRIKCCRSW |
| 68 | beta-defensin-10 | *Bos taurus* | QGVRSYLSCWGNRGICLLNRCPGRM RQIGTCLAPRVKCCR |
| 69 | beta-defensin-13 | *Bos taurus* | SGISGPLSCGRNGGVCIPIRCPVPMRQ IGTCFGRPVKCCRSW |
| 70 | beta-defensin-1 | *Bos taurus* | DFASCHTNGGICLPNRCPGHMIQIGIC FRPRVKCCRSW |
| 71 | coleoptericin | *Zophobas atratus* | SLQGGAPNFPQPSQQNGGWQVSPDL GRDDKGNTRGQIEIQNKGKDHDFNA GWGKVIRGPNKAKPTWHVGGTYRR |
| 72 | beta defensin-3 | *Homo sapiens* | MRIHYLLFALLFLFLVPVPGHGGIINT LQKYYCRVRGGRCAVLSCLPKEEQI GKCSTRGRKCCRRKK |
| 73 | defensin C | *Aedes aegypti* | ATCDLLSGFGVGDSACAAHCIARGN RGGYCNSKKVCVCRN |
| 74 | defensin B | *mytilus edulis* | GFGCPNDYPCHRHCKSIPGRYGGYC GGXHRLRCTC |
| 75 | sapecin C | *Sarcophaga peregrina* | ATCDLLSGIGVQHSACALHCVFRGN RGGYCTGKGICVCRN |
| 76 | macrophage antibiotic peptide MCP-1 | *Oryctolagus cuniculus* | MRTLALLAAILLVALQAQAEHVSVSI DEVVDQQPPQAEDQDVAIYVKEHES SALEALGVKAGVVCACRRALCLPRE RRAG FCRIRGRIHPLCCRR |
| 77 | cryptdin-2 | *Mus musculus* | MKPLVLLSALVLLSFQVQADPIQNTD EETKTEEQSGEEDQAVSVSFGDREG ASLQEESLRDLVCYCRTRGCKRRER MNGT CRKGHLMYTLCC |
| 78 | cryptdin-5 | *Mus musculus* | MKTFVLLSALVLLAFQVQADPIHKTD EETNTEEQPGEEDQ AVSISFGGQEGSALHEELSKKLICYCR IRGCKRRERVFGT CRNLFLTFVFCCS |
| 79 | cryptdin 12 | *Mus musculus* | LRDLVCYCRARGCKGRERMNGTCR KGHLLYMLCCR |
| 80 | defensin | *Pyrrhocoris apterus* | ATCDILSFQSQWVTPNHAGCALHCVI KGYKGGQCKITVCHCRR |
| 81 | defensin R-5 | *Rattus norvegicus* | VTCYCRSTRCGFRERLSGACGYRGRI YRLCCR |
| 82 | defensin R-2 | *Rattus norvegicus* | VTCSCRTSSCRFGERLSGACRLNGRI YRLCC |
| 83 | defensin NP-6 | *Oryctolagus cuniculus* | GICACRRRFCLNFEQFSGYCRVNGAR YVRCCSRR |
| 84 | beta-defensin-2 | *Pan troglodytes* | MRVLYLLFSFLFIFLMPLPGVFGGISD PVTCLKSGAICHP VFCPRRYKQIGTCGLPGTKCCKKP |
| 85 | beta-defensin-2 | *Homo sapiens* | MRVLYLLFSFLFIFLMPLPGVFGGIGD PVTCLKSGAICHP VFCPRRYKQIGTCGLPGTKCCKKP |
| 86 | beta-defensin-1 | *Homo sapiens* | MRTSYLLLFTLCLLLSEMASGGNFLT GLGHRSDHYNCVSS GGQCLYSACPIFTKIQGTCYRGKAKC CK |
| 87 | beta-defensin-1 | *Capra hircus* | MRLHHLLLVLFFLVLSAGSGFTQGIR SRRSCHRNKGVCAL TRCPRNMRQIGTCFGPPVKCCRKK |
| 88 | beta defensin-2 | *Capra hircus* | MRLHHLLLALFFLVLSAGSGFTQGIIN HRSCYRNKGVCAP ARCPRNMRQIGTCHGPPVKCCRKK |
| 89 | defensin-3 | *Macaca mulatta* | MRTLVILAAILLVALQAQAEPLQART DEATAAQEQIPTDNPEVVVSLAWDE SLAPKDSVPGLRKNMACYCRIPACL AGER RYGTCFYRRRVWAFCC |
| 90 | defensin-1 | *Macaca mulatta* | MRTLVILAAILLVALQAQAEPLQART DEATAAQEQIPTDNPEVVVSLAWDE SLAPKDSVPGLRKNMACYCRIPACL AGER RYGTCFYLGRVWAFCC |
| 91 | neutrophil defensin 1 | *Mesocricetus auratus* | VTCFCRRRGCASRERHIGYCRFGNTI YRLCCRR |
| 92 | neutrophil defensin 1 | *Mesocricetus auratus* | CFCKRPVCDSGETQIGYCRLGNTFYR LCCRQ |
| 93 | Gallinacin 1-alpha | *Gallus gallus* | GRKSDCFRKNGFCAFLKCPYLTLISG KCSRFHLCCKRIW |
| 94 | defensin | *Allomyrina dichotoma* | VTCDLLSFEAKGFAANHSLCAAHCL AIGRRGGSCERGVCICRR |
| 95 | neutrophil cationic peptide 1 | *Cavia porcellus* | RRCICTTRTCRFPYRRLGTCIFQNRVY TFCC |

The antimicrobial polypeptide can be a defensin. The compositions described herein can comprise one or more defensins. The antimicrobial polypeptide defensin can be BNP1 (also known as bactanecin and bovine dodecapeptide). The defensin may comprise the following consensus sequence: X₁CN₁CRN₂CN₃ERN₄CN₅GN₆CCX₂, wherein N and X represent conservatively or nonconservatively substituted amino acids and N₁ = 1, N₂ = 3 or 4, N₃ = 3 or 4, N₄ = 1, 2, or 3, N₆ = 5-9, X₁ and X₂ may be present, absent, or equal from 1-2. Representative defensins are provided in Tables 1 and 2.

| **Table 2 Defensins** | | | |
|---|---|---|---|
| **SEQ ID NO** | **Name** | **Organism** | **Sequence** |
| 38 | HNP-1 | Human | ACYCRIPACIAGERRYGTCIYQGRLWA FCC |
| 39 | HNP-2 | Human | CYCRIPACIAGERRYGTCIYQGRLWAF CC |
| 40 | HNP-3 | Human | DCYCRIPACIAGERRYGTCIYQGRLWA FCC |
| 41 | HNP-4 | Human | VCSCRLVFCRRTELRVGNCLIGGVSFTY CCTRV |
| 42 | NP-1 | Rabbit | VVCACRRALCLPRERRAGFCRIRGRIHP LCCRR |
| 43 | NP-2 | Rabbit | VVCACRRALCLPLERRAGFCRIRGRIHP LCCRR |
| 44 | NP-3A | Rabbit | GICACRRRFCPNSERFSGYCRVNGARY VRCCSRR |
| 45 | NP-3B | Rabbit | GRCVCRKQLLCSYRERRIGDCKIRGVR FPFCCPR |
| 46 | NP-4 | Rabbit | VSCTCRRFSCGFGERASGSCTVNGVRH TLCCRR |
| 47 | NP-5 | Rabbit | VFCTCRGFLCGSGERASGSCTINGVRHT LCCRR |
| 48 | RatNP-1 | Rat | VTCYCRRTRCGFRERLSGACGYRGRIY RLCCR |
| 49 | Rat-NP-3 | Rat | CSCRYSSCRFGERLLSGACRLNGRIYRL CC |
| 50 | Rat-NP-4 | Rat | ACTCRIGACVSGERLTGACGLNGRIYR LCCR |
| 51 | GPNP | Guinea pig | RRCICTTRTCRFPYRRLGTCIFQNRVYTF CC |

In general, defensins are a family of highly cross-linked, structurally homologous antimicrobial peptides found in the azurophil granules of polymorphonuclear leukocytes (PMN's) with homologous peptides being present in macrophages. *(See e.g.,* Selsted et al., Infect. Immun., 45:150-154 [1984]). Originally described as "Lysosomal Cationic Peptides" in rabbit and guinea pig PMN (Zeya et al., Science, 154:1049-1051 [1966]; Zeya et al., J. Exp. Med., 127:927-941 [1968]; Zeya et al., Lab. Invest., 24:229-236 [1971]; *Selsted et al.,* [1984], supra.), this mixture was found to account for most of the microbicidal activity of the crude rabbit PMN extract against various microorganisms (Zeya *et al.,* [1966], supra; Lehrer et al., J. Infect. Dis., 136:96-99 [1977]; Lehrer et al., Infect. Immun., 11:1226-1234 [1975]). Six rabbit neutrophil defensins have been individually purified and are designated NP-1, NP-2, NP-3A, NP-3B, NP-4, and NP-5. Their amino acid sequences were determined, and their broad spectra of activity were demonstrated against a number of bacteria (Selsted et al., Infect. Immun., 45:150-154 [1984]), viruses (Lehrer et al., J. Virol. 54:467 [1985]), and fungi (Selsted et al., Infect. Immun., 49:202-206 [1985]; Segal *et al.,* 151:890-894 [1985]). Defensins have also been shown to possess mitogenic activity (*e.g.*, Murphy et al., J. Cell. Physiol., 155:408-13 [1993]).

Four peptides of the defensin family have been isolated from human PMN's and are designated HNP-1, HNP-2, HNP-3, and HNP-4 (Ganz et al., J. Clin. Invest., 76:1427-1435 [1985]; Wilde et al., J. Biol. Chem., 264:11200-11203 [1989]). The amino acid sequences of HNP-1, HNP-2, and HNP-3 differ from each other only in their amino terminal residues, while each of the human defensins are identical to the six rabbit peptides in 10 or 11 of their 29 to 30 residues. These are the same 10 or 11 residues that are shared by all six rabbit peptides. Human defensin peptides have been shown to share with the rabbit defensins a broad spectrum of antimicrobial activity against bacteria, fungi, and enveloped viruses (Ganz *et al.,* [1985], *supra*).

Three defensins designated RatNP-1, RatNP-2, and RatNP-4, have been isolated from rat. (Eisenhauer et al., Infection and Immunity, 57:2021-2027 [1989]). A guinea pig defensin (GPNP) has also been isolated, purified, sequenced and its broad spectrum antimicrobial properties verified (Selsted et al., Infect. Immun., 55:2281-2286 [1987]). Eight of its 31 residues were among those invariant in six rabbit and three human defensin peptides. The sequence of GPNP also included three nonconservative substitutions in positions otherwise invariant in the human and rabbit peptides. Of the defensins tested in a quantitative assay HNP-1, RatNP-1, and rabbit NP-1 possess the most potent antimicrobial properties, while NP-5 possesses the least amount of antimicrobial activity when tested against a panel of organisms in stationary growth phase. (Selsted et al., Infect. Immun., 45:150-154 [1984]; Ganz et al. J. Clin. Invest. 76:1427-1435 [1985]). Defensin peptides are further described in US 4,543,252; 4,659,692; and 4,705,777).

The compositions described herein may comprise one or more defensins selected from the group consisting of SEQ ID NOs: 37-95.

Suitable antimicrobial peptides may comprise all or part of the amino acid sequence of a known peptide, more preferably incorporating at least some of the conserved regions identified in Table 2. The antimicrobial peptides may incorporate at least one of the conserved regions, more usually incorporating two of the conserved regions, preferably conserving at least three of the conserved regions, and more preferably conserving four or more of the conserved regions. The antimicrobial peptides may comprise fifty amino acids or fewer, although there may be advantages in increasing the size of the peptide above that of the natural peptides in certain instances. The peptides may have a length in the range from about 10 to 50 amino acids, preferably being in the range from about 10 to 40 amino acids, and most preferably being in the range from about 30 to 35 amino acids which corresponds generally to the length of the natural defensin peptides.

Described herein are antibodies (or portions thereof) fused to biocidal molecules (*e.g.*, lysozyme) (or portions thereof) suitable for use with processed food products as a whey based coating applied to food packaging and/or as a food additive. The compositions described herein may be formulated for use as disinfectants for use in food processing facilities. Further described herein are human and animal therapeutics.

### V. Applications

The methods and compositions of the present invention find use in a variety of applications, including, but not limited to, those described below.

### A. Exemplary Target Pathogens

### i) Escherichia coli O157:H7

Described herein are effective therapeutic treatments and prophylactic methods for combating the foodborne pathogen *E. coli* 0157:H7. *E. coli* 0157:H7 is a common component of the normal flora of the bovine gastrointestinal tract. Surveys of clinically normal cattle detect *E. coli* O157:H7 shedding in the feces of about 1-25% of animals. (D.D. Hancock DD et al., Epidemiol. Infect., 113(2):199-207 [1994]; MMWR Morb. Mortal. Wkly. Rep., 48(36):803-805 [1999]; and National Dairy Heifer Evaluation Project, USDA APHIS NAHMS [1994]). Human infection by *E. coli* 0157:H7 most often occurs through the fecal-oral route, either directly through handling of infected cattle, or more commonly as a result consuming contaminated meat products. Small-scale outbreaks of *E. coli* 0157:H7 disease have been associated with petting zoos and agricultural fairs. *(See e.g.,* G.C. Pritchard et al., Vet. Rec., 147:259-264 [2000]). Larger outbreaks of *E*. *coli* 0157:H7 disease have been traced to the widespread dissemination and consumption of contaminated meat products such as ground beef. (J. Tuttle et al., Epidemiol. Infect., 122:185-192 [1999]).

Contamination of meat products with fecal matter harboring *E. coli* 0157:H7 appears to occur primarily during slaughterhouse dehiding, evisceration, splitting, chilling, and fabrication operations. Further dissemination of *E*. *coli* 0157:H7 to otherwise uncontaminated meat products also occurs during grinding, processing, and transportation of meat products. Because of the severity of *E*. *coli* 0157:H7 disease and the potential for the contamination of large quantities of otherwise wholesome meat by a relatively small amount of contaminated meat, the recalls issued for potentially contaminated meat products are often very large.

Recent *E*. *coli* 0157:H7 contaminated meat recalls, include the recall of 24 million pounds of ground beef by the Hudson Beef in 1997, and most recently the recall of 19 million pounds of ground beef by the ConAgra Beef Company in July 2002. Recalls of this magnitude are obviously very costly; not only for actual value of the beef being destroyed, but also the logistical effort required to collect and dispose of the contaminated beef. More importantly, immeasurable costs arise from decreased consumer confidence in the meat packing industry and in the wholesomeness of food supply generally.

*E. coli* O157:H7 is particularly pathogenic because it produces a multi-unit verotoxin (or a shiga-like toxin) protein that binds receptors in the kidney and gastrointestinal tract of man. This toxin produces a hemolytic uremic syndrome in children and the elderly, and a hemorrhagic colitis in adults. The Center for Disease Control reported over 70,000 cases of *E*. *coli* O157:H7 disease each year and 60 deaths annually. The symptoms of hemorrhagic colitis last an average of 8 days. This implies that over half a million work days are lost per year due to *E*. *coli* O157:H7 infection.

*E. coli* O157:H7 disease is a costly and frustrating zoonosis, in part because its epidemiology is well understood yet very difficulty to prevent. The recent massive dissemination of the organism I contaminated meat products is a function of the industrialization processes that are essential to providing affordable food.

### ii) Listeria monocytogenes

Ingestion of the bacterium *Listeria monocytogenes* probably occurs quite often, as the bacteria has been isolated in food products worldwide. (B. Lorber, Clin. Infect. Dis., 24:1-11 [1997]; and J.M. Farber and P.I. Peterkin, Microbiol. Rev., 55:476-511 [1991]). Development of Listeriosis, the invasive disease caused by *L. monocytogenes* ingestion, is determined primarily by the integrity of the host's immune system (predominantly cell-mediated immune defects) and possibly also by inoculum size. (*See e.g.,* P. Aureli et al., New Engl. J. Med., 342:1236-1241 [2000]). *L. monocytogenes* crosses the mucosal barrier of the intestines and invades the bloodstream. The bacterium may disseminate to any organ, but has a clear predilection for the placenta and central nervous system (CNS), thus determining the main clinical syndromes caused by infection. Listeriosis is life-threatening zoonosis, especially in human fetuses and neonates, the elderly, and patients with certain predisposing conditions. Many cases of Listeriosis probably go unreported especially in perinates and newborns.

*L. monocytogenes* is a difficult bacterium to control because it thrives in vacuum packed food products and at temperatures typically used in food refrigeration. (S. Liu et al., Appl. Environ. Microbiol., 68(4):1697-1705 [2002]). Thus, *L. monocytogenes* is a particular problem in ready to eat foods that consumers expect are safe to eat with no further cooking. Typical food products contaminated with *L. monocytogenes* include unpasteurized or low acid dairy products and ready-to-eat (RTE) meat products such as luncheon meat and pates.

Many of the larger listeriosis outbreaks have been associated with fresh dairy products, especially Mexican soft cheeses and other non-aged or fermented cheese products. (M.J. Linnan et al., New Eng. J. Med., 319:823-828 [1988]). In specialty cheese production, *L. monocytogenes* has been found to accumulate in ripening rooms. (S.I. Pak *et al., supra*). Outbreaks have also been linked to processed and deli meat products, including turkey hot dogs, pate, and jellied tongue. (*See e.g.,* C. Jacquet et al., Appl. Env. Microbiol., 61:2242-2246 [1995]; J. McLaughlin et al., Brit Med. J., 303:773-775 [1991]; and Morbidity and Mortality Weekly Reports, 47:1085-1086 [1998]). *L. monocytogenes* is however, easily destroyed by cooking. As Listeria is heat labile, food products that cooked prior to eating and served hot have a lower risk profile.

The bacterium often contaminants food processing equipment, where it is typically found as a biofilm on steel and glass parts. *L. monocytogenes* tends to form biofilms on containers used to store food products. (A.C. Wong, J. Dairy Sci., 81(10):2765-2770 [1998]). It is also a common environmental contaminant of food storage facilities. *(See e.g*., M.S. Chae and H. Schraft, Int. J. Food. Microbiol., 62:103-111 [2000]). Thus, a pathogen originally considered an "environmental" organism on farms has invaded industrial food preparation facilities. Strains of *L. monocytogenes* have emerged with progressive resistance to antimicrobial agents. (C. Arizcun et al., J. Food Prot., 61:731-734 [1988]). Contamination of food processing equipment and storage often seed small amounts of bacteria onto food products which multiply during refrigeration. The length of time food is kept refrigerated, both at the retail outlet and in home, and the actual storage temperatures influence the risk that even low levels of initial *L. monocytogenes* contamination will grow to become problematic. Outbreaks of listeriosis have been associated with breakdowns in the environmental controls at food processing facilities (e.g., during plant renovation). Given the capacity of *L. monocytogenes* to grow under refrigeration, even very low levels *of L. monocytogenes* contamination in food products as they leave the processor can ultimately result in inoculums large enough to cause lethal infections in the consumer. Consequently, the present invention contemplates controlling foodborne listeriosis requires focusing on post-processing food handling and storage.

*L. monocytogenes* is recognized as an apparent and inapparent infection of livestock and is widespread in agricultural environments. (*See e.g.,* L. Hassan et al., J. Dairy Sci., 83(11):2441-2447 [2000]). *L. monocytogenes* is widespread in the environment and shed in the feces and milk of inapparently infected cattle. (L. Hassan *et al., supra).* Even low levels of *L. monocytogenes* contamination are enough to support the continued growth of the organism and are the primary source of human listeriosis.

Listeriosis is a serious disease. In milder its forms, listeriosis is a febrile illness with gastrointestinal signs but often progresses to bacteremia and meningitis with nervous system clinical manifestations including headache, loss of balance, and convulsions. Incubation periods can be several weeks making epidemiologic investigation more difficult. Listeriosis is particularly serious for pregnant women, infants, and individuals with compromised immune systems. (*See e.g*., A. Schuchat et al., Clinical Microbiol. Rev., 4:169-183 [1991]). Individuals with Acquired Immune Deficiency Syndrome (AIDS) are almost 300 times more likely to contract listeriosis than people with normally functioning immune systems. (J.G. Morris and M. Potter, Emerging Infectious Diseases, 3:435-441 [1997]). Diabetics and those affected by cancer and kidney disease are also at greater risk of contracting Listeriosis and are more prone to serious nervous system manifestations of the disease. Listeriosis in pregnant women can result in premature birth, still births, or birth of a critically infected child. Perinates and newborns are particularly at risk as the relative immaturity of their immune systems has been shown to contribute to the severity of disease (*See e.g.,* L. Slutzker and A. Schuchat, Listeriosis in humans. In: Listeria, Listeriosis and Food Safety, E.T. Ryser and E.M. Marth eds. Marcel Dekker Inc., New York, New York pp. 75-95 [1999]. Human foodborne listeriosis tends to result in either sporadic cases or epidemics depending on whether a common food source infects a group of people (H.R. Ibrahim et al., FEBS Lett., 506(1):27-32 [2001]). Contaminated meats, seafood, vegetables, fruits, and dairy products have all been the source of sporadic cases of listeriosis (S.I. Pak et al., Prev. Vet. Med., 53(1-2):55-65 [2002]).

### iii) Cryptosporidium parvum

*Cryptosporidium parvum* is a zoonotic apicomplexan parasite recognized as the cause of large outbreaks of acute diarrheal disease. The disease caused by *Cryptosporidium* infection is called cryptosporidiosis. Cryptosporidiosis has emerged as an important opportunistic infection in patients infected with HIV. With the advent of more effective HIV therapies, the association between *Cryptosporidium* infection and HIV has lessened in the US, however opportunistic *Cryptosporidiousis* following infection by HIV continues to be a major problem in developing countries.

*Cryptosporidium* is also recognized as a leading cause of traveler's diarrhea. An acutely debilitating diarrheal disease, accompanied by stomach cramps, cryptosporidiosis typically lasts 2-10 days. In the otherwise healthy host, cryptosporidiosis is rarely fatal, but deaths occur among the immunocompromised including AIDS patients, chemotherapy patients, malnourished individuals, and the elderly, who may become chronically diarrheic and in whom the parasite may establish hard-to-eliminate hepatobiliary and pancreatic infections.

*C. parvum* infects cattle and other livestock usually within the first few hours or days of life. Infected animals can become long-term shedders of *C*. *parvum* oocysts. C. *parvum* is an economically important cause of diarrheal disease and mortality among calves which provide a significant reservoir for human infection. In swine, clinical disease cryptosporidiosis is less common, but *C. parvum* has been recognized as a highly prevalent contaminant of swine manure holding facilities.

*C. parvum* oocysts can survive for extended periods of time in water and soil contaminated from human or animal fecal shedding. The oocysts are not inactivated by chlorination, nor removed by many water filtration systems. Drinking water, recreational water contact, and fecally contaminated foodstuffs are the principal sources of infection for humans. Type 1 and 2 *C*. *parvum* genotypes are epidemiologically and genetically distinct, although overlap occurs and heterogeneous infections can occur. Type 1 is transmitted from human to human, while type 2 is zoonotic and transmitted between cattle and other livestock, and humans. While genetic polymorphisms occur in both type 1 and 2 isolates, the extent of epitope homology between the genotypes and cross-protection is not fully understood. Dual infections may occur, but with no reproductive mixing of the genotypes. Nevertheless, three functionally defined antigens are conserved at the protein level between several type 1 and type 2 isolates. These antigens, CSL, P23 and GP25-200, were originally defined on type 2 isolates and previously shown to be the targets of neutralizing antibodies. The apparent conservation of antigens indicates that the compositions described herein using monoclonals antibodies to these antigens as neutralizing agents, either alone or to target a biocide to the parasite surface, will have application to both Type 1 and 2 infections. Further described herein are compositions comprising polyvalent antibody passive immunotherapies to treat epidemics of unknown origin.

Large outbreaks of human cryptosporidiosis demonstrate the potential for *Cryptosporidium* to be used as a bioterrorism agent. A 1993 outbreak of cryptosporidiosis in Milwaukee resulted in over 400,000 cases of clinical disease and several dozen deaths, following dissemination of *C*. *parvum* through the public water supply. The Milwaukee experience suggests that large waterborne or food borne outbreaks of cryptosporidiosis could be brought about by deliberate contamination. With the ratio of infective oocysts per gram of feces shed by an individual to the infective dose approximating one million to one (shedding 10⁶ or 10⁷ oocysts per gram, compared to an infective dose 10-100), the potential for producing major urban outbreaks is real.

*C. parvum* has several attributes that lend it for use as a potential bioterrorism agent: infectious oocysts are very hardy and easily transported; infective oocysts are shed in very large numbers but have a low infective dose; cryptosporidiosis is unlikely to be fatal to the terrorist handler; oocysts are readily available without access to reference collections or high security laboratories and can be easily propagated in neonatal ruminants (up to ∼10¹⁰ oocysts from a single calf); and widespread dissemination can be achieved in food or water. Given a high background incidence of *C*. *parvum* infections, an acute epidemic would be harder to trace back to a point source. Nevertheless, clinical signs are dramatic enough to cause panic, and to allow terrorist claims of responsibility to ring true. *C*. *parvum* thus fits the profile of an organism which might be deployed by a "low tech" terrorist group without access to a well-developed laboratory infrastructure.

The recent British detection of "ricin laboratories" indicate that low tech bioterrorism is today's reality. If the intent of bioterrorism is to produce mass hysteria rather than mass disease and fatalities, a few cases of cryptosporidiosis implicating contamination of a major food or water supplies could have a dramatic psychological effect.

Water distribution systems across the country are relatively difficult to secure. Security of water supply is also of concern in assuring a safe food supply. In an urban society dependent on complex food distribution chains, a point source contamination could affect people across a wide geographic area. For example, the recent nationwide recall of 28 million pounds of processed turkey due to *Listeria* contamination in a single processing plant illustrates that point source food contamination can have a very wide ripple effect. With only a moderate increment in microbiologic expertise, the effects of food bioterrorism could be devastating.

Dairy effluent is considered an important source of natural *C*. *parvum* infection, likewise, swine effluent is suspected as being reservoir for infection. Controlling infection in animal populations would help reduce the environmental risk of natural infections. Accordingly, certain embodiments of the present invention provide compositions to control zoonotic pathogens in animal reservoirs and agricultural environments.

Prior to the present, there were no effective parasite-specific drug therapies to control or curtail cryptosporidiosis in man or animals. Existing treatments are palliative and directed avoiding onset of dehydration. (S. Tzipori and H. Ward, Microbes Infect., 4:1047 [2002]). Naturally occurring cases of cryptosporidiosis in human and animal hosts with normal immunological systems can be severe, but are typically self limiting. (C.L. Chappell et al., Amer. J. Trop. Med. Hyg., 60:157-164 [1999]). Colostral antibodies fed to calves may limit infection and prevent clinical disease. Polyclonal hyperimmune antibodies raised against *C*. *parvum* may effectively limit clinical cases of the disease while allowing some active immunity to develop.

Described herein are antibody-based immunoprophylaxis and immunotherapy that effectively control acute *C*. *parvum* infections. The efficacy of compositions and methods of passive immunotherapy comprising administering antibodies specifically developed against neutralization-sensitive epitopes may be distinguishable from the host-produced antibodies in protection against natural infection, which depends on competent cell mediated immune responses (M. Riggs, Microbes Infect., 4:1067 [2002]).

Described herein are compositions and methods for administering passive immunotherapies against pathogens (*e.g., C*. *parvum* infections). Faced with a population exposed to deliberately contaminated food or water, or in a battle theater setting, a rapidly deployable, rapidly effective, passive immunotherapies are strategically and clinically very valuable. Described herein are orally administered monoclonal antibody compositions that specifically target pathogens (*e.g.*, parasites) and either prevent infection, or reduce an existing infection to subclinical levels and abbreviate existing clinical effects.

Described herein are monoclonal antibodies against defined apical complex and surface-exposed antigens to specifically neutralize infective stages of *C*. *parvum in vitro* and *in vivo.* Described herein are previously unavailable recombinant antibodies to *C*. *parvum.* Prior to the present disclosure high cost and inefficient production systems for recombinant and hybridoma monoclonals alike have generally removed widespread immunoprophylaxis and/or immunotherapies for cryptosporidiosis form serious clinical consideration.

Described herein is the use of an extensive bank of hybridoma lines directed to *cryptosporidial* antigens. A large number of *C*. *parvum* antigens of distinct function have been identified and characterized. (M.W, Riggs, Microbes. Infect., 4:1067 [2002]). Several antigens in particular have shown potential for independent targeting to neutralize sporozoite and merozoite infectivity, including, but not limited to, CSL, P23, and GP25-200. Briefly, CSL (∼1300 kDa) is an apical complex-derived glycoprotein expressed on the surface of sporozoite and merozoite infective stages. After antibody binding to CSL, sporozoites release the antigen in membranous antibody-CSL complexes and are rendered non-infective. (M.W. Riggs et al., J. Immunol., 158:1787-1795 [1997]). Since CSL has been shown to contain a ligand for a surface receptor on human intestinal epithelial cells *(See,* R.C. Langer and M.W. Riggs, Infect. Immun., 67:5282-5291 [1999]; and R.C. Langer et al., Infect. Immun., 69:1661-1670 [2001]), blocking of CSL is contemplated to account for the efficacy of anti-CSL antibodies in inhibiting sporozoite attachment. P23 (∼23 kDa) is a surface protein of sporozoites and merozoites believed to be involved in motility and invasion processes (*See,* L.E. Perryman et al., Vaccine, 17:2142-2149 [1999]). Monospecific antibodies to P23 have been shown to curtail disease in neonatal calves. (L.E. Perryman *et al., supra*). GP25-200 is a glycoprotein complex of variable size, found in the apical complex and on the surface of sporozoites and merozoites. (M.W. *Riggs et al., supra*). Schaefer *et al.* demonstrated that when hybridoma derived monoclonal antibodies to CSL, P23, and GP25-200 were applied singly, or in combination, significant sporozoite neutralization could be obtained. (D.A. Schaefer et al., Infect. Immun., 68:2608-2616 [2000]).

Optimal protection in neonatal mice can be achieved by combining three antibodies: 3E2 (IgM) to target CSL; 3H2 (IgM) to target GP25-200; and IE10 (IgG1) to target P23. When these three antibodies are orally administered, individually, in the neonatal mouse infection model, they are able to reduce intestinal infection by 50-60%. When these antibodies are administered as a polyvalent "cocktail" the three monoclonal antibodies reduced intestinal infection by 86-93%. Described herein are recombinant analogues of 3E2, 3H2, and 1E10 antibodies. Described herein are fusion proteins comprising cryptosporocidal enzymes and antibodies (*e.g*., IgG), or portions thereof, including, but not limited to, 3E2, 3H2, 1E10, and 4H9. 4H9 is a second antibody directed to GP25-200. It is an IgG1 antibody that is though to recognize a different epitope on GP25-200 than does 3H2. 4H9 may be able to reduce infection in neonatal mice by ∼50% when administered orally. Thus, compositions comprising 1E10 and 4H9 can be vehicles for delivering biocides to two different neutralization-sensitive molecules on the surface of sporozoites and merozoites.

Using a monoclonal antibody fusion protein to direct otherwise naturally occurring biocides to specific pathogenic organisms (*e.g., E. coli* O157:H7, *L. monocytogenes, Cryptosporidium parvum* and the like) can have wide applicability in human and animal health. Described herein are compositions to control other pathogenic feedlot organisms including, but not limited to, E. *coli* K99 in calves and E. *coli* K88 in piglets.

Other pathogens responsible for foodborne illnesses and other emerging infectious diseases may be controlled as a component of the Nation's food security and bioterrorism response. For example, potential foodborne bioterrorism agents such as, *Clostridium botulinum, Clostridium perfringens, Staphylococcus aureus, Staphylococcus epidermidis, Staphylococcus pneumoniae, Staphylococcus saprophyticus, Shigella dysenteriae, Salmonella typhi, Salmonella paratyphi, Salmonella enteritidis,* fungal agents and the like can be controlled.

### iii). Other Exemplary Target Pathogens

The methods and compositions disclosed herein are directed to specifically controlling (*e.g.*, therapeutic treatments or prophylactic measures) diseases caused by the following pathogens: *Bartonella henselae, Borrelia burgdorferi, Campylobacter jejuni, Campylobacter fetus, Chlamydia trachomatis, Chlamydia pneumoniae, Chylamydia psittaci, Simkania negevensis, Escherichia coli* (*e.g.,* O157:H7 and K88), *Ehrlichia chafeensis, Clostridium botulinum, Clostridium perfringens, Clostridium tetani, Enterococcus faecalis, Haemophilius influenzae, Haemophilius ducreyi, Coccidioides immitis, Bordetella pertussis, Coxiella burnetii, Ureaplasma urealyticum, Mycoplasma genitalium, Trichomatis vaginalis, Helicobacter pylori, Helicobacter hepaticus, Legionella pneumophila, Mycobacterium tuberculosis, Mycobacterium bovis, Mycobacterium africanum, Mycobacterium leprae, Mycobacterium asiaticum, Mycobacterium avium, Mycobacterium celatum, Mycobacterium celonae, Mycobacterium fortuitum, Mycobacterium genavense, Mycobacterium haemophilum, Mycobacterium intracellulare, Mycobacterium kansasii, Mycobacterium malmoense, Mycobacterium marinum, Mycobacterium scrofulaceum, Mycobacterium simiae, Mycobacterium szulgai, Mycobacterium ulcerans, Mycobacterium xenopi, Corynebacterium diptheriae, Rhodococcus equi, Rickettsia aeschlimannii, Rickettsia africae, Rickettsia conorii, Arcanobacterium haemolyticum, Bacillus anthracis, Bacillus cereus, Lysteria monocytogenes, Yersinia pestis, Yersinia enterocolitica, Shigella dysenteriae, Neisseria meningitides, Neisseria gorzorrhoeae, Streptococcus bovis, Streptococcus hemolyticus, Streptococcus mutans, Streptococcus pyogenes, Streptococcus pneumoniae, Staphylococcus aureus, Staphylococcus epidermidis, Staphylococcus pneumoniae, Staphylococcus saprophyticus, Vibrio cholerae, Vibrio parahaemolyticus, Salmonella typhi, Salmonella paratyphi, Salmonella enteritidis, Treponema pallidum,* among other bacteria.

### B. Biofilms

The methods and compositions of the present invention may target bacteria present as a biofilm. *Listeria monocytogenes* can form biofilms on a variety of materials used in food processing equipment and other food and non-food contact surfaces (Blackman, J Food Prot 1996; 59:827-31; Frank, J Food Prot 1990; 53:550-4; Krysinski, J Food Prot 1992; 55:246-51; Ronner, J Food Prot 1993; 56:750-8). Biofilms can be broadly defined as microbial cells attached to a surface, and which are embedded in a matrix of extracellular polymeric substances produced by the microorganisms. Biofilms are known to occur in many environments and frequently lead to a wide diversity of undesirable effects. For example, biofilms cause fouling of industrial equipment such as heat exchangers, pipelines, and ship hulls, resulting in reduced heat transfer, energy loss, increased fluid frictional resistance, and accelerated corrosion. Biofilm accumulation on teeth and gums, urinary and intestinal tracts, and implanted medical devices such as catheters and prostheses frequently lead to infections (Characklis WG. Biofilm processes. In: Characklis WG and Marshall KC eds. New York: John Wiley & Sons, 1990:195-231; Costerton et al., Annu Rev Microbiol 1995; 49:711-45).

Biofilm formation is a serious concern in the food processing industry because of the potential for contamination of food products, leading to decreased food product quality and safety (Kumar CG and Anand SK, Int J Food Microbiol 1998; 42:9-27; Wong, J Dairy Sci 1998; 81:2765-70; Zottola and Sasahara, Int J Food Microbiol 1994; 23:125-48). The surfaces of equipment used for food handling or processing are recognized as major sources of microbial contamination. (Dunsmore et al., J Food Prot 1981; 44:220-40; Flint et al., Biofouling 1997; 11:81-97; Grau, In: Smulders FJM ed. Amsterdam: Elsevier, 1987:221-234; Thomas et al., In: Smulders FJM ed. Amsterdam: Elsevier, 1987:163-180). Biofilm bacteria are generally hardier than their planktonic (free-living) counterparts, and exhibit increased resistance to antimicrobial agents such as antibiotics and disinfectants. It has been shown that even with routine cleaning and sanitizing procedures consistent with good manufacturing practices, bacteria can remain on equipment, food and non-food contact surfaces and can develop into biofilms. In addition, *L. monocytogenes* attached to surfaces such as stainless steel and rubber, materials commonly used in food processing environments, can survive for prolonged periods (Helke and Wong, J Food Prot 1994; 57:963-8). This would partially explain their ability to persist in the processing plant. Common sources *of L. monocytogenes* in processing facilities include equipment, conveyors, product contact surfaces, hand tools, cleaning utensils, floors, drains, walls, and condensate (Tomkin et al., Dairy, Food Environ Sanit 1999; 19:551-62; Welbourn and Williams, Dairy, Food Environ Sanit 1999; 19:399-401).

### C. Plant Pathogens

Further described herein are methods and compositions targeted towards plant pathogens. Plant fungi have caused major epidemics with huge societal impacts. The Irish potato famine, with its consequent economic disaster and human population displacement, was the result of the sudden introduction of the fungus *Phytophthora infestans.*

South American cocoa crops are under threat of two major fungal diseases: witches broom caused by *Crinipellis perniciosa* and frosty pod (*Moniliophthora roreri*), which together threaten the viability of the chocolate production industry in the western hemisphere.

Phytophthora blight, caused by the oomycete *Phytophthora capsici,* has become one of the most serious threats to production of cucurbits (cucumbers, squash, pumpkins) and peppers, both in the United States and worldwide (Erwin, D. C., and Ribeiro, O. K. 1996. Phytophthora Diseases Worldwide. American Phytopathological Society, St. Paul, MN).

Banana crops worldwide are affected by Black Sigatoka is caused by the ascomycete, *Mycosphaerella fijiensis. Fusarium* scab affects small grain crops (wheat and barley). Ganoderma spp fungi have produced deaths of ornamental palms, as do several species of Phytophthora (Elliott and Broschat, T. K. 2001. Palms 45:62-72; Nagata and Aragaki, M. 1989. Plant Dis. 73:661-663).

Plants are also affected by bacteria and viruses. *Burkholderia cepacia* is a bacterium which produces economic losses to onion crops (Burkholder 1950. Phytopathology 40:115-118). Numerous plant viruses cause significant crop losses worldwide. Exemplary of such plant viruses are soybean mosaic virus, bean pod mottle virus, tobacco ring spot virus, barley yellow dwarf virus, wheat spindle streak virus, soil born mosaic virus, wheat streak virus in maize, maize dwarf mosaic virus, maize chlorotic dwarf virus, cucumber mosaic virus, tobacco mosaic virus, alfalfa mosaic virus, potato virus X, potato virus Y, potato leaf roll virus and tomato golden mosaic virus.

### D. Sanitation

In yet other embodiments, the methods and compositions of the present invention find use in the sanitation of household and other areas. Listeria spp are common contaminants of the domestic environment. As many as 47% of households sampled were contaminated, with dishcloths, and drain areas being common sites of contamination. (Beumer et al., Epidemiol Infect. 1996 Dec;117(3):437-42).

Pseudomonas aeruginosa is frequently isolated from showers and baths and hot tubs (Zichichi et al., Int J Dermatol. 2000 Apr;39(4):270-3; Silverman and Nieland, J Am Acad Dermatol. 1983 Feb;B(2):153-6).

Where a family member has an infectious disease, transmission may occur through contamination of domestic objects (Barker et al., J Appl Microbiol. 2000 Jul;89(1):137-44).

Domestic food preparation and storage areas are also a source of bacterial food contaminants. The public is showing increasing awareness of the need to control domestic microbial contamination (Mattick et al., Int J Food Microbiol. 2003 Aug 25;85(3):213-26; Kusumaningrum et al., Int J Food Microbiol. 2003 Aug 25;85(3):227-36).

### E. Building structures

In other embodiments, the present invention provides methods of targeting building structures. There has been increasing public attention to the potential health risks of mold exposure, particularly in wet buildings. A variety of molds have been isolated from both damaged homes and businesses, including agents that secrete toxigenic materials. *Stachybotrys chartarum* is a fungus that has become notorious as a mycotoxin producer that can cause animal and human mycotoxicosis. Indeed, over the past 15 years in North America, evidence has accumulated implicating this fungus as a serious problem in homes and buildings and one of the causes of the "sick building syndrome." (Mahmoudi et al., J Asthma. 2000 Apr;37(2):191-8).

Legionella spp.bacteria replicate in manmade water containing structures, especially when these are heated, such as industrial cooling towers, heating and air conditioning systems. Legionnaires disease pneumonia is contracted by susceptible individuals that breathe water droplets from such sources. Preventive and remedial treatment of water containing structures is needed to eliminate the source of infection to building inhabitants (Shelton et al., AIHAJ. 2000 Sep-Oct;61(5):738-42).

### F. Military and Bioterrorism Applications

The methods and compositions of the present invention further find use in military and bioterrorism applications. For example, in some embodiments, the methods and compositions of the present invention are used in the decontamination of surfaces exposed to unknown bacteria (e.g., military equipment and personal protective gear).

The methods and compositions of the present invention further find use in combating unknown and drug resistant organisms. The prevalence of bacteria that resist standard antibiotic therapy is increasing rapidly. Furthermore, the ability to engineer organisms with multiple drug resistance to standard antibiotics creates a significant threat in bioweapons development. Because the broad spectrum antimicrobials of the present invention are suitable for use against broad classes of pathogens, they can respond to unknown bacteria and their bactericidal effect is independent of antibiotic resistance mechanisms.

### G. Medical Applications

The methods and compositions of the present invention additionally find use in the treatment of subjects (e.g., humans) infected with a microorganism (e.g., food borne pathogens). The methods and compositions of the present invention are particularly well suited for use against antibiotic resistant organisms are targeted by the methods and compositions of the present invention.

### DETAILED DESCRIPTION OF THE INVENTION

Described herein are constructs that encode novel microorganism targeting molecules (*e.g.*, innate immune receptors ligands or monoclonal antibodies), novel fusion proteins, and chimeric monoclonal antibodies and to methods of using and producing the same. Described herein are methods of producing novel monoclonal antibody biocide (*e.g.*, bactericidal enzymes) fusion proteins in transgenic animals (*e.g.*, bovines) and in cell cultures. Described herein are therapeutic and prophylactic methods of using monoclonal antibody biocide fusion proteins in health care (*e.g.*, human and veterinary), agriculture (*e.g*., animal and plant production), and food processing (*e.g*., beef carcass processing). Described herein are methods of using monoclonal antibody biocide fusion proteins in various diagnostic applications in number of diverse fields such as agriculture, medicine, and national defense.

Described herein is the production of novel monoclonal antibodies and chimeric monoclonal antibody fusion proteins in host cells containing multiple integrated copies of an integrating vector. Integrating vectors (*i.e.*, vectors that integrate via an integrase or transposase or have the capability to code for these enzymes) may be utilized to create cell lines containing a high copy number of a nucleic acid encoding a gene of interest. The transfected genomes of the high copy number cells are stable through repeated passages (*e.g.,* at least 10 passages, preferably at least 50 passages, and most preferably at least 100 passages). Furthermore, the host cells may be capable of producing high levels of protein (*e.g.*, more than 1 pg/cell/day, preferably more than 10 pg/cell/day, more preferably more than 50 pg/cell/day, and most preferably more than 100 pg/cell/day).

Described herein are methods for the production of transgenic non-human animals that express novel proteins in their tissues (*e.g.*, mammary glands). The transgenic animals may be non-human ruminant (*Ruminantia*) mammals. The transgenic animals may be ungulates. The mammals may be female ruminants (*e.g*., bovines) that preferentially express the novel proteins in their mammary glands. The novel protein compositions produced in the transgenic animals can be collected, purified, and subsequently incorporated into a variety of additional compositions (*e.g.,* food additives, pharmaceuticals, disinfecting agents, *etc*.) and/or used in a variety of therapeutic or prophylactic methods. The proteins of interest (the novel fusion proteins disclosed herein) can be mixed with colostrum (or colostrum substitute(s)) and subsequently feed to nursing feedlot animals (e.g., beef calves, piglets, lambs, kids, and the like). The proteins of interest can be formulated with one or more carriers (*e.g.*, whey) and used in the meat processing industry either a topical disinfecting agent applied to animal carcasses or as an edible supplement mixed into finished meat products. The proteins of interest (*e.g.*, novel monoclonal antibodies and chimeric monoclonal antibody fusion proteins disclosed herein) can be purified from the lactation of transgenic non-human animals and subsequently processed and formulated for administration to subjects (*e.g.*, humans and non-human animals) as therapeutic or prophylactic medicaments.

Described herein are methods for producing transgenic non-human animals by the introduction of exogenous DNA into pre-maturation oocytes and mature, unfertilized oocytes (*i.e*., pre-fertilization oocytes) using retroviral vectors that transduce dividing cells (*e.g*., vectors derived from murine leukemia virus [MLV], Moloney murine leukemia virus [MMLV], and the like). In addition, described herein are methods and compositions for cytomegalovirus promoter-driven, as well as, mouse mammary tumor LTR expression of various recombinant proteins. The present invention is not limited however to the aforementioned constructs, promoters, and other genetic elements. Indeed, the present invention provides numerous examples of contemplated genetic constructs (*e.g.*, retroviral vectors) and methods of producing stably transfected cell lines (*e.g.*, mammalian, amphibian, insect, and plant) and transgenic non-human animals (*e.g.*, bovines).

Retrovector transgenic technologies (described in greater detail herein) may be used to overcome problems inherent in earlier methods for creating transgenic mammals. Genes of interest (*e.g.*, genes encoding at least a portion of a recombinant antibody) can be introduced into unfertilized oocytes (*e.g.*, bovine oocytes). After entry of the retroviral RNA into the cell, and reverse transcription into DNA, the integration of the DNA provirus into the host cell genome is mediated by the retroviral integrase and specific nucleotide sequences at the ends of the retroviral genome. By introducing genes to the oocyte (*e.g.*, a bovine oocyte) at metaphase II arrest, the vector has access to the oocyte DNA when there is no nuclear membrane in place. This technology may negate the need for dividing cells for retroviral integration to occur. Depending on the conditions, such integrations can occur at one or several independent sites in the genome and are transmitted in standard Mendelian patterns upon subsequent animal (*e.g.*, bovine) breeding. The integrated gene is transcribed like other indigenous cell genes, and the proteins it encodes are expressed at high levels. The retrovector backbone used may lack the genes essential for viral structure and enzyme functions, therefore the retroviral constructs are replication defective. Constructs that preclude the need for a selectable may be used marker. Importantly, the removal of selectable markers (*e.g.*, antibiotic-dependent selection markers) may provide a significant advantage, especially upon consideration of regulatory requirements for transgenic livestock.

The contemplated approach may have major advantages. For example, the efficiency of transgenic live births using the contemplated transgenic methods is high *e.g.*, from about 25% -75% of animals (cattle) born when genes without a selection marker are used. Additionally, retroviral genes may insert as single copies, thus decreasing the risk of genetic instability upon subsequent cell replication, which tends to splice out tandem repeats of genes typical in pronuclear injection and nuclear transfer technologies. Transgenes may be inserted prior to fertilization to reduce the risk of producing mosaic animals, which are only transgenic in some tissues but not all.

### I. Production of Recombinant Antibodies

Described herein is obtaining hybridoma cell lines that produce monoclonal antibodies against particular pathogens of interest (*e.g., E. coli* strain O157:H7) from one or more sources (e.g., ATCC). The cell lines are subsequently used to isolate the heavy and light chain genes that encode for pathogen (*e.g., E. coli* O157:H7 and *Listeria monocytogenes*) specific monoclonal antibodies according to standard molecular biology methods.

For example, hybridoma cell line ATCC HB 10452, which makes monoclonal antibody 4E8C12 specific for *E*. *coli* O157:H7 and O26:H11, are grown according to the depositors instructions. The cells are maintained in cRPMI at 37 °C and 5% CO₂ atmosphere and split biweekly at a 1:10 ratio. In another example, monoclonal antibody hybridomas to the pathogen *L. monocytogenes* from the cell line ATCC 4689-4708 are likewise grown according to the depositors instructions. Candidate monoclonal antibodies may be chosen based upon their binding affinity to the pathogen of interest (*e.g., L. monocytogenes*) as well as their binding specificity that in certain instances includes as many different pathogen serotypes as possible. Candidate monoclonals may preferably show no or only weak cross-reaction with other species of bacteria and mammalian cells.

These cultures are expanded and grown in roller bottles under the described conditions to allow production of approximately milligram amounts of purified monoclonal antibodies. The antibodies may be purified using any suitable protocol such as ammonium sulfate precipitation. The purified monoclonal antibodies may be used to perform various *in vitro* functionality tests. Purified monoclonal antibodies can be used to perform affinity and specificity tests in order to select for the antibodies that have the best binding properties to the surface of the pathogen of interest (*e.g., L. monocytogenes*) and/or that include binding to a broad range of serotypes. Contemplated functionality tests include, but are not limited to, enzyme-linked immunosorbent assays (ELISA) and competitive ELISA assays. Varying concentrations of different monoclonal antibodies may be allowed to bind to immobilized heat killed pathogens *(e.g., L. monocytogene*). Using a competitive assay, various concentrations of competing antigen can be added to the wells of test plate and the binding of the monoclonal antibodies is measured. Quantitative immunofluorescence assays can be used to allow the determination of binding affinity based on fluorescence intensity per cell. Determining the affinity of the monoclonal antibodies based on their binding capacity to the pathogen of interest (*e.g., L. monocytogenes*), may allows the selection of the one monoclonal antibody that is best for topical applications against viable pathogens.

Cells from the highest affinity hybridoma clone will be used to extract total RNA with the purpose of isolating the monoclonal antibody-specific heavy and light chain gene transcripts. Upon total RNA extraction, the RNA is reverse transcribed using standard molecular biology kits and protocols, such as the RIBOCLONE cDNA synthesis system from Promega (Promega Corp., Madison, WI). Preferably, the procedures used create double stranded cDNA of all RNA transcripts in a cell, including the transcripts from the murine heavy and light chain genes. The total cDNA is used as a template to specifically amplify the mouse IgG2a heavy chain and the Igk light chain. Site-directed mutagenesis primers are used to amplify these sequences. These primers add short sequences of DNA, and introduces suitable restriction sites thus allowing direct cloning of the product into the retrovector backbone.

Once the genes for the murine heavy and light chain have been isolated, they are separated by an IRES element and inserted into the retrovector expression system under the control of the simian cytomegalovirus and the bovine alpha-lactalbumin promoter. The genes for the murine heavy and light antibody chains may be cloned into the GPEX gene product expression system under the control of the simian cytomegalovirus (sCMV) promoter (Gala Design, Inc., Middleton, WI) or other suitable multigenic gene expression systems. This process allows for the production of cell lines that secrete high levels of the monoclonal antibodies.

In particular, the heavy chain followed by an internal ribosome entry site (IRES) element are cloned into the retrovector backbone at the same site. Similarly, the light chain is then cloned into the retrovector backbone. Once the retroviral construct is complete, quality control sequencing will confirm that all the elements are present. The use of the IRES element in between heavy and light chain genes may yield fully functional antibodies expressed and secreted into the medium at exceptionally high levels (*e.g*., >100pg/cell/day in CHO cells). After the retroviral constructs are complete, quality control sequencing can be used to confirm that all the elements are present. The retrovector construct are then used to transform host cells along with the plasmid that encodes the vesicular stomatitis virus glycoprotein (VSV-G) used for pseudotyping the retrovirus. This procedure creates intermediate level viral titer that is used to infect production cell lines (*e.g.*, 293H or CHO cells). The population of transduced cells are subjected to clonal selection based on the antibody levels present in the medium supernatant. The clone with the highest level of antibodies secreted into the supernatant is selected to produce milligram amounts of murine monoclonal antibody 4E8C12. The recombinant antibodies can be purified from cell supernatants using standard techniques well known to those in the art.

Figure 1 shows one retroviral construct for expression of murine and chimeric bovine murine antibodies with lysozyme.

### II. Production of Recombinant Chimeric Antibodies

The bovine IgG1 and IgG2 heavy chain genes can be used to modify the constructs made above to produce constructs encoding chimeric bovine-murine antibodies. For example, the constant portion of the murine heavy chain gene can be replaced with the constant portion of the bovine heavy chain gene to create a chimeric bovine-murine monoclonal antibodies. A suitable bovine heavy chain IgG1 sequence may be selected from, but is not limited to, the following GenBank Accession Numbers: BD105809; S82409; U32264; U32263; U32262; U32261; U32260; U32259; U32258; U32257; U32256; U32255; U32254; U32253; U32252; U32251; U32250; U32249; U34749; U34748; U32852; U32851; U32850; U36824; U36823; S82407; X62917; X62916; and X16701. Likewise, a suitable bovine heavy chain IgG2 sequence may be selected from, but is not limited to, the following GenBank Accession Numbers: S82409; S82407; Z37506; and X16702. In one example, GenBank Accession No. S282409 (SEQ ID NO: 1) provides bovine IgG1/IgG2 sequences. *(See,* I. Kacskovics and J.E. Butler, Mol. Immunol., 33(2):189-195 [1996]). Preferably, the murine IgG2a heavy chain gene will be replaced by the bovine sequence for IgG1 or IgG2a. Thus, modified with bovine IgG1/IgG2 sequences, the vectors described above are used in subsequent cloning steps.

Following sequence analysis of the construct, the constructs can be used to create vectors for the transduction of production cell lines (*e.g.*, 293H) and packaging cell lines (*e.g.*, 293gp). Standard clonal analysis techniques are used to select for clones that produce high levels of the bovine-murine chimeric antibody. Once a top clone has been selected, enough chimeric antibody will be produced from this clone to conduct functionality tests with the derived chimeric monoclonal antibody.

Production cell lines that secrete high levels of the monoclonal antibodies can be made from the above-mentioned constructs. The retroviral construct containing the chimeric murine-bovine monoclonal antibody genes are used to transduce at least one production cell line (*e.g.*, the 293H production cell line). Upon transduction and expansion, the cell pool is subjected to limited dilution cloning to select for clones that produce high levels of the chimeric monoclonal antibody as determined by standard assay techniques (*e.g.*, ELISA assays). One of the top clones is used to produce chimeric murine-bovine monoclonal antibodies in milligram amounts that are subsequently used in the functionality tests described below.

Described herein is the production of retrovector packaging cell lines that produce high titers of retrovector containing the gene for the monoclonal antibodies in preparation for making transgenic animals, such as bovines. For example, the retrovector construct containing the chimeric murine-bovine monoclonal antibody genes are used to transduce a packaging cell line (*e.g*., 293gp packaging cell line). The transduced packaging cell pool is then subjected to limiting dilution cloning and clones that produce the highest infectious viral titers are used for virus production. After a thorough quality control of the top virus titer producing clone, which ensures that the construct is complete, an appropriate amount of pseudotyped virus are purified and cryopreserved for use in oocyte injections.

### III. Production of Pathogen Specific Monoclonal Antibodies in a Multigenic Expression System

The production of *L. monocytogenes*-specific monoclonal antibody can be conducted in the GPEX gene product expression system (Gala Design, Inc., Middleton, WI). In an initial step, the transduced production cell pool is subjected to clonal analysis to select the top antibody producing clones. Preferably, the retrovector construct will be used to transform host cells along with the plasmid that encodes the vesicular stomatitis virus glycoprotein (VSV-G) used for pseudotyping the retrovirus. This procedure creates intermediate level viral titer used to infect production cell lines (*e.g*., 293H and CHO cells among others). The population of transduced cells is then subjected to a clonal selection, based on antibody levels present in the medium supernatant.

The selected clones can be then expanded and used to produce sufficient quantities of monoclonal *L. monocytogenes*-specific antibodies to perform one or more functionality studies similar to those mentioned above.

The clone with the highest level of antibody secreted into the supernatant is then chosen to produce milligram amounts of recombinant murine monoclonal antibody against *L. monocytogenes.* Additional experiments with the purified monoclonal antibodies, similar to those mentioned above are contemplated. The objective of these experiments is to determine whether the production of the selected high-affinity monoclonal antibody affects the binding capacity when compared to the original hybridoma-derived antibody.

### IV. Comparison of Murine and Chimeric Murine-bovine Antibodies

Described herein is additional functionality testing of the purified murine monoclonal antibody as compared to the hybridoma-derived product. For example, a number of tests are conducted to demonstrate that the 4E8C12 monoclonal is highly specific for *E*. *coli* strain 0157:H7 and strain 026:H11 and no other related strains or species. The assays contemplated for determining the specific bactericidal activity may be divided into two phases. First, the bactericidal activity of the monoclonal antibody and fusion proteins are tested *in vitro* for inactivation of the pathogenic strain (*e.g., E. coli* 0157:H7). Second, the monoclonal antibody and fusion proteins are evaluated by adding to formulations in turkey slurries.

In particular, to assess *in vitro* inactivation, *E*. *coli* 0157:H7 (five food and outbreak isolates) are grown in trypticase soy broth (TSB) until late log phase (~24 h). The cells are harvested by centrifugation, washed in 67 mM sodium phosphate buffer, pH 6.6 (PB), and strains mixed in approximately equal concentrations. The *E*. *coli* mixture is then added to a level of 105 per ml to PB. The monoclonal conjugates are added starting at concentrations that correspond to the bactericidal concentration of lysozyme and phospholipas A2 alone and down at least 3 logs. The suspensions are incubated at 4° and 10° C and cell viability determined at 0,1, 4, 8 and 24 h by direct plating on TSB and MacConkey sorbitol agars. The cell suspensions are examined microscopically for clumping. If clumping is observed, further experimental techniques are used to separate the cells (*e.g*., addition of surfactants, such as Tween 80, changing pH, and mild sonication). Controls without added monoclonal conjugates are also contemplated for testing. All conditions are tested in triplicate and standard deviations of viability are determined.

Cooked, uncured, and unsmoked turkey breast can be obtained from a manufacturer. Slurries of this meat product are prepared as described by Schlyter et al., Int. J. Food Microbiol., 19(4):271-281 (1993) adjusted to the appropriate brine content, and pasteurized to 68°C. Two levels of filter-sterilized monoclonal conjugates, depending on *in vitro* results, are added to the slurries after pasteurization Flasks are cooled to 4°C and subsequently inoculated with *E. coli* 0157:H7 (five strain mixture of food and outbreak isolates) to yield about a 105 cfu/ml slurry, and dispensed 3 ml per sterile polystyrene tube for incubation at 4 and 10°C for up to 4 weeks. Triplicate samples per variable can be assayed weekly for changes in *E*. *coli* O157:H7 populations using direct plating on MacConkey sorbitol agar techniques.

In addition to the bactericidal tests, additional experiments are described herein to determine whether the chimeric bovine-murine antibodies are more effective than their murine counterparts in mediating pathogen ingestion by phagocytes. While there is a substantial amount of data available on the efficacy of humanizing therapeutic murine antibodies in order to improve beneficial reactions between immune cells and target cells (for example ADCC, phagocytosis, antigen presentation) in humans, however, the efficacy of a chimeric bovine-murine antibodies in mediating ingestion and killing of a pathogen in cattle has yet to be determined. Accordingly, functional assays of bovine monocyte/macrophage are described herein to measure killing/ingestion of *E. coli* O157:H7 in the presence of the murine monoclonal antibody, or the chimeric antibody, or no antibody. It is expected that the chimeric bovine-murine antibody described herein is superior in mediating phagocytosis compared to murine only versions.

Further described herein is the purification of sufficient quantities of retrovectors containing genes for the chimeric monoclonal antibodies to conduct further functional assays and additional tests. Based upon the results obtained in the above-mentioned assays and tests, further clonal analysis of packaging cell lines that express the chimeric antibody is described herein. Briefly, a high viral titer producing clone is chosen and expanded. The expanded culture are subsequently induced to produce infective viral particles and viral preparations to enrich viral particles to a titer of approximately 1-5x108 cfu/ml. Such titers have proven effective in producing transgenic animals when used for oocyte injection in transgametic systems.

### V. Transgenic Animal Technologies

The methods and compositions used in certain embodiments of the present invention for creating transgenic animals (*e.g.*, bovines and other ungulates) for expression of the biocidal fusion proteins are described in greater detail below.

### A. Retroviruses and Retroviral Vectors

Retroviruses (family Retroviridae) are divided into three groups: the spumaviruses (*e.g*., human foamy virus); the lentiviruses (*e.g*., human immunodeficiency virus and sheep visna virus) and the oncoviruses (*e.g*., MLV, Rous sarcoma virus).

Retroviruses are enveloped (*i.e*., surrounded by a host cell-derived lipid bilayer membrane) single-stranded RNA viruses that infect animal cells. When a retrovirus infects a cell, its RNA genome is converted into a double-stranded linear DNA form (*i.e*., it is reverse transcribed). The DNA form of the virus is then integrated into the host cell genome as a provirus. The provirus serves as a template for the production of additional viral genomes and viral mRNAs. Mature viral particles containing two copies of genomic RNA bud from the surface of the infected cell. The viral particle comprises the genomic RNA, reverse transcriptase and other *pol* gene products inside the viral capsid (which contains the viral *gag* gene products), which is surrounded by a lipid bilayer membrane derived from the host cell containing the viral envelope glycoproteins (also referred to as membrane-associated proteins).

The organization of the genomes of numerous retroviruses is well known in the art and this has allowed the adaptation of the retroviral genome to produce retroviral vectors. The production of a recombinant retroviral vector carrying a gene of interest is typically achieved in two stages. First, the gene of interest is inserted into a retroviral vector which contains the sequences necessary for the efficient expression of the gene of interest (including promoter and/or enhancer elements which may be provided by the viral long terminal repeats [LTRs] or by an internal promoter/enhancer and relevant splicing signals), sequences required for the efficient packaging of the viral RNA into infectious virions (*e.g*., the packaging signal [Psi], the tRNA primer binding site [-PBS], the 3' regulatory sequences required for reverse transcription [+PBS] and the viral LTRs). The LTRs contain sequences required for the association of viral genomic RNA, reverse transcriptase and integrase functions, and sequences involved in directing the expression of the genomic RNA to be packaged in viral particles. For safety reasons, many recombinant retroviral vectors lack functional copies of the genes that are essential for viral replication (these essential genes are either deleted or disabled); the resulting virus is said to be replication defective.

Second, following the construction of the recombinant vector, the vector DNA is introduced into a packaging cell line. Packaging cell lines provide viral proteins required in *trans* for the packaging of the viral genomic RNA into viral particles having the desired host range (*i.e*., the viral-encoded gag, pol and env proteins). The host range is controlled, in part, by the type of envelope gene product expressed on the surface of the viral particle. Packaging cell lines may express ecotrophic, amphotropic or xenotropic envelope gene products. Alternatively, the packaging cell line may lack sequences encoding a viral envelope (env) protein. In this case the packaging cell line will package the viral genome into particles that lack a membrane-associated protein (*e.g*., an env protein). In order to produce viral particles containing a membrane associated protein that will permit entry of the virus into a cell, the packaging cell line containing the retroviral sequences is transfected with sequences encoding a membrane-associated protein (*e.g*., the G protein of vesicular stomatitis virus [VSV]). The transfected packaging cell will then produce viral particles that contain the membrane-associated protein expressed by the transfected packaging cell line; these viral particles, which contain viral genomic RNA derived from one virus encapsidated by the envelope proteins of another virus are said to be pseudotyped virus particles.

Viral vectors, including recombinant retroviral vectors, provide a more efficient means of transferring genes into cells as compared to other techniques such as calcium phosphate-DNA co-precipitation or DEAE-dextran-mediated transfection, electroporation or microinjection of nucleic acids. It is believed that the efficiency of viral transfer is due in part to the fact that the transfer of nucleic acid is a receptor-mediated process (*i.e*., the virus binds to a specific receptor protein on the surface of the cell to be infected). In addition, the virally transferred nucleic acid once inside a cell integrates in controlled manner in contrast to the integration of nucleic acids which are not virally transferred; nucleic acids transferred by other means such as calcium phosphate-DNA co-precipitation are subject to rearrangement and degradation.

The most commonly used recombinant retroviral vectors are derived from the amphotropic Moloney murine leukemia virus (MoMLV) (Miller and Baltimore, Mol. Cell. Biol., 6:2895 [1986]). The MoMLV system has several advantages: 1) this specific retrovirus can infect many different cell types, 2) established packaging cell lines are available for the production of recombinant MoMLV viral particles and 3) the transferred genes are permanently integrated into the target cell chromosome. The established MoMLV vector systems comprise a DNA vector containing a small portion of the retroviral sequence (the viral long terminal repeat or "LTR" and the packaging or "psi" signal) and a packaging cell line. The gene to be transferred is inserted into the DNA vector. The viral sequences present on the DNA vector provide the signals necessary for the insertion or packaging of the vector RNA into the viral particle and for the expression of the inserted gene. The packaging cell line provides the viral proteins required for particle assembly (Markowitz et al., J. Virol., 62:1120 [1988]).

Despite these advantages, existing retroviral vectors based upon MoMLV are limited by several intrinsic problems: 1) they do not infect non-dividing cells (Miller et al., Mol. Cell. Biol., 10:4239 [1992]), 2) they produce low titers of the recombinant virus (Miller and Rosman, BioTechn., 7: 980 [1989]; and Miller, Nature 357: 455 [1992]) and 3) they infect certain cell types (*e.g*., human lymphocytes) with low efficiency (Adams et al., Proc. Natl. Acad. Sci. USA 89:8981 [1992]). The low titers associated with MoMLV-based vectors has been attributed, at least in part, to the instability of the virus-encoded envelope protein. Concentration of retrovirus stocks by physical means (*e.g*., ultracentrifugation and ultrafiltration) leads to a severe loss of infectious virus.

The low titer and inefficient infection of certain cell types by MoMLV-based vectors has been overcome by the use of pseudotyped retroviral vectors which contain the G protein of VSV as the membrane associated protein. Unlike retroviral envelope proteins which bind to a specific cell surface protein receptor to gain entry into a cell, the VSV G protein interacts with a phospholipid component of the plasma membrane (Mastromarino et al., J. Gen. Virol., 68:2359 [1977]). Because entry of VSV into a cell is not dependent upon the presence of specific protein receptors, VSV has an extremely broad host range. Pseudotyped retroviral vectors bearing the VSV G protein have an altered host range characteristic of VSV (*i.e*., they can infect almost all species of vertebrate, invertebrate and insect cells). Importantly, VSV G-pseudotyped retroviral vectors can be concentrated 2000-fold or more by ultracentrifugation without significant loss of infectivity (Burns et al., Proc. Natl. Acad. Sci. USA, 90:8033 [1993]).

The VSV G protein has also been used to pseudotype retroviral vectors based upon the human immunodeficiency virus (HIV) (Naldini et al., Science 272:263 [1996]). Thus, the VSV G protein may be used to generate a variety of pseudotyped retroviral vectors and is not limited to vectors based on MoMLV.

The present invention is not limited to the use of the VSV G protein when a viral G protein is employed as the heterologous membrane-associated protein within a viral particle. The G proteins of viruses in the Vesiculovirus genera other than VSV, such as the Piry and Chandipura viruses, that are highly homologous to the VSV G protein and, like the VSV G protein, contain covalently linked palmitic acid (Brun et al., Intervirol., 38:274 [1995]; and Masters et al., Virol., 171:285 [1990]). Thus, the G protein of the Piry and Chandipura viruses can be used in place of the VSV G protein for the pseudotyping of viral particles. In addition, the VSV G proteins of viruses within the Lyssa virus genera such as Rabies and Mokola viruses show a high degree of conservation (amino acid sequence as well as functional conservation) with the VSV G proteins. For example, the Mokola virus G protein has been shown to function in a manner similar to the VSV G protein (*i.e*., to mediate membrane fusion) and therefore may be used in place of the VSV G protein for the pseudotyping of viral particles (Mebatsion et al., J. Virol., 69:1444 [1995]). Viral particles may be pseudotyped using either the Piry, Chandipura or Mokola G protein as described in the art with the exception that a plasmid containing sequences encoding either the Piry, Chandipura or Mokola G protein under the transcriptional control of a suitable promoter element (*e.g*., the CMV intermediate-early promoter; numerous expression vectors containing the CMV IE promoter are available, such as the pcDNA3.1 vectors [Invitrogen]) is used in place of pHCMV-G. Sequences encoding other G proteins derived from other members of the Rhabdoviridae family may be used; sequences encoding numerous rhabdoviral G proteins are available from the GenBank database.

### B. Integration of Retroviral DNA

The majority of retroviruses can transfer or integrate a double-stranded linear form of the virus (the provirus) into the genome of the recipient cell only if the recipient cell is cycling (*i.e*., dividing) at the time of infection. Retroviruses that have been shown to infect dividing cells exclusively, or more efficiently, include MLV, spleen necrosis virus, Rous sarcoma virus and human immunodeficiency virus (HIV; while HIV infects dividing cells more efficiently, HIV can infect non-dividing cells).

It has been shown that the integration of MLV virus DNA depends upon the host cell's progression through mitosis and it has been postulated that the dependence upon mitosis reflects a requirement for the breakdown of the nuclear envelope in order for the viral integration complex to gain entry into the nucleus (Roe et al., EMBO J., 12:2099 [1993]). However, as integration does not occur in cells arrested in metaphase, the breakdown of the nuclear envelope alone may not be sufficient to permit viral integration; there may be additional requirements such as the state of condensation of the genomic DNA (Roe *et al., supra*).

### C. Introduction of Retroviral Vectors into Gametes Before the Last Meiotic Division

The nuclear envelope of a cell breaks down during meiosis as well as during mitosis. Meiosis occurs only during the final stages of gametogenesis. The methods described herein exploit the breakdown of the nuclear envelope during meiosis to permit the integration of recombinant retroviral DNA and permit for the first time the use of unfertilized oocytes (*i.e*., pre-fertilization and pre-maturation oocytes) as the recipient cell for retroviral gene transfer for the production of transgenic animals. Because infection of unfertilized oocytes permits the integration of the recombinant provirus prior to the division of the one cell embryo, all cells in the embryo will contain the proviral sequences.

Oocytes which have not undergone the final stages of gametogenesis are infected with the retroviral vector. The injected oocytes are then permitted to complete maturation with the accompanying meiotic divisions. The breakdown of the nuclear envelope during meiosis permits the integration of the proviral form of the retrovirus vector into the genome of the oocyte. When pre-maturation oocytes are used, the injected oocytes are then cultured *in vitro* under conditions that permit maturation of the oocyte prior to fertilization *in vitro.* Conditions for the maturation of oocytes from a number of mammalian species (*e.g*., bovine, ovine, porcine, murine, caprine) are well known to the art. In general, the base medium used herein for the *in vitro* maturation of bovine oocytes, TC-M199 medium, may be used for the *in vitro* maturation of other mammalian oocytes. TC-M199 medium is supplemented with hormones (*e.g*., luteinizing hormone and estradiol) from the appropriate mammalian species. The amount of time a pre-maturation oocyte must be exposed to maturation medium to permit maturation varies between mammalian species as is known to the art. For example, an exposure of about 24 hours is sufficient to permit maturation of bovine oocytes while porcine oocytes require about 44-48 hours.

Oocytes may be matured *in vivo* and employed in place of oocytes matured *in vitro*. For example, when porcine oocytes are to be employed in the methods described herein, matured pre-fertilization oocytes may be harvested directly from pigs that are induced to superovulate as is known to the art. Briefly, on day 15 or 16 of estrus the female pig(s) is injected with about 1000 units of pregnant mare's serum (PMS; available from Sigma and Calbiochem). Approximately 48 hours later, the pig(s) is injected with about 1000 units of human chorionic gonadotropin) (hCG; Sigma) and 24-48 hours later matured oocytes are collected from oviduct. These *in vivo* matured pre-fertilization oocytes are then injected with the desired retroviral preparation as described herein. Methods for the superovulation and collection of *in vivo* matured (*i.e*., oocytes at the metaphase 2 stage) oocytes are known for a variety of mammals (*e.g*., for superovulation of mice, see Hogan *et al., supra* at pp. 130-133 [1994]; for superovulation of pigs and *in vitro* fertilization of pig oocytes see Cheng, Doctoral Dissertation, Cambridge University, Cambridge, United Kingdom [1995]).

Retroviral vectors capable of infecting the desired species of non-human animal, which can be grown and concentrated to very high titers (*e.g*., $.1 x 10⁸ cfu/ml) are preferentially employed. The use of high titer virus stocks allows the introduction of a defined number of viral particles into the perivitelline space of each injected oocyte. The perivitelline space of most mammalian oocytes can accommodate about 10 picoliters of injected fluid (those in the art know that the volume that can be injected into the perivitelline space of a mammalian oocyte or zygote varies somewhat between species as the volume of an oocyte is smaller than that of a zygote and thus, oocytes can accommodate somewhat less than can zygotes).

The vector used may contain one or more genes encoding a protein of interest; alternatively, the vector may contain sequences that produce anti-sense RNA sequences or ribozymes. The infectious virus is microinjected into the perivitelline space of oocytes (including pre-maturation oocytes) or one cell stage zygotes. Microinjection into the perivitelline space is much less invasive than the microinjection of nucleic acid into the pronucleus of an embryo. Pronuclear injection requires the mechanical puncture of the plasma membrane of the embryo and results in lower embryo viability. In addition, a higher level of operator skill is required to perform pronuclear injection as compared to perivitelline injection. Visualization of the pronucleus is not required when the virus is injected into the perivitelline space (in contrast to injection into the pronucleus); therefore injection into the perivitelline space obviates the difficulties associated with visualization of pronuclei in species such as cattle, sheep and pigs.

The virus stock may be titered and diluted prior to microinjection into the perivitelline space so that the number of proviruses integrated in the resulting transgenic animal is controlled. The use of a viral stock (or dilution thereof) having a titer of 1 x 10⁸ cfu/ml allows the delivery of a single viral particle per oocyte. The use of pre-maturation oocytes or mature fertilized oocytes as the recipient of the virus minimizes the production of animals which are mosaic for the provirus as the virus integrates into the genome of the oocyte prior to the occurrence of cell cleavage.

In order to deliver, on average, a single infectious particle per oocyte, the micropipets used for the injection are calibrated as follows. Small volumes (*e.g*., about 5-10 pl) of the undiluted high titer viral stock (*e.g*., a titer of about 1 x 10⁸ cfu/ml) are delivered to the wells of a microtiter plate by pulsing the micromanipulator. The titer of virus delivered per a given number of pulses is determined by diluting the viral stock in each well and determining the titer using a suitable cell line (*e.g*., the 208F cell line) as described in the art. The number of pulses which deliver, on average, a volume of virus stock containing one infectious viral particle (*i.e*., gives a MOI of 1 when titered on 208F cells) are used for injection of the viral stock into the oocytes.

Prior to microinjection of the titered and diluted (if required) virus stock, the cumulus cell layer is opened to provide access to the perivitelline space. The cumulus cell layer need not be completely removed from the oocyte and indeed for certain species of animals (*e.g*., cows, sheep, pigs, mice) a portion of the cumulus cell layer must remain in contact with the oocyte to permit proper development and fertilization post-injection. Injection of viral particles into the perivitelline space allows the vector RNA (*i.e.,* the viral genome) to enter the cell through the plasma membrane thereby allowing proper reverse transcription of the viral RNA.

### D. Detection of the Retrovirus Following Injection into Oocytes or Embryos

The presence of the retroviral genome in cells (*e.g*., oocytes or embryos) infected with pseudotyped retrovirus may be detected using a variety of means. The expression of the gene product(s) encoded by the retrovirus may be detected by detection of mRNA corresponding to the vector-encoded gene products using techniques well known to the art (*e.g*., Northern blot, dot blot, *in situ* hybridization and RT-PCR analysis). Direct detection of the vector-encoded gene product(s) is employed when the gene product is a protein which either has an enzymatic activity (*e.g*., α-galactosidase) or when an antibody capable of reacting with the vector-encoded protein is available.

Alternatively, the presence of the integrated viral genome may be detected using Southern blot or PCR analysis. For example, the presence of the LZRNL or LSRNL genomes may be detected following infection of oocytes or embryos using PCR as follows. Genomic DNA is extracted from the infected oocytes or embryos (the DNA may be extracted from the whole embryo or alternatively various tissues of the embryo may be examined) using techniques well known to the art. The LZRNL and LSRNL viruses contain the *neo* gene and the following primer pair can be used to amplify a 349-bp segment of the *neo* gene: upstream primer: 5'-GCATTGCATCAGCCATGATG-3' (SEQ ID NO:103) and downstream primer: 5'-GATGGATTGCACGCAGGTTC-3' (SEQ ID NO:104). The PCR is carried out using well known techniques (*e.g*., using a GeneAmp kit according to the manufacturer's instructions [Perkin-Elmer]). The DNA present in the reaction is denatured by incubation at 94EC for 3 min followed by 40 cycles of 94EC for 1 min, 60EC for 40 sec and 72EC for 40 sec followed by a final extension at 72EC for 5 min. The PCR products may be analyzed by electrophoresis of 10 to 20% of the total reaction on a 2% agarose gel; the 349-bp product may be visualized by staining of the gel with ethidium bromide and exposure of the stained gel to UV light. If the expected PCR product cannot be detected visually, the DNA can be transferred to a solid support (*e.g*., a nylon membrane) and hybridized with a ^{32P}-labeled *neo* probe.

Southern blot analysis of genomic DNA extracted from infected oocytes and/or the resulting embryos, offspring and tissues derived therefrom is employed when information concerning the integration of the viral DNA into the host genome is desired. To examine the number of integration sites present in the host genome, the extracted genomic DNA is typically digested with a restriction enzyme, which cuts at least once within the vector sequences. If the enzyme chosen cuts twice within the vector sequences, a band of known (*i.e*., predictable) size is generated in addition to two fragments of novel length which can be detected using appropriate probes.

### E. Detection of Foreign Protein Expression in Transgenic Animals

Also described herein are transgenic animals that are capable of expressing foreign proteins in their milk, urine and blood. The transgene is stable, as and shown to be passed from a transgenic bull to his offspring. In addition, the transgenic animals described herein express foreign proteins in their body fluids (*e.g*., milk, blood, and urine). Thus, the present disclosure further demonstrates the utility of using the MoMLV LTR as a promoter for driving the constitutive production of foreign proteins in transgenic cattle. It is also contemplated that such a promoter could be used to control expression of proteins that would prevent disease and/or infection in the transgenic animals and their offspring, or be of use in the production of a consistent level of protein expression in a number of different tissues and body fluids.

For example, the MoMLV LTR described herein will find use in driving expression of antibody to pathogenic organisms, thereby preventing infection and/or disease in transgenic animals created using the methods described herein. For example, it is contemplated that antibodies directed against organisms such as *E. coli, Salmonella ssp., Streptococcus ssp., Staphylococcus spp., Mycobacterium spp.,* produced by transgenic animals will find use preventing mastitis, scours, and other diseases that are common problems in young animals. It is also contemplated that proteins expressed by transgenic animals described herein will find use as bacteriostatic, bactericidal, fungistatic, fungicidal, viricidal, and/or anti-parasitic compositions. Thus, transgenic animals described herein will be resistant to various pathogenic organisms. Furthermore, the milk produced by female transgenic animals would contain substantial antibody levels. These antibodies may be useful in the protection of other animals (*e.g*., through passive immunization methods).

### VI. Considerations for Combating Cryptosporidium and Other Parasites

### A. Production of Transgenic Expression System for Monoclonal 3E2 Antibodies Against C. parvum

An established hybridoma line (as described herein) can be used as a source for the 3E2 genes for insertion into a replication defective retrovector. 3E2 may have especially potent neutralizing capabilities against sporozoites because it is of the IgM isotype. It is thought that through binding to repetitive epitopes of the CSL antigen the circumsporozoite precipitate (CSP)-like reaction is induced (M.W. Riggs et al., J. Immunol., 143:1340-1345 [1989]) that renders the sporozoite non-infective. IgM antibodies exist in several forms, one, in unstimulated B-lymphocytes they are membrane-bound and, two, upon stimulation of the B-lymphocyte, IgM is secreted as a pentamer joined by the J-chain. J-chain expression plays an important role in inducing the pentamerization process of IgM. In studies done by Niles *et al.,* high expression of the J-chain resulted in a high percentage of pentameric IgM. (M.J. Niles et al., Proc. Natl. Acad. Sci. USA, 92:2884-2888 [1995]). A third possible configuration for IgM was shown to be a hexamer. (A. Cattaneo and M.S. Neuberger, EMBO J., 6:2753-2758, and T.D. Randall et al., Eur. J. Immunol., 20:1971-1979 [1990]). Described herein is a cloning strategy that addresses the pentamer and hexamer configurations. The hexamer configuration of IgM may provide better efficacy against *Cryptosporidium* sporozoites than IgG.

An IgM isotype control (of irrelevant specificity) may be constructed in parallel following the cloning strategy described herein. Briefly, the retrovectors are pseudotyped with VSVg to give pantropic infectivity and used to achieve gene transfer to bovine oocytes and to CHO cells (component C). For transgenic expression in mammals (*e.g*., bovines), as opposed to expression in cell culture, the construct is designed to remove antibiotic-based selection markers (i.e., undesirable in an animal population), and to insert a promoter that links expression closely to lactation thus restricting expression to the mammary cells. An alphalactalbumin promoter can be used for this purpose. To assure high probability of infection and transgene integration into the oocyte genome, very high retrovector titer is needed for injection into the very small perivitelline space. It is contemplated that using pseudotyped VSVG vector envelope stabilizes the vector and increases the ability to concentrate vector sufficiently for injection in picoliter amounts. Preferably, transgenic embryos are produced by injection of unfertilized oocytes, *in vitro* fertilization, and transfer to recipient animals (*e.g*., surrogate bovine mothers). After transgenic offspring have been verified as transgenic and grown to 6-8 months, a hormone regimen is used to initiate lactation.

A consideration in using retrovectors is the need to provide assurances that no reversion, recombination, or mutation of replication defective retrovectors to viral competence has occurred. Thus, a testing protocol can be followed for testing packaging cell lines and transgenic offspring.

Two different IRES elements can be used to reduce the likelihood of recombination events that can be triggered by different identical sequences in a vector. The use of the IRES element in between heavy and light chain genes has been tested extensively and proven to yield fully functional antibodies, expressed and secreted into the medium at high levels (up to 100 pg/cell/day in CHO cells in serum free medium).

### B. Selection and Testing of Biocidess, and Preparation of Vector for Cryptosporidium Neutralizing Monoclonal Antibodies and Fusion Proteins

Additional antibodies can be selected from a large previously reported test panel. (*See,* X.D. et al., Infect. Immun., 64:5161-5165 [1996]). For example, 1E10 is an IgG1 isotype, that targets the P23 antigen; 3H2 is an IgM, that targets the GP25-200 antigen. Because, IgG may be preferred for biocide fusion proteins, the 4H9 antibody can be also expressed. 4H9 is an IgG that targets GP25-200, but a different epitope than 3H2. (D.A. Schaefer et al., Infect. Immun., 68:2608-2616 [2000]). A 4 different antibody-biocide fusion types (Figure 2) from each IgG antibody can be constructed. These molecules are expressed in the GPEX cell culture system (Gala Design, Middleton, WI) and tested for their efficacy against sporozoites *in vitro* and *in vivo.* The considerations pertaining to production of tricistronic constructs for IgM, discussed above with respect to 3E2, are also relevant to 3H2.

To select appropriate biocides, the preliminary testing of sporozoite neutralization by potential biocides can be expanded to include additinoal candidates, and comparison of human PLA2 to bee venom PLA2.

Molecular modeling can be used to guide the structural assembly of the fusion molecules. The relative geometry of a monoclonal antibody molecule with a molecule of biocidal activity attached to the C-terminus is similar to that of complement binding to the Fc region of the MAb HC when bound to a pathogen, which results in destruction of the membrane. Thus, the *C. parvum* binding site affinity of the MAb molecule can be used to bring the biocidal activity into close apposition to its substrate by attachment of the biocide to the C-terminus of the monoclonal heavy chain.

Secretory PLA2 is a relatively small molecule (~14 kDa) and is comparable in size to one of the CH1 or CH2 domains of an antibody molecule. As an alternative, an N-terminal extension linker on the PLA2 portion of the molecule is created to move the phospholipase domains a short distance from the MAb molecule. One linker contemplated for use for constructing single chain monoclonal-cytokine fusion proteins is a -(Gly4-Ser)3-extension (~16-20 angstrom extension). (*See e.g.,* C.R. Robinson and R.T. Sauer, Proc. Natl. Acad. Sci. USA, 95:5929-5934 [1998]). This is a relatively neutral sequence that is flexible and does not have a strong structure-forming propensity. A proline can be inserted into the middle of the extension arm to provide a "kink", with freedom to rotate in the extension chain and thus allow different geometrical relationships between the biocide and the antibody molecule.

### C. Expression of Monoclonal Antibodies and Monoclonal Antibody-Biocide Fusions in Cell Culture and Animal Models

Described herein are animal based expression systems for producing large quantities of present compositions, and high yielding cell lines. Cell based production is more expensive and on a smaller scale than production in transgenic animals (*e.g*., bovines), significant quantities of antibodies and fusion products are rapidly obtainable as compared to the proposed transgenic-derived products.

After expression and testing *in vitro* the production can be scaled up using roller bottles to make sufficient recombinant product to test in mice. Then the most promising compounds, based on their efficacy in mice, are tested in an animal model where clinical disease is observed. The neonatal mouse model provides an essential, cost-effective means for the initial *in vivo* evaluation of product efficacy in reducing intestinal infection levels and is widely accepted for this purpose. However, *C*. *parvum* infection in neonatal mice does not cause diarrhea or other signs of disease, hence the need for subsequent evaluation in a clinical model for compositions having demonstrated anti-cryptosporidial activity in mice.

Biglets can be selected as the clinical model of choice because of their small size, availability in adequate numbers to permit comparative studies and statistical analysis, and development of intestinal lesions resulting in acute watery diarrhea, dehydration, malabsorption, and weight loss when infected with *C*. *parvum* (C. W. Kim, Cryptosporidiosis in Pigs and Horses. In: J.P. Dubey, C.A. Speer, and R. Fayer eds. Boca Raton, FL: CRC Press, pp. 105-111 [1990]). Importantly, as monogastrics, the pathogenesis and control of cryptosporidiosis in piglets is thought to closely model that of human infections and response to treatment. (S. Tzipori, Adv. Parasitol., 40:187-221 [1998]).

Criteria for determining efficacy in piglets include, but are not limited to, clinical signs, weight loss, fecal volume and dry matter, and fecal oocyst quantitation and duration of shedding. Following euthanasia at 10 days post infection, extensive histopathological examination complete the data set.

### VII. Transgenic Plant Technologies

The fusion proteins described herein can be expressed in transgenic organisms such as transgenic plants having a transgene inserted into its nuclear or plastidic genome. Techniques of plant transformation are known as the art. (*See e.g*., Wu and Grossman, Methods in Enzymology, Vol. 153, Recombinant DNA Part D, Academic Press [1987], and EP 693554 Foreign nucleic acids can be introduced into plant cells or protoplasts by several methods. For example, nucleic acid can be mechanically transferred by microinjection directly into plant cells by use of micropipettes. Foreign nucleic acid can also be transferred into plant cells by using polyethylene glycol to form a precipitation complex with the genetic material that is taken up by the cell. (*See e.g.,* Paszkowski et al., J. EMBO, 3:2712-2722 [1984]). Foreign nucleic acid can be introduced into plant cells by electroporation. (*See e.g.,* Fromm et al., Proc. Nat. Acad. Sci. USA, 82:5824 [1985]). Briefly, plant protoplasts are electroporated in the presence of plasmids or nucleic acids containing the relevant genetic construct. Electrical impulses of high field strength reversibly permeabilize the plant cell's biomembranes thus allowing the introduction of the plasmids. Electroporated plant protoplasts reform the cell wall, divide, and form a plant callus. Preferably, selection of the transformed plant cells with the transformed gene is accomplished using phenotypic markers.

The cauliflower mosaic virus (CaMV) can be used as a vector to introduce foreign nucleic acids into plant cells. (*See e.g.,* Hohn et al., "Molecular Biology of Plant Tumors," Academic Press, New York, pp. 549-560 [1982]; and US 4,407,956. CaMV viral DNA genome is inserted into a parent bacterial plasmid creating a recombinant DNA molecule which can be propagated in bacteria. The recombinant plasmid can be further modified by introduction of the desired DNA sequence. The modified viral portion of the recombinant plasmid is then excised from the parent bacterial plasmid, and used to inoculate the plant cells or plants.

High velocity ballistic penetration by small particles can be used to introduce foreign nucleic acid into plant cells. Nucleic acid is disposed within the matrix of small beads or particles, or on the surface. (*See e.g.,* Klein et al., Nature, 327:70-73 [1987]). Although typically only a single introduction of a new nucleic acid segment is required, this method also provides for multiple introductions.

A nucleic acid can be introduced into a plant cell by infection of a plant cell, an explant, an ineristem or a seed with *Agrobacterium tumefaciens* transformed with the nucleic acid. Under appropriate conditions, the transformed plant cells are grown to form shoots, roots, and develop further into plants. The nucleic acids can be introduced into plant cells, for example, by means of the Ti plasmid of *Agrobacterium tumefaciens.* The Ti plasmid is transmitted to plant cells upon infection by *Agrobacterium tumefaciens,* and is stably integrated into the plant genome. (*See e.g*., Horsch et al., Science, 233:496-498 [1984]; and Fraley et al., Proc. Nat. Acad. Sci. USA, 80:4803 [1983]).

Plants from which protoplasts can be isolated and cultured to give whole regenerated plants can be transformed so that whole plants are recovered which contain the transferred foreign gene. All plants that can be produced by regeneration from protoplasts can also be transfected using the process according to the invention (*e.g*., cultivated plants of the genera *Fragaria, Lotus, Medicago, Onobrychis, Trifolium, Trigonella, Vigna, Citrus, Geranium, Manihot, Daucus, Arabidopsis, Brassica, Raphanus, Sinapis, Atropa, Capsicum, Hyoscyamus, Lycopersicon, Nicotiana, Solarium, Petunia, Digitalis, Majorana, Ciohorium, Helianthus, Lactuca, Bromus, Asparagus, Antirrhinum, Hererocallis, Nemesia, Pelargoniwm, Panicum, Pennisetum, Ranunculus, Senecio, Salpiglossis, Cucumis, Browaalia, Glycine, Lolium, Zea, Triticum, Sorghum, Datura, Solanum, Beta, Pisum, Phaseolus, Allium, Avena, Hordeum, Oryzae, Setaria, Secale, Sorghum, Triticum, Musa, Cocos, Cydonia, Pyrus, Malus, Phoenix, Elaeis, Rubus, Fragaria, Prunus, Arachis, Saccharum, Coffea, Camellia, Ananas,* or *Vitis).* In general, protoplasts are produced in accordance with conventional methods. (*See e.g.,* US 4,743,548; 4,677,066, 5,149,645; and 5,508,184). Plant tissue may be dispersed in an appropriate medium having an appropriate osmotic potential (*e.g*., 3 to 8 wt. % of a sugar polyol) and one or more polysaccharide hydrolases (*e.g*., pectinase, cellulase, *etc*.), and the cell wall degradation allowed to proceed for a sufficient time to provide protoplasts. After filtration the protoplasts may be isolated by centrifugation and may then be resuspended for subsequent treatment or use.

Plant regeneration from cultured protoplasts is described in Evans et al., "Protoplasts Isolation and Culture," Handbook of Plant Cell Cultures 1:124-176 (MacMillan Publishing Co. New York 1983); M.R. Davey, "Recent Developments in the Culture and Regeneration of Plant Protoplasts," Protoplasts (1983)-Lecture Proceedings, pp. 12-29, (Birkhauser, Basal 1983); P.J. Dale, "Protoplast Culture and Plant Regeneration of Cereals and Other Recalcitrant Crops," Protoplasts (1983)-Lecture Proceedings, pp. 31-41, (Birkhauser, Basel 1983); and H. Binding, "Regeneration of Plants," Plant Protoplasts, pp. 21-73, (CRC Press, Boca Raton 1985).

Regeneration from protoplasts varies from species to species of plants, but generally a suspension of transformed protoplasts containing copies of the exogenous sequence is first generated. In certain species, embryo formation can then be induced from the protoplast suspension, to the stage of ripening and germination as natural embryos. The culture media can contain various amino acids and hormones, such as auxins and cytokinins. It can also be advantageous to add glutamic acid and proline to the medium, especially for such species as corn and alfalfa. Shoots and roots normally develop simultaneously. Efficient regeneration will depend on the medium, on the genotype, and on the history of the culture. If these three variables are controlled, then regeneration is fully reproducible and repeatable.

In vegetatively propagated crops, the mature transgenic plants can be propagated by the taking of cuttings or by tissue culture techniques to produce multiple identical plants for trailing, such as testing for production characteristics. Selection of a desirable transgenic plant is made and new varieties are obtained thereby, and propagated vegetatively for commercial sale. In seed propagated crops, the mature transgenic plants can be self crossed to produce a homozygous inbred plant. The inbred plant produces seed containing the gene for the newly introduced foreign gene activity level. These seeds can be grown to produce plants that have the selected phenotype. The inbreds can be used to develop new hybrids. In this method, a selected inbred line is crossed with another inbred line to produce the hybrid.

Parts obtained from a transgenic plant, such as flowers, seeds, leaves, branches, fruit, and the like are described herein which include cells which have been so transformed. Progeny and variants, and mutants of the regenerated plants are also described herein which comprise the introduced DNA sequences. Progeny and variants, and mutants of the regenerated plants are also described herein.

Selection of transgenic plants or plant cells can be based upon a visual assay, such as observing color changes (*e.g*., a white flower, variable pigment production, and uniform color pattern on flowers or irregular patterns), but can also involve biochemical assays of either enzyme activity or product quantitation. Transgenic plants or plant cells are grown into plants bearing the plant part of interest and the gene activities are monitored, such as by visual appearance (for flavonoid genes) or biochemical assays (Northern blots); Western blots; enzyme assays and flavonoid compound assays, including spectroscopy. (*See e.g*., Harborne et al., (Eds.) "The Flavonoids, Vols: 1 and 2, Acad. Press 1975). Appropriate plants are selected and further evaluated. Methods for generation of genetically engineered plants are further described in US 5,283,184; 5,482,852, and EPO Application EP 693,554.

### VIII. Pharmaceutical Compositions

Described herein are novel methods and compositions for treating diseases characterized by pathogenic infection comprising administering subjects (*e.g*., bovines, humans, and other mammals) a pharmaceutical and/or nutraceutical composition comprising chimeric recombinant antibodies either in food based (*e.g*., whey protein) carriers, or common pharmaceutical carriers, including any sterile, biocompatible pharmaceutical carrier (*e.g*., saline, buffered saline, dextrose, water, and the like) to subjects.

The methods described herein comprise administering the compositions in suitable pharmaceutical carriers. These pharmaceutical compositions may contain a mixture of at least two types of antibody-biocide compositions co-administered to a subject. The pharmaceutical compositions may comprise a plurality of antibody-biocide compositions administered to a subject under one or more of the following conditions: at different periodicities, different durations, different concentrations, different administration routes, *etc*.

The compositions and methods described herein find use in treating diseases or altered physiological states characterized by pathogenic infection. However, the present disclosure is not limited to ameliorating (*e.g*., treating) only these types of conditions in a subject. Indeed, a range of physiological symptoms and disease etiologies in subjects generally characterized by infection with a pathogen (*e.g*., bacteria, archeae, viruses, mycoplasma, fungi, *etc*.) can be treated.

Depending on the condition being treated, these pharmaceutical compositions are formulated and administered systemically or locally. Techniques for formulation and administration are found in the latest edition of "Remington's Pharmaceutical Sciences" (Mack Publishing Co, Easton Pa.). Accordingly, pharmaceutical compositions can be administered in accordance with acceptable pharmaceutical delivery methods and preparation techniques. For example, some compounds described herein are used in administration to a subject intravenously in a pharmaceutically acceptable carrier such as physiological saline. For injection, the pharmaceutical compositions can be formulated in aqueous solutions, preferably in physiologically compatible buffers (*e.g*., Hanks' solution, Ringer's solution, or physiologically buffered saline). For tissue or cellular administration, penetrants appropriate to the particular barrier to be permeated are preferably used in the formulations. Such penetrants are generally known in the art. Standard methods for intracellular delivery of pharmaceutical agents can be used (*e.g*., delivery via liposomes). Such methods are well known to those skilled in the art.

The present compositions can be formulated for parenteral administration, including intravenous, subcutaneous, intramuscular, and intraperitoneal. These compositions optionally can include aqueous solutions (*i.e*., water-soluble forms). Additionally, suspensions of the active compounds may also be prepared as oily injection suspensions as appropriate. Suitable lipophilic solvents or vehicles include fatty oils such as sesame oil, or synthetic fatty acid esters, such as ethyl oleate or triglycerides, or liposomes. Aqueous injection suspensions may contain substances that increase the viscosity of the suspension, such as sodium carboxymethyl cellulose, sorbitol, or dextran. Optionally, the suspension may also contain suitable stabilizers or agents that increase the solubility of the compounds to allow for the preparation of highly concentrated solutions.

Therapeutic co-administration of some compositions is also be accomplished using gene therapy techniques described herein and commonly known in the art.

The present compositions can be formulated using pharmaceutically acceptable carriers and in suitable dosages for oral administration. Such carriers enable the compositions to be formulated as tablets, pills, capsules, dragees, liquids, gels, syrups, slurries, suspensions and the like, for oral or nasal ingestion by a patient to be treated.

Pharmaceutical preparations for oral use can be obtained by combining the active compounds (*e.g*., chimeric antibody biocide fusion proteins) with a solid excipient, optionally grinding the resulting mixture, and processing the mixture of granules, after adding suitable auxiliaries, if desired, to obtain tablets or dragee cores. Suitable excipients are carbohydrate or protein fillers such as sugars, including lactose, sucrose, mannitol, or sorbitol; starch from corn, wheat, rice, potato, *etc*.; cellulose such as methyl cellulose, hydroxypropylmethyl-cellulose, or sodium carboxymethylcellulose; and gums including arabic and tragacanth; and proteins such as gelatin and collagen. If desired disintegrating or solubilizing agents may be added, such as the cross-linked polyvinyl pyrrolidone, agar, alginic acid or a salt thereof such as sodium alginate.

Ingestible formulations of the present compositions may further include any material approved by the United States Department of Agriculture for inclusion in foodstuffs and substances that are generally recognized as safe (GRAS), such as, food additives, flavorings, colorings, vitamins, minerals, and phytonutrients. The term "phytonutrients" as used herein, refers to organic compounds isolated from plants that have a biological effect, and includes, but is not limited to, compounds of the following classes: isoflavonoids, oligomeric proanthcyanidins, indol-3-carbinol, sulforaphone, fibrous ligands, plant phytosterols, ferulic acid, anthocyanocides, triterpenes, omega 3/6 fatty acids, polyacetylene, quinones, terpenes, cathechins, gallates, and quercitin.

Dragee cores are provided with suitable coatings such as concentrated sugar solutions, which may also contain gum arabic, talc, polyvinylpyrrolidone, carbopol gel, polyethylene glycol, and/or titanium dioxide, lacquer solutions, and suitable organic solvents or solvent mixtures. Dyestuffs or pigments may be added to the tablets or dragee coatings for product identification or to characterize the quantity of active compound, (*i.e*., dosage).

Compositions described herein that can be used orally include push-fit capsules made of gelatin, as well as soft, sealed capsules made of gelatin and a coating such as glycerol or sorbitol. The push-fit capsules can contain the active ingredients mixed with fillers or binders such as lactose or starches, lubricants such as talc or magnesium stearate, and, optionally, stabilizers. In soft capsules, the active compounds may be dissolved or suspended in suitable liquids, such as fatty oils, liquid paraffin, or liquid polyethylene glycol with or without stabilizers.

Therapeutic agents can be used in administration of a patient alone, or in combination with one or more other drugs or therapies (*e.g*., antibiotics and antiviral agents *etc*.) or in pharmaceutical compositions where it is mixed with excipient(s) or other pharmaceutically acceptable carriers. The pharmaceutically acceptable carrier can be pharmaceutically inert.

Pharmaceutical compositions suitable for use in the present invention include compositions wherein the active ingredients are contained in an effective amount to achieve the intended purpose. For example, an effective amount of therapeutic compound(s) may be that amount that destroys or disables pathogens as compared to control pathogens.

In addition to the active ingredients, preferred pharmaceutical compositions optionally comprise pharmaceutically acceptable carriers, such as, excipients and auxiliaries that facilitate processing of the active compounds into preparations that can be used pharmaceutically.

The pharmaceutical compositions used in the methods of the present invention can be manufactured according to well-known and standard pharmaceutical manufacturing techniques (*e.g*., by means of conventional mixing, dissolving, granulating, dragee-making, levigating, emulsifying, encapsulating, entrapping or lyophilizing processes).

Dosing is dependent on severity and responsiveness of the disease state to be treated, with the course of treatment lasting from several days to several months, or until a cure is effected or a diminution of the disease state is achieved. Optimal dosing schedules are calculated from measurements of composition accumulation in the subject's body. The administering physician can easily determine optimum dosages, dosing methodologies and repetition rates. Optimum dosages may vary depending on the relative potency of compositions agents, and can generally be estimated based on the EC₅₀s found to be effective in *in vitro* and *in vivo* animal models. Additional factors that may be taken into account, include the severity of the disease state; the age, weight, and gender of the subject; the subject's diet; the time and frequency of administration; composition combination(s); possible subject reaction sensitivities; and the subject's tolerance/response to treatments. In general, dosage is from 0.001 µg to 100 g per kg of body weight, and may be given once or more daily, weekly, monthly or yearly. The treating physician can estimate repetition rates for dosing based on measured residence times and concentrations of the drug in bodily fluids or tissues. Following successful treatment, it may be desirable to have the subject undergo maintenance therapy to prevent the recurrence of the disease state, wherein the therapeutic agent is administered in maintenance doses, ranging from 0.001 µg to 100 g per kg of body weight, once or more daily, weekly, or other period.

For any compound used in the methods described herein, the therapeutically effective dose can be estimated initially from cell culture assays. Then, preferably, dosage can be formulated in animal models (particularly murine or rat models) to achieve a desirable circulating concentration range that results in increased PKA activity in cells/tissues characterized by undesirable cell migration, angiogenesis, cell migration, cell adhesion, and/or cell survival. A therapeutically effective dose refers to that amount of compound(s) that ameliorate symptoms of the disease state (*e.g*., pathogenic infection). Toxicity and therapeutic efficacy of such compounds can be determined by standard pharmaceutical procedures in cell cultures or experimental animals, *e.g*., for determining the LD₅₀ (the dose lethal to 50% of the population) and the ED₅₀ (the dose therapeutically effective in 50% of the population). The dose ratio between toxic and therapeutic effects is the therapeutic index, and it can be expressed as the ratio LD₅₀/ ED₅₀. Compounds that exhibit large therapeutic indices are preferred. The data obtained from cell culture assays and additional animal studies can be used in formulating a range of dosage, for example, mammalian use (*e.g*., humans). The dosage of such compounds lies preferably, however the present invention is not limited to this range, within a range of circulating concentrations that include the ED₅₀ with little or no toxicity.

Guidance as to particular dosages and methods of delivery is provided in the literature (*See*, US 4,657,760; 5,206,344; or 5,225,212). Use in Administration of some agents to a patient's bone marrow may necessitate delivery in a manner different from intravenous injections.

### EXAMPLES

The present invention provides the following non-limiting examples to further describe certain contemplated embodiments of the present invention. Examples and figures which are not covered by the claims are used as referential examples which are useful for understanding the invention and are not embodiments of the invention.

### EXAMPLE 1

### Effects of PLA2 on Sporozoite Infectivity

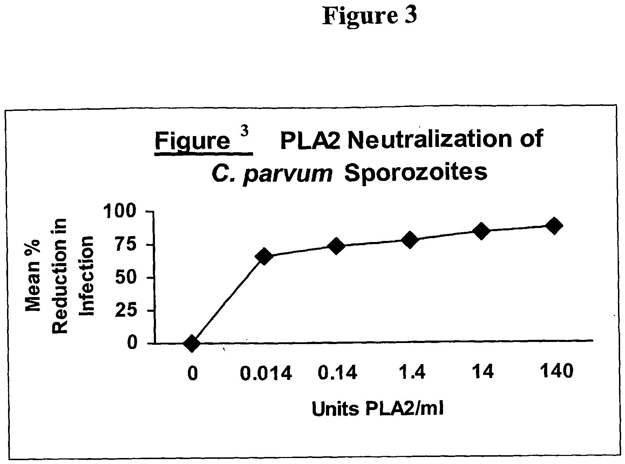

This experiment describes the effects of PLA2 on sporozoite infectivity. Briefly, sporozoites were incubated in an isotonic saline solution (37 °C, 30 min) with a range of concentrations of PLA2 isolated from honey bee venom (Sigma-Aldrich Corp., St. Louis, MO, 1.5 U/µg protein). Control sporozoites were identically incubated in buffer containing concentration-matched BSA but no PLA2. Sporozoites were then washed in medium and inoculated onto replicate Caco-2 human intestinal epithelial cell monolayers. 24 h later, infection was quantified in test and control monolayers by immunofluorescence assay as described herein and the mean percent reduction of infection calculated. The results indicate that PLA2 achieved a highly significant reduction of sporozoite infectivity at a concentration as low as 0.014 U/ml (Figure 3, P < 0.0005). Percent viability of Caco-2 cells following exposure to PLA2-treated sporozoites (86%) was similar to that of uninoculated control cells (91 %) at 24 hrs, as determined by trypan blue dye exclusion. This finding suggests little if any toxic effect of residual PLA2 on host cells. The data suggests that PLA2 is a viable candidate for antibody fusion. Through fusion of PLA2 to a monoclonal antibody with a high affinity for sporozoites, such as 1E10 or 4H9, lethal concentrations of biocide are deliverable to the sporozoite surface with relatively low amounts of biocide being used.

### Reference EXAMPLE 2

### Target Antigens CSL, P23, and GP25-200 are Conserved in both Type 1 and Type 2 C. parvum Isolates

Western blotting of type 1 and type 2 *C. parvum* was performed to evaluate expression of the antigens and epitopes defined by the monoclonal antibodies proposed herein. For these studies, human *C. parvum* isolates were obtained from Peruvian patients and genotyped by nested PCR primers designed to amplify a region within the 18S rRNA gene, followed by RFLP analysis of the amplicons to differentiate Type 1 from Type 2 according to G.D. Sturbaum et al., Appl. Environ. Microbiol., 67:2665-2668 [2001]). Two human isolates determined to be of the Type 1 genotype were evaluated by Western blot for recognition by monoclonal antibodies 3E2 (anti-CSL), 1E10 (anti-P23), and 3H2 (anti-GP25-200) using previously described methods. (M.W. Riggs et al., Infect. Immun., 62:1927-1939 [1994]). The Iowa Type 2 isolate (J. Heine et al., J. Infect. Dis., 150:768-775 [1984]) was examined in parallel. Each monoclonal bound to both Type 1 isolates. In addition, the molecular weights and immunoreactivities of the Type 1 antigens recognized by each monoclonal were indistinguishable from those recognized in the Type 2 isolate in blots of antigen resolved by either 2-12% or 4-20% reducing SDS-PAGE. Importantly, these findings suggest conservation of the antigens and epitopes defined by 3E2, 1E10, and 3H2 between Type 1 and Type 2 *C. parvum*, and are consistent with the functional role ascribed to each antigen.

### Reference EXAMPLE 3

### Various Genetic Engineering Techniques

The present example describes the isolation of the genes for the heavy, light, and J-chains from the 3E2 murine hybridoma cell line, cloning into the retrovector backbone in two configurations (for cell culture expression and for transgenic production), and clonal analysis of the vector producing packaging lines to identify high titer lines maintaining the fidelity of the protein.

Cells from the 3E2 hybridoma are used to extract total RNA with the purpose of isolating the monoclonal antibody-specific heavy, light and J-chain transcripts. Upon total RNA extraction, the RNA is reverse transcribed to create cDNA using standard molecular biology protocols. The total cDNA is then used as a template to specifically amplify the mouse IgM-heavy and light chains as well as the J chain. Site-directed mutagenesis primers are used to amplify the sequences. The use of these primers adds short sequences of DNA that introduce suitable restriction sites that allow direct cloning of the product into the retrovector backbone.

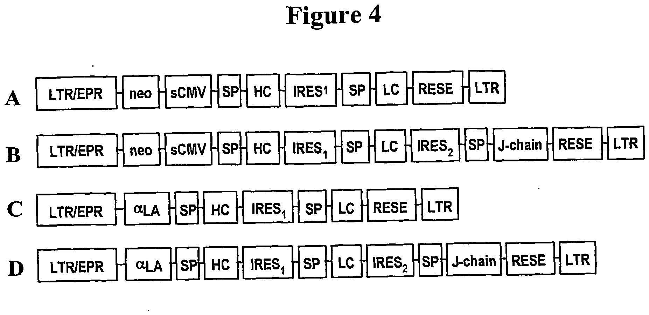

As mentioned herein, two retroviral constructs are made containing the hybridoma-derived antibody genes. Preferably, one is a bicistronic construct, aimed at producing hexameric IgM. This construct bears the genes for IgM heavy and light chain and upon expression in a cell, spontaneous hexamer formation of IgM takes place. The elements of this and other contemplated constructs are shown in Figures 4A-4D grant. The construct is Figure 4A provides a bicistronic antibody. The construct provided in Figure 4B is tricistronic and contains, in addition to the IgM heavy and light chain, the J-chain that for pentamer formation of IgM. (Figure 4B).

To create the tricistronic vector the following cloning steps are performed. First, the heavy chain (HC) is cloned into the multiple cloning site (MCS) of the disclosed retrovector backbone. Second, the genes for genes for the encephalomyocarditis virus (EMCV) IRES (internal ribosome entry site) element, the signal peptide (SP) and the IgM light chain (LC) are combined. Preferably, the IRES is engineered to optimize the secondary initiation of protein synthesis, thus allowing consistent performance in obtaining equimolar expression of heavy and light chains. The genes for the foot-and-mouth disease virus (FMDV) IRES element, the SP and the J-chain gene are combined in parallel. (*See e.g.,* M. Harries et al., J. Gene Med., 2:243-249 [2000]; and X.Y. Wen et al., Cancer Gene Tber., 8:361-370 [2001]). Third, the IRES-SP-LC element is cloned into the backbone after the HC. Fourth, the third element of the construct, the J-chain, preceded by the second IRES is cloned into the backbone. Using two different IRES elements may reduce the likelihood of recombination events that are usually triggered by different identical sequences in a vector. The use of the IRES element in between heavy and light chain genes has been extensively tested and has proven to yield fully functional antibodies, expressed and secreted into the medium at exceptionally high levels (up to 100 pg/cell/day in CHO cells in serum free medium).

Once the retroviral constructs are complete, quality control sequencing is used to confirm that all the elements are present. The retrovector construct are then used to transfect production cell lines using the GPEX system (Gala Design, Inc., Middleton, WI). Following production in the GPEX system, purified product (either pentameric or hexameric IgM 3E2) is tested using standard *in vitro* inhibition tests described herein and/or of known technologies.

For the r3E2 monoclonal antibody to be expressed in the milk of cows, lactation specific promoter based on the bovine alpha-lactalbumin promoter is used (G.T. Bleck and R.D. Bremel, Gene, 126:213-218 [1993]), and the neo-selectable marker is removed from the construct. (*See*, Figures 4C and 4D). In a standard cloning step, the sCMV promoter used in the GPEX system is replaced with the alpha-lactalbumin promoter. Clonal analysis performed on the packaging cell lines to identify the antibody-producing clones that give the highest expression. In addition, an IgM isotype control (of irrelevant specificity) is constructed in parallel following the same cloning strategy.

### Reference EXAMPLE 4

### Production of Vector and Injection into Bovine Oocytes to Make Transgenic Embryos for Transfer to Recipient Animals (Cattle)

After quality assurance, the alphalactalbumin- bearing construct is used to transfect the 293 gp packaging cell line along with the plasmid encoding for the VSVg surface glycoprotein for pseudotyping the viral particles. *(See,* A.W. Chan et al., Proc. Natl. Acad. Sci. USA, 95:14028-14033 [1998]). 293gp packaging cells used in this process are derived from working stocks supplied by Gala Designs, Inc. (Middleton, WI) for cGMP production. The resulting viral supernatant is used to infect packaging cells at a low virus to cell ratio so as to achieve single insertions of the virus. The packaging cell pool are then subjected to a clonal analysis and supernatants of single clones are monitored for high titer viral particle production. One clone is chosen based on viral titer and quality assurance for use in the TRANSGAMETIC system (Gala Design, Inc., Middleton, WI). Bovine oocytes are harvested and grown in culture to metaphase 2 arrest (16 hrs). At this stage, which is prolonged in the bovine oocyte, the nuclear membrane has dispersed, allowing vector to gain access to the nucleus. Pseudotyped vector injected into the perivitelline space infects the oocyte and provirus is integrated into the oocyte's haploid DNA. Upon viral particle injection, the oocytes are fertilized using sexed semen, which allows for almost 100 % female calves to be born. The transgenic frequency is expected to be between 25-75%. Preferably, embryos are biopsied and screened by PCR for presence of the transgene prior to transfer into recipients, to optimize the transfer of transgene positive embryos.

*In vitro* tests are done to confirm that the tricistronic expression of the 3E2 IgM antibody has no influence on specificity and affinity of this antibody. Following positive *in vitro* tests of the recombinant 3E2 monoclonal antibody and achievement of high numbers of transgenic embryos, the embryos are transferred into surrogate mothers. Accordingly, groups of young mature female cattle (heifers) are hormonally synchronized to receive embryos at seven days post fertilization. Cattle are observed throughout pregnancy and ultrasounds are conducted to confirm pregnancy and sex of the embryo at 70 days. After the 280 day gestation period, calves are delivered by cesarian section (a routine surgery performed under epidural anesthesia in a standing surviving cow) and tested for the transgene.

### Reference EXAMPLE 5

### Confirmation of Transgene Presence

Following birth of the offspring they are tested for presence of the transgene and raised to near puberty. Lactation is then hormonally induced to identify the best protein expression and to provide product for evaluation.

At the age of approximately 8 months, lactation is induced in transgenic heifers, using a hormonal regimen, and milk analyzed for expression of the r3E2 product. Product is collected, purified from whey, and quantified for efficacy studies in mice and piglets.

A progestin implant is used to simulate a short pseudopregnancy and then initiate milking in peripubertal (6-8 months old) heifers. Heifers should yield up to 250-1000 ml per day of milk, increasing rapidly to approximate a first lactation heifer yield of 15-20 liters a day. Subsequent fertility is not impaired. Milk product is tested for the presence of murine antibody using established Western blot and ELISA procedures. The animals are milked until enough product is obtained to conduct efficacy testing in mice and the neonatal pig model using assays described herein. For quantification to carry out efficacy testing in pigs, monoclonal antibody is purified after fat removal from milk by continuous flow centrifuge while the milk is at animal body temperature. A skim milk product is used for further processing. Size exclusion chromatography and tangential-flow ultrafiltration may allow purification of sufficient amounts. MAbs are recovered from the milk serum with affinity chromatography or size exclusion chromatography with a similar efficiency as from cell culture fluids.

### Reference EXAMPLE 6

### Evaluation of Efficacy of Milk Production

Efficacy studies of milk production can be preformed in neonatal mouse and piglet models respectively. *In vivo* efficacy assays for *C. parvum* neutralizing r3E2 are preformed in mice. Studies of the effect of milk expressed 3E2 on the infectivity of *C. parvum* sporozoites in mice are performed as described herein.
*In vivo* efficacy assays for *C. parvum* neutralizing r3E2 are conducted in piglets. These studies are performed following the same protocol as described herein. Three groups of 8 piglets are assigned to treatment (milk derived r3E2), isotype rIgM control, and placebo control groups. Dosages, experimental regimens, and blinded evaluations are conducted as described herein.

### Reference EXAMPLE 7

### Founder Animals

Lines of founder animals are identified for propagation to develop production herds. Suitable high expressing transgenic founder animals (*e.g.*, cattle) are identified and superovulated for propagation of a herd of production animals for large scale production of r3E2. Yields of r3E2 in milk are compared between founder animals and the best animal(s) selected for super ovulation and insemination. Embryos are harvested and stored in liquid nitrogen for future herd expansion.

### Reference EXAMPLE 8

### Identification of Candidate for Expression as Recombinant Antibody Biocide Fusion Proteins

Various biocides can be evaluated for potential neutralizing activity against *C. parvum* sporozoites using the *in vitro* assay described in herein. Candidate biocides include, but are not limited to: PLA2, both from human and bee venom; protease inhibitors such as leupeptin, aprotinin, antipain, ainastatin, and soybean trypsin inhibitor; lysozyme; and phosphatidylinositol-specific phospholipase C. The preceding protease inhibitor candidates were selected based on their reported activity against *C. parvum.* (*See e.g*., J.R. Forney et al., J. Parasitol., 82:638-640 [1996]; J.R. Forney et al., J. Parasitol., 83:771-774 [1997]; and P.C. Okhuysen et al., Antimicrob. Agents Chemother., 40:2781-2784 [1996]).

For this assay, isolated sporozoites are incubated (15 min, 37°C) with an individual biocide in isotonic buffer over a range of concentrations that would theoretically be achievable at the sporozoite surface by targeted delivery as a MAb-biocide fusion protein. PLA2 concentrations are based in part on preliminary data which showed that < 0.02 units/ml was effective in neutralization. In parallel, viability of control sporozoites after incubation with the selected biocide concentrations is determined by fluorescein diacetate assay. *(See,* M.W. Riggs et al., Infect. Immun., 62:1927-1939 [1994]). Following incubation with biocide, sporozoites are washed, and then inoculated onto individual Caco-2 human intestinal epithelial cell monolayers grown in microscopy grade 96-well plates (10 replicates per treatment). For comparison, control monolayers are inoculated with sporozoites identically incubated with: 1) MEM; 2) murine hybridoma-derived neutralizing MAb 3E2 as a positive control; or 3) non-toxic control proteins such as BSA, each concentration-matched to the biocide being tested. Samples are then processed and evaluated as described herein. The mean numbers of intracellular parasite stages per host cell in test and control cultures is examined for significant differences using ANOVA. Each experiment is performed three times. In parallel experiments, to monitor potential host cell toxicity of residual biocide, control monolayers are inoculated with the final wash medium from biocide incubation tubes to which no sporozoites were added, but which otherwise have been processed identically to test samples. Cell viability in control and sporozoite inoculated monolayers is determined 24 hrs post-inoculation using an acridine orange-ethidium bromide viability assay and epifluorescence microscopy. *(See,* R.C. Duke and J.J. Cohen, Morphological and biochemical assays of apoptosis. John Wiley & Sons, New York, NY, [2002]).

### Reference EXAMPLE 9

### Isolation of Genes for Antibody Heavy, Light chains, and J chains

The genes for antibody heavy, light chains, and J chains where applicable, can be isolated from the 1E10, 3H2, and 4H9 hybridoma cell lines. The genes are cloned into the GPEX retrovector (Gala Design, Inc., Middleton, WI) as standalone antibody constructs for each antibody, and for the IgG1s 1E10 and 4H9, as fusions to a biocide gene. 4 structurally different antibody-biocide fusion variants are considered for 1E10 and 4H9. At the same time, vectors with a promoter suitable for transgenic expression are prepared.

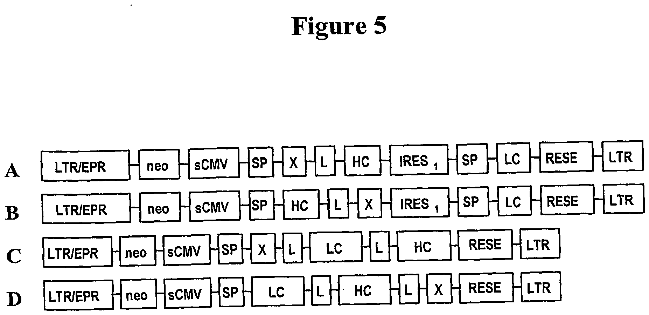

The following hybridoma cell lines are used for antibody gene extraction: 3H2, which expresses an IgM against GP25-200; 4H9, which expresses an IgG1 against GP25-200; and IE10, which expresses an IgG1 against P23. An isotype control for IgG is constructed and prepared in parallel using a hybridoma of irrelevant specificity. Total RNA is extracted from cells with the purpose of isolating the monoclonal antibody-specific heavy and light chain genes as described herein. The immunoglobulin genes are be cloned into the GPEX retrovector backbone as bicistronic constructs; in the case of 3H2, a cloning strategy identical to the one applied for the 3E2 constructs described herein can be applied. (*See*, Figures 5A-5D). Standalone recombinant constructs of each antibody are produced. In addition, two IgG isotypes are engineered to contain a biocide attached to either the N-terminus or the C-terminus of the antibody. The cDNA for the biocide found to be most effective in neutralizing *C. parvum* sporozoites *in vitro,* and least toxic to host cells is acquired through either the NIH Mammalian Gene Collection (human PLA2) or synthesized (Blue Heron Biotechnology, Seattle). The PLA2, or other biocide, cDNA is expanded through standard amplification in *E. coli* laboratory strains. Plasmid are extracted and sequenced for quality control purposes. The biocide genes are then cloned into 4 different antibody fusion constructs using glycine-serine (G4S)3-4 linkers. When expressed in the GPEX system, these constructs produce: a full size antibody with a biocide fusion to either the N-terminus (Figure 5A) or to the C-terminus (Figure 5B) of the heavy chain; or a single chain antibody with a biocide fusion to the N-terminus of the light chain (Figure 5C) or to the C-terminus of the heavy chain (Figure 5D). The antibody-biocide fusions are tested for their efficacy in mediating neutralization and killing of sporozoites *in vitro* and reducing infection *in vitro* and *in vivo.*

Constructs are also prepared for the production of transgenic embryo monoclonal antibody to be expressed in the milk of cows, using a lactation specific promoter based on the bovine alpha-lactalbumin promoter (G.T. Bleck and R.D. Bremel, Gene, 126:213-218 [1993]), and the neo-selectable marker is removed from the construct (Figure 4). In a standard cloning step, the sCMV promoter used in the GPEX system (Gala design, Inc., Middleton, WI) will be replaced with the alpha-lactalbumin promoter.

Retrovector constructs are used to transduce host cells and produce pseudotyped replication deficient retrovector. Pool populations of transduced cells are subjected to a clonal selection, based on antibody levels present in the medium supernatant determined by *C. parvum* ELISA. Clones with the highest level of antibody secreted into the supernatant are chosen to produce milligram amounts of recombinant murine monoclonal antibody and monoclonal antibody-biocide fusions against *C. parvum.*

Constructs needed for transgenic cattle production are also prepared. Constructs contain lactation specific promoter based on the bovine alpha-lactalbumin promoter (G.T. Bleck and R.D. Bremel, *infra*), and no neo-selectable marker (Figure 4). In a standard cloning step, the sCMV promoter used in the GPEX system is replaced with the alpha-lactalbumin promoter.

### Reference EXAMPLE 10

### Cloning of Vector Constructs

The above vector construct, and those for antibody 3E2 are clonally selected and expressed in the GPEX cell culture system (Gala Design, Inc., Middleton, WI) to obtain adequate quantities of assembled antibody or antibody-biocide fusion protein for testing in *vitro* and *in vivo.*

Briefly, the retrovector constructs prepared above are used to transform host cells along with the plasmid that encodes the vesicular stomatitis virus glycoprotein (VSV-G) used for pseudotyping the retrovirus. This procedure creates intermediate level viral titer that is used to infect production cell lines (CHO cells). CHO cells used in this process are derived from a working stock used to established a cGMP production. The population of transduced cells is subjected to a clonal selection, based on antibody levels present in the medium supernatant. Antibody levels are determined by standard ELISA methods using sporozoite lysate antigen prepared as described in Schaefer *et al.* (D.A. Schaefer et al., Infect. Immun., 68:2608-2616 [2000]). The clones with the highest level of antibody secreted into the supernatant are chosen to produce milligram amounts of recombinant monoclonal antibody.
Using the GPEX cell culture in a roller bottle system, gram scale quantities of rMAbs 3E2, 3H2, 1E10, 4H9, and the rMAb-fusion parasiticides are expressed. Based on a 30 pg/cell/day average, one roller bottle produces approximately 20 mg product per week.

Complete product purification is unnecessary to formulate oral immunotherapies, especially when milk derived. However, for the purposes of standardization of tests, purification of the monoclonals from tissue culture medium can follow protocols established for other monoclonals. Briefly, harvested media is filtered through a 0.45 micron sterile filter to remove cells and the immunoglobulins (IgG1, IgG2, and IgG4) and are captured using a protein A affinity column, or in case of IgM, using HiTrap IgM Purification columns (Amersham Biosciences, Piscataway, NJ) or for the purification of single chain antibodies Thiophilic Resin columns (BD Biosciences Clontech, Palo Alto, CA). After washing, the immunoglobulins are eluted by low pH and the pooled eluate fractions are neutralized to pH 7.5. A second chromatography step can be employed to remove contaminants, host cell DNA and to act as a viral clearance step. This typically utilizes anion exchange chromatography (*e.g.*, Q-Sepharose). The final polishing step utilizes size exclusion chromatography (*e.g.*, Sephadex 200), to separate aggregates from monomers. Antibody are further concentrated or formulated as required.

### Reference EXAMPLE 11

### Recombinant Monoclonal Antibodies and Monoclonal Antibody Biocide Fusion Products Efficacy in Neutralizing Sporozoites In vitro

Recombinant monoclonal antibodies and monoclonal antibody biocide fusion products expressed herein are tested for their efficacy in neutralizing sporozoites *in vitro.*

Prior to testing in neutralization assays, the monoclonals are evaluated for retention of sporozoite and merozoite reactivity by IFA, and for antigen specificity by Western immunoblot. (*See e.g.,* M.W. Riggs et al., Infect. Immun., 62:1927-1939 [1994]; M.W. Riggs et al., J. Immunol., 158:1787-1795 [1997]).

### In vitro neutralization assay for C. parvum

To quantify specific neutralizing activity of each of the four MABs and the fusion biocides against the infective sporozoite stage, an *in vitro* neutralization assay is used. *(See,* R.C. Langer et al., Infect. Immun., 67:5282-5291 [1999]). The antibody-biocide fusions based on the r1E10 and r4H9 antibodies in the four configurations depicted in Figure 5, and full size versions of r1E10, r4H9, and r3H2 are each tested individually. For this assay, isolated sporozoites are incubated with the selected MAB (10 µg/ml final concentration), then inoculated onto individual Caco-2 human intestinal epithelial cell monolayers (ATCC HTB37) (M. Pinto et al., Biol. Cell, 47:323-330 [2002]). Prior to inoculation, monolayers of Caco-2 cells are grown to ∼90% confluency in microscopy grade 96-well tissue culture plates. For comparison, control monolayers are inoculated with sporozoites which have been identically incubated with: 1) tissue culture medium (MEM); 2) murine hybridoma-derived neutralizing monoclonal; or 3) isotype- and concentration- matched recombinant control MAb of irrelevant specificity. Ten replicates are performed for each treatment. After incubation, inoculation medium is aspirated from monolayers and replaced with MEM. At 24 hrs post-inoculation, monolayers are washed, fixed, blocked, and processed for automated immunofluorescence assay (IFA) using MAb 4B10 and AlexaFluor488 affinity-purified goat anti-mouse IgM to detect intracellular stages. MAb 4B10, prepared against *C. parvum* as previously described (M.W. Riggs et al., J. Immunol. 158:1787-1795 [1997]), recognizes all parasite stages in Caco-2 cells through 72 hrs post-inoculation (R.C. Langer and M.W. Riggs, Infect. Immun., 67:5282-5291 [1999]). Intestinal epithelial cell nuclei are counterstained with 300 nM 4,6-diamidino-2-phenylindole. Using an Olympus-IMT2 inverted microscope equipped for automated digital image capture, 50 standardized visual fields per well are read and stored on the program computer. Intracellular parasite stages and epithelial cell nuclei are then quantified using Compix SimplePCI software (Compix, Inc., Cranberry Township, PA). Mean numbers of intracellular parasite stages per host cell in test and control cultures are examined for significant differences using ANOVA. Each experiment is performed three times. BSL-2 precautions are observed to prevent accidental infection of project personnel with *C. parvum.*

### Cryptosporidium parvum propagation for use in the proposed studies

The Iowa C. *parvum* isolate (J. Heine et al., J. Infect. Dis., 150:768-775 [1984]) (genotype 2, bovine origin) has been maintained since 1988 by propagation in newborn *Cryptosporidium*-free calves (M.W. Riggs et al., J. Immunol., 143:1340-1345 [1989]; and M.W. Riggs and L.E. Perryman, Infect. Immun., 55:2081-2087 [1987]). This well-characterized isolate is infectious for humans and animal models, including neonatal mice and pigs. *(See e.g.,* J. Heine et al., J. Infect. Dis., 150:768-775 [1984]; R.C. Langer and M.W. Riggs, Infect. Immun., 67:5282-5291 [1999]; H.W. Moon and W.J. Bemrick, Vet. Pathol., 18:248-255 [1981]; and S. Tzipori H. and Ward, Microbes. Infect., 4:1047 [2002]). Parasites are obtained by propagation in newborn calves as previously described. (M.W. Riggs and L.E. Perryman, *supra*). Oocysts are isolated from the feces of experimentally infected calves as previously described, and stored in 2.5% KCr₂O₇ (4 °C) (M.J. Arrowood K. and Donaldson et al., J. Eukaryot. Microbiol., 43:895 [1996]; and M.W. Riggs and L.E. Perryman, *supra*). To obtain isolated sporozoites, oocysts are hypochlorite-treated prior to excystation, then passed through a sterile polycarbonate filter. For mouse and piglet experiments, oocysts are used within 30 days of isolation and disinfected with 1% peracetic acid prior to administration.

### Reference EXAMPLE 12

### In vivo Neutralizing Activity Assays

Each of the monoclonal antibody-biocide fusions based on the r1E10 and r4H9 antibodies and monoclonal antibodies r3E2, r1E10, r4H9, and r3H2 determined to have significant *in vitro* sporozoite neutralizing activity, is individually tested to quantify *in vivo* efficacy against infection. The neonatal mouse model is used. (*See,* M.W. Riggs MW and L.E. Perryman, Infect. Immun., 55:2081-2087 [1987]; and D.A. Schaefer et al., Infect. Immun., 68:2608-2616 [2000]). Groups of 15 six-day-old specific pathogen free ICR mice (Harlan Sprague Dawley) are administered 5 x 10⁴ oocysts (50 X mouse ID₅₀) by gastric intubation. After 48 hrs, culture-derived r3E2 (4 mg MAb/ml, 75 µl) are given by intubation. Every 12 hrs thereafter, mice are administered additional r3E2 (4 mg MAb/ml, 100 µl), for a total of eight treatments. Cimetidine (10 mg/kg) are included with all treatments. For comparison, groups of 15 six-day-old control mice are infected and treated identically with: 1) murine hybridoma-derived neutralizing 3E2, or 2) isotype- and concentration- matched recombinant control MAb of irrelevant specificity. After euthanasia at 140-142 hrs post-inoculation, the jejunum, ileum, cecum, and colon are collected from each mouse and processed for histopathology. Sections are coded and examined by the same investigator, without knowledge of treatment group, for *C.* p*arvum* stages in mucosal epithelium. Scores are assigned to longitudinal sections representing the entire length of: i) terminal jejunum; ii) ileum; iii) cecum; and (iv) colon, then summed to an infection score for each mouse. (*See,* M.W. Riggs MW and L.E. Perryman, Infect. Immun., 55:2081-2087 [1987]; and D.A.. Schaefer et al., Infect. Immun., 68:2608-2616 [2000]). Each experiment is performed twice. Mean infection scores within each experiment are analyzed by Student's one-tailed t test. Mean infection scores between experiments are analyzed by ANOVA. Additionally, all intestinal sections and sections of stomach, liver, and kidney from mice treated with antibody-biocide fusions are examined by an ACVP Board-Certified Veterinary Pathologist to determine if any lesions suggestive of biocide-host toxicity are present.

### Reference EXAMPLE 13

### In vivo Efficacy Assays of rMAbs and rMAb-Fusion Parasiticides in a Neonatal Piglet Model

This example provides *in vivo* efficacy assays for *C. parvum* neutralizing rMAb. Newborn male piglets for the proposed studies are obtained by project personnel at the time of parturition from sows in which the perineum has been thoroughly cleaned using standard methods equivalent to pre-surgical preparation. Piglets, collected as born and colostrum-deprived, are immediately placed in disinfected isolation crates for transport to BSL-2 isolation facilities. Precautions are taken to prevent animal exposure to an exogenous source of *C. parvum* and other potential diarrheal agents. *(See e.g.,* L.E. Perryman et al., Mol. Biochem. Parasitol., 80:137-147 [1996]; L.E. Perryman et al., Vaccine, 17:2142-2149 [1999]; and M.W. Riggs and L.E. Perryman, Infect. Immun., 55:2081-2087 [1987]). Following arrival at BSL-2 isolation facilities, piglets are assigned to either treatment (8 piglets) or control groups (8 piglets) by blind code. Group assignments and coding are made by an independent third party not be involved in conducting the experiments, data collection, or interpretation of results. All personnel involved with the experiments have no knowledge of piglet group assignments. Codes are revealed only at completion of the study. Testing of rMAbs and rMAb-biocide fusion proteins, individually and in combination to be selected, proceeds as follows.

### Testing of individual rMAbs

To allow accurate comparisons between activities of the six rMAb constructs being evaluated, the concentration of each is standardized on an equimolar basis. Using the experimental design for rMAb 3E2 as an example, each construct is evaluated, individually, as follows. One group of 8 piglets is administered 107 oocysts by gastric intubation at 24 hrs of age. Forty-eight hours later, each piglet receives 250 mg culture-derived rMAb 3E2 by intubation. At 12 hrs and every 12 hrs thereafter, each piglet is administered 50 mg additional rMAb 3E2 for a total of 10 treatments (750 mg MAb r3E2 total/piglet). Omeprazole (PRILOSEC, Astra-Merck) [1mg/kg] is administered 6-8 hrs prior to each rMAb treatment to block production of gastric acid according to a regimen previously shown to elevate gastric pH in pigs to ∼7 (D.L. Foss and M.P. Murtaugh, Vaccine, 17:788-801 [1999]). As an additional precaution against gastric degradation, rMAb is formulated in NaHCO₃ buffer prior to administration. For comparison, a group of 8 control piglets is identically infected with 10⁷ oocysts and administered recombinant isotype control MAb construct according to the same treatment regimen as the principals. Piglets are confined, individually, in elevated metabolic isolation cages equipped with fecal collection pans, and maintained on ESBILAC (PetAg, Inc., Hampshire, IL) for the duration of the experiment. To prevent urine from contaminating feces for subsequent analyses, a diversion device is attached and sealed around the prepucial orifice of each piglet to divert urine into a drainage outlet. Piglets are examined twice daily by a veterinarian, without knowledge of treatment group, and assigned numerical scores based on clinical assessment for symptoms of depression, anorexia, and dehydration. Piglet weights at the time of infection and at the end of the experiment are also recorded. The total volume of feces excreted and percent dry matter for successive 24 hrs fecal collections is determined to provide an objective, quantitative index of diarrhea for each piglet. Fecal samples are examined for oocysts prior to challenge and daily thereafter by IFA using oocyst-specific MAb 4D3 to determine pre-patent and patent periods as previously described. *(See,* M.W. Riggs et al., Antimicrob. Agents Chemother., 46:275-282 [2002]). Total oocyst counts (number oocysts per ml of feces X total ml feces) for each piglet is determined from samples of well-mixed feces collected over successive 12 hrs periods (M.W. Riggs *et al*., *supra*). Feces from each piglet is examined for possible bacterial and viral enteropathogens by standard methods. Piglets are euthanized 10 days post-infection, or before if clinically indicated. Sections of duodenum, jejunum, ileum, cecum, and colon from identically sampled sites in each piglet are collected for histopathology. Sections are coded and examined histologically without knowledge of treatment group by an ACVP board-certified veterinary pathologist. Villus length to crypt depth ratios and the density of organisms per unit length of mucosa is determined as previously described *(See,* M.W. Riggs et al., Infect. Immun., 62:1927-1939 [1994]; M.W. Riggs. and L.E. Perryman, Infect. Immun., 55:2081-2087 [1987]). Infection scores of 0, 1, 2 or 3 (0, no infection; 1, < 33% of mucosa infected; 2, 33 to 66% of mucosa infected; and 3, > 66% of mucosa infected) are assigned to longitudinal sections from the (i) terminal jejunum, (ii) ileum, (iii) cecum, and (iv) proximal colon, then summed to obtain an infection score (0 to 12) for each piglet. (M.W. Riggs. and L.E. Perryman, *supra*). Additionally, all intestinal sections, and sections of stomach, liver, and kidney from piglets treated with rMAb-biocide constructs are examined by an ACVP Board-Certified Veterinary Pathologist to determine if any lesions suggestive of biocide-host toxicity are present. Clinical, parasitologic, and histologic data is analyzed statistically by ANOVA using the General Linear Models Program of SAS.

### Testing of combined rMAbs

Following evaluation of the individual rMAbs above, the necessary data is available to decide which rMAbs are the best candidates for testing in combination for additive efficacy. Based on previous findings in mice, an optimal combination comprises up to three MAbs, one against each of the three target antigens (CSL, P23, GP25-200) (L.E. Perryman et al., Mol. Biochem. Parasitol., 80:137-147 [1996]). Because the neutralizing activity of anti-CSL MAb 3E2 is profoundly greater than that of all other Mabs against *C. parvum*, this MAb can be an important component in the selected combination. Either rMAb 1E10 or rMAb 1E10-biocide fusion, whichever demonstrates greater efficacy in the above experiments, is included in the combination to target P23. To target GP25-200, rMAb 3H2, rMAb 4H9, or rMAb 4H9-biocide fusion, whichever demonstrates the greatest efficacy in the above experiments, can be included. Thus, the combination to be evaluated may contain rMAbs 3E2 + 1E10 (standalone or biocide fusion) + 3H2 or 4H9 (standalone or biocide fusion).

Previous studies on MAb combinations show that hybridoma-derived MAbs 3E2, 1E10, 3H2, and 4H9 recognize distinct epitopes and do not inhibit binding of each other to *C. parvum.* Nevertheless, it is useful to repeat binding inhibition experiments with the recombinant candidates selected for combination testing to confirm that they do not inhibit binding of each other due to steric hindrance or other influences introduced by recombinant expression. In brief, this is evaluated by ELISA using biotin-labeled (Sulfo-NHS-biotin, Pierce) and unlabeled rMAb candidates as previously described. (*See,* L.E. Perryman *et al., supra*). Immulon-4 96-well ELISA plates are coated with solubilized sporozoite antigen, washed, and blocked. Plates are incubated with an individual unlabeled rMAb, then biotinylated competitor rMAb, washed, and developed with peroxidase-labeled Streptavidin and substrate. Mean ODs of replicate wells for each treatment and control group are analyzed for significant differences.

After determining that the three rMAbs selected for combination testing in piglets do not significantly inhibit binding of each other, they are combined and the concentration of each standardized on an equimolar basis to match that previously evaluated individually. Efficacy testing of the combined rMAbs in piglets then proceeds as described for individual rMAbs above. In brief, one group of 8 piglets are infected with 10⁷ oocysts at 24 hrs of age and receive the combined rMAbs in NaHCO3 buffer 48 hrs later by intubation. At 12 hrs and every 12 hras thereafter, piglets receive additional combined rMAbs for a total of 10 treatments. For comparison, a group of 8 control piglets are identically infected and administered an appropriate isotype control rMAb combination according to the same treatment regimen. Clinical assessment scores, piglet weights, total volume of feces excreted and percent dry matter for successive 24 hr collections, pre-patent and patent periods, and total oocyst counts are determined as above. Ten days post-infection, sections of duodenum, jejunum, ileum, cecum, and colon from each piglet are collected, and examined histologically to assess lesions and assign infection scores. Tissues are also examined to determine if any lesions suggestive of biocide-host toxicity are present. Clinical, parasitologic, and histologic data is analyzed statistically as described above. Data from testing of the individual rMAbs is compared with data from testing of the rMAb combination by one-way ANOVA stratified by treatment group.

### EXAMPLE 14

### Targeted Biocides Using Innate Receptor Recognition

This Example describes the construction and analysis of fusion proteins of an innate receptor and a biocide.

### A. Genetic engineering of sCD14, LBP, SP-D and MBL into retrovirus backbone for secretion

All the innate receptor molecules were previously cloned and produced in various cell lines as recombinant molecules. The gene for the human CD 14 receptor is obtained from total RNA extracted from human PBMCs. Following reverse transcription of the RNA into cDNA, the specific gene for CD 14 is cloned by PCR. Primers are designed to amplify the sequence starting with the signal peptide sequence down to the first codon of the GPI anchor. The GPI anchor is excluded to facilitate secretion. The GPI anchor sequence is replaced by a portion of the human immunoglobulin Fc region. Adding Ig domains to proteins does not interfere with proper folding; an added Fc portion can contribute to the stability of a fusion protein and extend its half-life (*e.g.*, Chang et al., Surgery 2002; 132:149-56). The hinge region, CH2 and CH3 domains of human immunoglobulin are already part of a construct library and are transferred to the CD 14 receptor construct. Constructs that contain the Fc portion with a biocide already connected are available from prior work.

LPS binding protein (LBP; accession number NM_004139) naturally occurs as a secreted protein. The amino acid sequence of the secreted protein is known (Schumann et al., Science 1990; 249:1429-31) and the secreted form has been produced as a recombinant protein (Han et al., J Biol Chem 1994; 269:8172-5; Theofan et al., J Immunol 1994; 152:3623-9). The gene for LBP is cloned from the mammalian gene collection construct into the retroviral construct. As LBP is a secreted protein, it is not necessary to attach an immunoglobulin Fc portion to stabilize it. The linker-biocide portion is attached directly to the C-terminus of LBP.

SP-D and MBL are from the defense collectin family. These molecules form multimers, increasing their overall avidity to the pathogen surface. Surfactant protein D, SP-D has a glomerular structure at its c-terminal end. This structure is thought to interact with LPS either in solution or as part of a pathogen surface. If the linker-biocide portion is added to the c-terminal end it will most likely be very close to the binding site. An N-terminally attached biocide version of the fusion protein is also produced. SP-D can obtain a cross-like tetrameric conformation. Based on electron microscopy images (Holmskov et al., Annu Rev Immunol 2003; 21:547-78) bound SP-D adopts a fairly two-dimensional structure when bound to its specific surface, bringing the N-termini close to the surface as well. An N-terminally attached biocide is thus accessible enough to destroy a bacterial membrane.

The mannan-binding lectin (MBL) is also a collectin, forming multimeric complexes to achieve a highly efficient binding to microorganism surfaces. The gene for MBL is obtained from the human gene collection, accession number 67483. Like the other collectins MBL is a soluble secreted protein therefore not requiring modifications to make it soluble. The gene is used in its native confirmation and the biocide is added via the linker. The same cloning strategy as that described above for SP-D is used.

### B. Expression of riR in vitro - test binding to bacterial components

The retroviral constructs containing the genes for the innate receptor (or the complete construct containing the Fc-portion plus biocide) are co-transfected with the VSV-G envelope plasmid into the packaging cell line and infectious retroviral particles are produced by transient expression. Packaging cells are grown to exponential phase and then exposed to a calcium chloride solution containing a mixture of the VSV-G encoding plasmid and the retroviral construct containing the immunoglobulin genes. Cells are then grown for 16-24 hours until pseudotyped retrovector is harvested from the supernatant over a period of several days. The titers resulting are typically in the range of 10⁵ - 10⁶ infectious units per ml culture media. Media is concentrated to be used at high multiplicity of infection on the target production cell line (CHO or 293 cells). Once these production cells have been exposed to high titer retrovirus (transduction), they start to express product (monoclonal antibody or others) typically in the range of 10-25 µg/ml for well-expressed monoclonal antibody molecules in standard plastic tissue culture vessels. The pool population of transduced production cells are subjected to clonal analysis to obtain high-level producing clones.

The recombinant innate receptor molecules (riR) are tested for their interaction with target cells (bacteria), first by enzyme linked immunosorbent assay (ELISA). For this assay, the cell product is first quantified. The natural antigen (*e.g.*, whole *E. coli* cells) is used as a capture agent to monitor specificity. A similar assay has been established to monitor *Listeria* surface antigens. Briefly, antigenic fractions or whole bacteria are used to coat 96-well plates. After washing, samples containing riR are incubated at serial dilutions with the antigen. Secondary conjugates are used to quantify binding. Flow cytometry analysis allows the measurement of the number of riR molecules interacting with whole microorganism cells. Assays are designed to compare all the different recombinant innate receptor candidates simultaneously against one or multiple different targets (*e.g.* different *E. coli* isolates). These assays identify which candidates (clonal lines) are suitable for the cloning procedures described below.

### C. Reengineer riR construct to contain biocide fusion portion

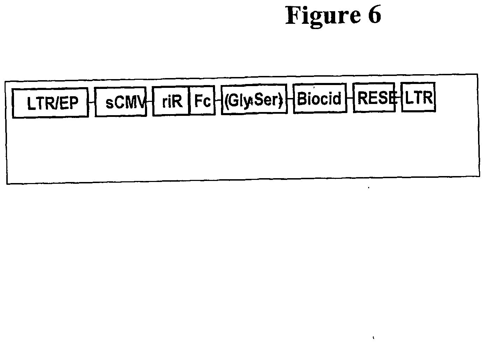

Upon determining which of the riR retain good binding capabilities when expressed in mammalian tissue culture, the corresponding genetic constructs are modified to contain the gene for one of the two biocides. Biocides are attached to the riRs at either the N-terminus or at the C-terminus of the riR. The CD14 riR is made with a C-terminal Fc-portion that helps stabilize it. Therefore CD14 is made as a C-terminal biocide only. The other candidates LBP, MBL and SP-D are made as both N-terminal and C-terminal fusions. Figure 6 shows the components of these constructs featuring a (Gly₄Ser)₃ linker which has been used widely. The design of the linker is optimized for functionality of the fusion protein. The linker can be modified as described (George and Heringa, Protein Eng 2002; 15:871-9). Alternatively, the linker is designed with a symmetric sequence of (Gly₄Ser)₂-P-P-(Gly₄Ser)₂ placing the most favored amino acid pair (a structure breaking Pro-Pro) in the middle of the non-helical linkers. Phospholipase and lysozyme constructs are expressed in cell culture to use as controls.

### D. Expression of riR-biocide fusions and binding tests

The constructs for the riR-biocide fusions are introduced into a mammalian tissue culture system as described above. Upon production of milligram amounts of riR-biocide fusions, binding tests including ELISA and flow cytometry are done. In addition precise affinity measurements are undertaken using surface plasmon resonace (SPR) on a Biacore 2000 machine. This allows for the determination of which riR-biocide binds best to its target, and also if the linker and fusion elements change the original affinity. Multiple bacterial surface-binding innate immunity receptors are expressed in mammalian tissue culture. In addition, multiple riR-biocide fusion proteins are assembled.

### Reference EXAMPLE 15

### Targeted Biocides using Antibody Targeting PAMPs

This Example describes the construction and analysis of constructs comprising monoclonal antibodies and biocides.

### A. Antigens for immunization

Antigens that are not specific to one bacterial isolate but rather common to a broad group are identified (*e.g.*, all Gram negatives or all Gram positives, or all of a major family), in order to obtain a broadly reactive monoclonal antibody. Such targets include structural components such as lipopolysaccharides, peptidoglycans, porins that are common in structure and function among the classes of bacteria (Feng et al., J Gen Microbiol 1990; 136 (Pt 2):337-42; Klebba et al., J Biol Chem 1990; 265:6800-10; Peters et al., Infect Immun 1985; 50:459-66). Preferred targets are genetically defined and can either be purified directly or over-expressed in an *E.coli* expression system. Both of these methods can be used to immunize mice (Feng et al., J Gen Microbiol 1990; 136 (Pt 2):337-42; He et al., Appl Environ Microbiol 1996; 62:3325-32). For dominant structural components minimal purification is needed to arrive at an antigen preparation that has a strong probability of providing cross reactive antibodies. In some embodiments, *E.coli* O157:H7 and *L. monocytogenes* are utilized as PAMP "donors".

### B. Immunize mice to produce monoclonal broad spectrum antibody

An array of monoclonal antibodies is prepared by Neoclone (Madison, WI). The monoclonal antibodies are used to screen for reactivity with an array of target organisms.

### C. Antibody binding tests against wide variety of microorganisms, selection of best candidates

Monoclonal antibodies are obtained as ascitic fluid containing high levels of monoclonal antibody plus the corresponding immortalized B-cells. The antibody contained in the ascitic fluid is used to perform specificity and affinity tests on various targets. Multiple strains of pathogenic *E. coli, Salmonella, Listeria,* and *Lactobacillus* are used for testing. Initial tests include ELISA assays to establish general binding patterns. The antigenic structures that were used for the immunization of the mice leading to these monoclonals are well defined and the distribution patterns over the surface of a bacterial cell are known. Based on this and what is known about other monoclonals to similar or the same antigenic structures, the quantity of interaction expected can be estimated. In addition, commercially available monoclonal antibodies to these structures are used as controls (*e.g.*, those available from Inotek Pharmaceuticals, Beverly, MA). It is contemplated that some of the monoclonals will have a broad range of cross-reactions with other organisms. This has been described on various occasions, especially between *E. coli* and other *Enterobacteriacae* (Feng *et al.,* supra; Klebba et al., J Biol Chem 1990; 265:6800-10), but also between *E. coli* and *Salmonella* (O-antigens) (Westerman et al., J Clin Microbiol 1997; 35:679-84). The cross reactivity increases the number of potential target organisms that can be treated with one single broad-reacting monoclonal antibody.

### D. Clone immunoglobulin genes from candidates into retroviral backbone - express in vitro - test binding

The heavy and light chain variable regions of the immunoglobulin genes are amplified out of the cDNA by PCR using a commercially available Ig-primer kit. These primers consist of degenerate upper primers specific to the signal-peptide sequence at the 5'-end of the immunoglobulin heavy- or light chain and lower primers to different sections in the constant region, depending on the section that is to be isolated. A lower primer to the joining region is chosen to amplify just the variable region. Alternativley, a lower primer to the 3' UTR of the heavy- or light chain gene is chosen to amplify the entire framework. Once the PCR products are obtained using high-fidelity polymerase that ensures that error-free amplicons are generated, they are cloned into blunt end PCR cloning kits that are commercially available. Upon cloning of these fragments, multiple clones are analyzed by sequencing and sequences aligned with each other for comparison. Once the complete constructs have been confirmed by sequencing they are used in the first round of transfections that result in high titer pseudotyped retrovector for the transduction of production cell lines.

### E. Reengineer the antibody constructs to contain biocide fusions

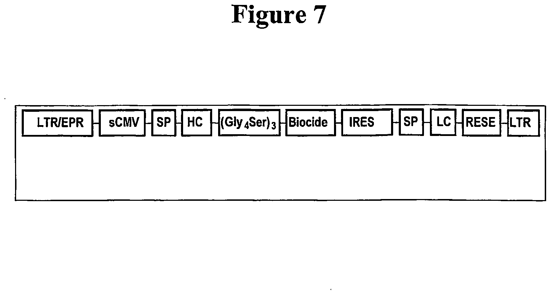

The heavy chain gene is removed from the construct and replaced with a fusion that consists of the heavy chain gene, a C-terminal linker element and the gene of either human lysozyme or human phospholipase A (Figure 7). The heavy chain-linker-lysozyme element is separated from the light chain gene via the IRES (internal ribosome binding site) element that enables individual translation of both products. The C terminus is used as it is least likely to give rise to steric hindrance at the antibody binding site. The (Gly₄Ser)₃ linker is used.

### F. Expression of biocide fusions and binding tests

The retrovector is used to transduce production cell lines (CHO or 293) and obtain cells that have stably integrated copies of the construct. Upon clonal analysis to select clones with the highest production of fusion protein, the products are again tested for affinity and specificity as described in the above examples. Surface plasmon resonance analysis is used to determine exactly what influence the linker and biocide fusion portion have on the binding affinity of the antibody portion.

### Reference EXAMPLE 16

### Targeted biocides using secretory IgA and pentameric IgM as delivery molecules

The example describes the construction and analysis of constructs comprising IgA and IgMs and biocides.

### A. Obtain secretory component (SC) through engineering of pIgR gene and clone into retroviral backbone

The cloning strategy for the murine polymeric immunoglobulin receptor (pIgR) gene (accession U06431) is based on published information about the sequence, structure and interaction of murine pIgR with IgA (Piskurich et al., J Immunol 1995; 154:1735-47). As a source for the pIgR gene total RNA is isolated from murine liver cells, which have been shown to produce high levels of pIgR (Piskurich *et al.,* supra). After reverse transcribing the RNA, PCR is used to obtain the gene for the pIgR. Primers specific to the 3' UTR region and to the region upstream of position 2020 of the transmembrane region are designed and used to amplify a truncated version of the pIgR. The downstream primer is designed to introduce a stop codon right after amino acid position 594. The sequence corresponds to the cleaved secretory component found in circulation. A similar procedure has been published to make human secretory IgA (Rindisbacher et al., J Biol Chem 1995; 270:14220-8).

### B. Create secretory component-producing cell line by using existing J-chain-biocide producing cell line

The truncated pIgR gene obtained as described above is cloned into the retroviral backbone behind the simian CMV promoter as described earlier. CHO cells that already produce J-chain linked to biocide are superinfected with the pIgR construct and ELISA and Western Blot-based clonal analysis are performed to find clones that produce similar amounts of J-chain and secretory component. The resulting SC and J-chain-biocide producing cell line is used as a recipient cell line for the IgA constructs.

### C. Use this cell line as recipient for IgA constructs

The next step towards a secretory IgA-producing cell line is to transduce the SC+J-chain-biocide producing cell line with reengineered IgA that is made by genetic assembly as described below. This construct is the first "fully artificially" produced mouse secretory IgA that incorporates all three components, the secretory component, the J-chain and a rearranged IgA, in one single production cell line. In addition it is the first time that the J-chain is engineered into a fusion protein.

### D. Express secretory IgA-biocide fusion in vitro and test binding

Production cell populations producing sIgA-biocide are subjected to clonal analysis in order to choose the highest producers. The selection of these clones focuses on dimeric secretory IgA-biocide detection. In order to make sure that the reengineered sIgA-biocide maintains its binding capabilities to bacterial surfaces, supernatants containing sIgA-biocide are tested by ELISA and flow cytometry as described above. Once satisfactory binding characteristics have been confirmed, candidates are chosen to go into the extensive testing series described below.

### E. Engineer variable regions from PAMP-reactive immunoglobulins onto constant IgA or IgM region

The constant IgA regions are obtained through extraction from IgA producing hybridomas. The immortalized monoclonal antibody-producing cell lines generated as described above are the source for the variable region genes. To obtain the variable regions from these cells the same procedures as described above with the exception that lower primers that anneal to the hinge region so that only the variable portion of the gene is amplified are used. The products are then cloned in frame into the existing IgA and IgM constant region constructs. These constructs representing anti-PAMP IgA are used to make sIgA-biocide fusion in cells that make SC and J-chain-biocide. Since the IgM does not incorporate the secretory component, the constructs representing anti-PAMP on an IgM framework are used to transduce production cells that make only the J-chain-biocide.

### F. Express pentameric IgM-biocide fusion in vitro and test binding

The anti-PAMP IgM constructed above is used to transduce JC-biocide producing production cells in analogy to the procedures described in Example 14. Product is tested for the pentameric structure and binding capabilities to PAMPs as well as whole bacterial cells. Candidates that are chosen based on their good binding and structure are taken into the extensive testing series described below.

### EXAMPLE 17

### Bactericidal Testing System

This example describes testing methods for the analysis of candidate fusion proteins identified as described above. Constructs developed as described above are evaluated under the same conditions. Three test systems are used, as described below:

### A. Testing in Bacterial Cell Culture

The lowest stringency test evaluates the bactericidal effect on test organisms in culture. Cultures of the test organism are exposed to a range of concentrations of the test product and control media for times ranging from 1-24 hours at 4°C and 10°C. Aliquots in triplicate are re-plated on a growth medium to quantify the residual bacteria. The cell suspensions are also examined microscopically for clumping; various techniques are used to separate cells for quantification.

### B. Testing in Biofilms and Ground Meat

For *Listeria* and *Lactobacillus,* the efficacy of constructs is tested in a biofihn model system; for *Lactobacillus, E. coli* and *Salmonella* the efficacy of bacterial killing is tested in a ground meat suspension.

Protocols for biofilm development and antimicrobial testing are known (Somers *et al*., 88th Ann. Meet. International Association for Food Protection, Minneapolis, MN. Abstract P055. 2001). Stainless steel is used as a prototype surface for initial evaluation of the anti-listerial activity. Biofilms comprising a cocktail of *L. monocytogenes* or *Lactobacillus* isolates are established on 1cm² pre-sterilized steel chips. Biofilm chips are exposed to various concentrations of the fusion protein products for up to 2 hours at room temperature. Chips are retrieved and evaluated for the number of viable cells quantified; adherent cells are removed by vortexing with glass beads and appropriate dilutions of the detached cells plated on nutrient agar plates for enumeration.

Raw beef or turkey is harvested from the interior of a large muscle section under sterile conditions to keep the background contamination at a minimum. This meat is then comminuted under sterile conditions to a slurry and pH adjusted. Several concentrations of the test products are added to the slurries. Following inoculation with test organisms the samples are incubated at 4° and 10°C for up to 4 weeks. Triplicate samples per variable are assayed weekly for changes in bacterial population.

### C. Simulation of Packing Plant Conditions

Meat product suspensions or a range of surface presentations of bacteria are used to simulate plant equipment and facilities. SPR testing of affinity binding guides the range of conditions tested. SPR is used to measure the effect of low or high pH, high salt concentrations and high temperatures on the capability of a given fusion protein to 'hang on' to its epitope. Those conditions that still allow the fusion protein to bind to its target are then tested in separate in vitro experiments for bacterial killing. For biofilms factors to be evaluated are attachment surface (*e.g.*, buna N and food grade silicone rubber, polyurethane, polyester, polyethylene, and polypropylene), presence of food residues, cleaning/sanitizing agents, temperature, pH, and mixed biofilms.

Test organisms include, but are not limited to, field isolates of *E. coli* O157:H7, *Salmonella*, *Listeria monocytogenes* and *Lactobacillus plantarum.* Multiple isolates of the relevant organisms are available.

## Claims

1. A composition comprising a recombinant fusion protein, wherein said protein comprises a microorganism targeting molecule joined to at least a portion of a protein biocide molecule, wherein said microorganism targeting molecule binds to bacteria, and wherein said microorganism targeting molecule is CD14, and wherein said fusion protein neutralizes said bacteria.

2. The composition of claim 1, wherein said microorganism targeting molecule and said at least a portion of a protein biocide molecule are joined by a poly amino acid linker molecule from 2 to 500 amino acids long.

3. The composition of claim 2, wherein said poly amino acid linker molecule is from 5 to 100 amino acids long, preferably from 10 to 30 amino acids long.

4. The composition of claim 2, wherein said poly amino acid linker molecule consists of amino acids selected from the group consisting of Gly, Ser, Asn, Thr, Ala, and Pro.

5. The composition of claim 2, wherein said amino acid linker comprises a sequence of amino acid residues having the formula:
(Serₙ-Glyₓ)_{y}
wherein n ≥ 1,
wherein x ≥ 1, and
wherein y ≥ 1.

6. The composition of claim 5, wherein n =1, wherein x = 4, and wherein y ≥ 1.

7. The composition of claim 5, wherein y = 1, 2, 3, 4, 5, 6, 7, or 8.

8. The composition of claim 1, wherein said protein biocide is selected from the group consisting of: lysozyme, phopholipase A2, lactoferrin, lactoperoxidase, bacterial permeability increasing protein, lysostaphin, and aprotinin.

9. A vector construct comprising a nucleic acid sequence encoding a microorganism targeting molecule linked to a portion of a biocide molecule, wherein said microorganism targeting molecule binds to bacteria, and wherein said microorganism targeting molecule is CD14, and wherein said fusion protein neutralizes said bacteria.

10. The vector of claim 9, wherein said vector is a retroviral vector.

11. A method of treating an object, wherein said object is not a living animal, comprising:
a) providing
i) a composition of claim 1; and
ii) a non-living object suspected of being contaminated with bacteria; and
b) applying said composition to said object under conditions such that said recombinant fusion protein neutralizes said bacteria suspected of contaminating said object.

12. The method of claim 11, wherein said object is selected from the group consisting of food processing equipment, military equipment, personal protective gear, medical devices, domestic objects, and building structures.

13. The method of claim 12, wherein said domestic object is selected from the group consisting of an appliance and a surface.

14. The method of claim 12, wherein said building structure is selected from the group consisting of heating and ventilation equipment, a wall or wall cavity, and a plumbing system.

15. The method of claim 11, wherein said object is an animal carcass or part thereof.

16. The method of claim 15, wherein said animal carcass comprises a bovine carcass or part thereof, a porcine carcass or part thereof, an avian carcass or part thereof, or an aquatic animal carcass or part thereof.

17. The method of claim 11, wherein the object is a food product.

18. The method of claim 17, wherein said food product is selected from the group consisting of a meat product, a processed meat product, vegetable, leaf, stem, seed, fruit, root, beer, wine, a dairy product, and animal feed.

19. The method of claim 17, wherein said food product is in the process of being produced.

20. A fusion protein comprising a microorganism targeting molecule linked to a portion of a biocide molecule, wherein said microorganism targeting molecule binds to bacteria, and wherein said microorganism targeting molecule is CD14, and wherein said fusion protein neutralizes said bacteria for use in treating a subject of being infected with a bacteria.

21. The fusion protein of claim 20, wherein said subject is a mammal, a ruminant, a bovine, a human, or an avian species.

22. The fusion protein of claim 20, wherein said subject is suspected of being infected with antibiotic resistant bacteria.

23. The fusion protein of claim 20, wherein said subject is suspected of being infected with artificially engineered bacteria.

24. The fusion protein of claim 20, wherein said subject is deceased.

25. A method of supplementing a food ration comprising:
a) providing
i) a recombinant fusion protein comprising a microorganism targeting molecule joined to at least a portion of a biocide molecule by a poly amino acid linker molecule, wherein said microorganism targeting molecule binds to bacteria, and wherein said microorganism targeting molecule is CD14, and wherein said fusion protein neutralizes said bacteria; and
ii) a food ration; and
b) supplementing said food ration with said recombinant fusion protein.

26. A food comprising a composition of claim 1 and at least one foodstuff.

27. The food of Claim 26, wherein said food is for humans, a farm animal, or a companion animal.

28. A composition comprising a milk protein and a composition of claim 1.

29. A method of treating a plant or a part of a plant, comprising:
a) providing
i) a recombinant fusion protein, wherein said protein comprises a microorganism targeting molecule joined to at least a portion of a biocide molecule, wherein said microorganism binding molecule is CD14 and binds to bacteria; and
ii) a plant suspected of being infected or contaminated by bacteria; and
b) applying said recombinant fusion protein to said plant or plant part under conditions such that said recombinant fusion protein neutralizes said bacteria.

30. The method of claim 29, wherein said plant or plant part is selected from the group consisting of a seed, a vegetable, a stem, a leaf, a root, a growing plant, and a fruit.

31. A transgenic non-human organism comprising a nucleic acid sequence encoding a microorganism targeting molecule linked to at least a portion of a protein biocide molecule, wherein said microorganism targeting molecule binds to bacteria, and wherein said microorganism targeting molecule is CD14, and wherein said fusion protein neutralizes said bacteria.

32. The transgenic organims of claim 31, wherein said transgenic organism is selected from the group consisting of an animal, a plant, and a microorganism.

## Patentansprüche

1. Zusammensetzung, umfassend ein rekombinantes Fusionsprotein, wobei das Protein ein auf ein Mikroorganismus zielendes Molekül umfasst, das zumindest an ein Teil eines Biozidproteinmoleküls gekoppelt ist, wobei das auf den Mikroorganismus zielende Molekül an Bakterien bindet und wobei das auf den Mikroorganismus zielende Molekül CD14 ist und wobei das Fusionsprotein die Bakterien neutralisiert.

2. Zusammensetzung nach Anspruch 1, wobei das auf den Mikroorganismus zielende Molekül und der Mindestteil des Biozidproteinmoleküls mittels eines Polyaminosäureverknüpfungsmoleküls mit einer Länge von 2 bis 500 Aminosäuren gekoppelt sind.

3. Zusammensetzung nach Anspruch 2, wobei das Polyaminosäureverknüpfungsmolekül 5 bis 100 Aminosäuren lang ist, vorzugsweise 10 bis 30 Aminosäuren lang ist.

4. Zusammensetzung nach Anspruch 2, wobei das Polyaminosäureverknüpfungsmolekül aus Aminosäuren besteht, die ausgewählt sind aus der Gruppe bestehend aus Gly, Ser, Asn, Thr, Ala und Pro.

5. Zusammensetzung nach Anspruch 2, wobei der Aminosäureverknüpfer eine Sequenz von Aminosäureresten mit der Formel umfasst:
(Serₙ-Glyₓ)_{y}
wobei n ≥ 1 ist,
wobei x ≥ 1 ist und
wobei y ≥ 1 ist.

6. Zusammensetzung nach Anspruch 5, wobei n =1 ist, x = 4 ist und wobei y ≥ 1 ist.

7. Zusammensetzung nach Anspruch 5, wobei y = 1, 2, 3, 4, 5, 6, 7 oder 8 ist.

8. Zusammensetzung nach Anspruch 1, wobei das Biozidprotein ausgewählt ist aus der Gruppe bestehend aus: Lysozym, Phopholipase A2, Lactoferrin, Lactoperoxidase, bakterielles, die Permeabilität erhöhendes Protein, Lysostaphin und Aprotinin.

9. Vektorkonstrukt, umfassend eine Nukleinsäuresequenz, die für ein auf ein Mikroorganismus zielendes Molekül kodiert, das an ein Teil eines Biozidmoleküls gekoppelt ist, wobei das auf den Mikroorganismus zielende Molekül an Bakterien bindet und wobei das auf den Mikroorganismus zielende Molekül CD14 ist und wobei das Fusionsprotein die Bakterien neutralisiert.

10. Vektor nach Anspruch 9, wobei der Vektor ein retroviraler Vektor ist.

11. Verfahren zur Behandlung eines Objekts, wobei das Objekt kein lebendes Tier ist, umfassend:
a) die Bereitstellung
i) einer Zusammensetzung nach Anspruch 1; und
ii) eines nicht lebenden Objekts, das verdächtig wird mit Bakterien kontaminiert zu sein; und
b) die Behandlung des Objekts mit der Zusammensetzung unter solchen Bedingungen, dass das rekombinante Fusionsprotein die Bakterien neutralisiert, die verdächtigerweise das Objekt kontaminieren.

12. Verfahren nach Anspruch 11, wobei das Objekt ausgewählt ist aus der Gruppe bestehend aus Equipment für die Lebensmittelverarbeitung, Militärequipment, persönlichen Schutzein- und Schutzausrüstungen, medizinischen Vorrichtungen, häuslichen Objekten und Gebäudeeinrichtungen/Bauwerken.

13. Verfahren nach Anspruch 12, wobei die häuslichen Objekte ausgewählt sind aus der Gruppe bestehend aus einem Apparat/Mittel/Einrichtung und einer Oberfläche.

14. Verfahren nach Anspruch 12, wobei die Gebäudeeinrichtung / das Bauwerk ausgewählt ist aus der Gruppe bestehend aus Heizungs- und Lüftungsequipment, einer Wand oder einem Hohlraum/ einer Aussparung in der Wand und einem Installationssystem.

15. Verfahren nach Anspruch 11, wobei das Objekt ein Tierkörper oder ein Teil davon ist.

16. Verfahren nach Anspruch 15, wobei der Tierkörper ein Körper eines Rindes oder Teils davon, ein Körper eines Schweins oder Teils davon, ein Geflügelkörper oder Teils davon oder ein Körper eines im Wasser lebenden Tieres oder Teils davon ist.

17. Verfahren nach Anspruch 11, wobei das Objekt ein Nahrungsmittel ist.

18. Verfahren nach Anspruch 17, wobei das Nahrungsmittel ausgewählt ist aus der Gruppe bestehend aus einem Fleischprodukt, einem verarbeiteten Fleischprodukt, Gemüse, Blatt, Sprosse oder Halm, Samen, Obst, Wurzel, Bier, Wein, einem Molkereiprodukt und Tierfutter.

19. Verfahren nach Anspruch 17, wobei das Nahrungsmittel einem Herstellungsprozess unterliegt.

20. Fusionsprotein, umfassend ein auf einen Mikroorganismus zielendes Molekül, das an ein Teil eines Biozidmoleküls gekoppelt ist, wobei das auf den Mikroorganismus zielende Molekül an Bakterien bindet und wobei das auf den Mikroorganismus zielende Molekül CD14 ist und wobei das Fusionsprotein die Bakterien neutralisiert zur Verwendung in der Behandlung eines mit einem Bakterium infizierten Objekts.

21. Fusionsprotein nach Anspruch 20, wobei das Objekt ein Säugetier, ein Wiederkäuer, ein Rind, ein Mensch oder eine Geflügelart ist.

22. Fusionsprotein nach Anspruch 20, wobei das Objekt verdächtigt wird mit gegen Antibiotika resistenten Bakterien infiziert zu sein.

23. Fusionsprotein nach Anspruch 20, wobei das Objekt verdächtigt wird mit künstlich konstruierten Bakterien infiziert zu sein.

24. Fusionsprotein nach Anspruch 20, wobei das Objekt gestorben ist.

25. Verfahren zur Ergänzung einer Nahrungsration, umfassend:
a) die Bereitstellung
i) eines rekombinanten Fusionsproteins, umfassend ein auf ein Mikroorganismus zielendes Molekül, das zumindest an einen Teil eines Biozidmoleküls mittels eines Polyaminosäureverknüpfungsmoleküls gekoppelt ist, wobei das auf den Mikroorganismus zielende Molekül an Bakterien bindet und wobei das auf den Mikroorganismus zielende Molekül CD14 ist und wobei das Fusionsprotein die Bakterien neutralisiert; und
ii) einer Nahrungsration; und
b) die Ergänzung der Nahrungsration mit dem rekombinanten Fusionsprotein.

26. Nahrung, umfassend eine Zusammensetzung nach Anspruch 1 und mindestens ein Nahrungsmittel.

27. Nahrung nach Anspruch 26, wobei die Nahrung für Menschen, Nutztiere oder Haustiere ist.

28. Zusammensetzung, umfassend ein Milchprotein und eine Zusammensetzung nach Anspruch 1.

29. Verfahren zur Behandlung einer Pflanze oder eines Teils einer Pflanze, umfassend:
a) die Bereitstellung
i) eines rekombinanten Fusionsproteins, wobei das Protein ein auf einen Mikroorganismus zielendes Molekül umfasst, das zumindest an einen Teil eines Biozidmoleküls gekoppelt ist, wobei das auf den Mikroorganismus zielende Molekül CD14 ist und an Bakterien bindet; und
ii) einer Pflanze, die verdächtigt wird mit Bakterien infiziert oder kontaminiert zu sein; und
b) die Behandlung der Pflanze oder des Pflanzenteils mit dem rekombinanten Fusionsprotein unter solchen Bedingungen, dass das rekombinante Fusionsprotein die Bakterien neutralisiert.

30. Verfahren nach Anspruch 29, wobei die Pflanze oder der Teil der Pflanze ausgewählt ist aus der Gruppe bestehend aus einem Samen, einem Gemüse, einer Sprosse oder einem Halm, einem Blatt, einer Wurzel, einer wachsenden Pflanze und einer Frucht oder Obst.

31. Transgener nicht-menschlicher Organismus, umfassend eine Nukleinsäuresequenz, die für ein auf einen Mikroorganismus zielendes Molekül kodiert, das zumindest an einen Teil eines Biozidproteinmoleküls gekoppelt ist, wobei das auf den Mikroorganismus zielende Molekül an Bakterien bindet und wobei das auf den Mikroorganismus zielende Molekül CD14 ist und wobei das Fusionsprotein die Bakterien neutralisiert.

32. Transgener Organismus nach Anspruch 31, wobei der transgene Organismus ausgewählt ist aus der Gruppe bestehend aus einem Tier, einer Pflanze und einem Mikroorganismus.

## Revendications

1. Composition comprenant une protéine de fusion recombinante, où ladite protéine comprend une molécule ciblant un micro-organisme jointe à au moins une partie d'une molécule de protéine biocide, où ladite molécule ciblant un micro-organisme se lie à des bactéries, et où ladite molécule ciblant un micro-organisme est CD14, et où ladite protéine de fusion neutralise lesdites bactéries.

2. Composition selon la revendication 1, dans laquelle ladite molécule ciblant un micro-organisme et ladite au moins une partie d'une molécule de protéine biocide sont jointes par une molécule de connecteur de poly(acides aminés) de 2 à 500 acides aminés de long.

3. Composition selon la revendication 2, dans laquelle ladite molécule de connecteur de poly acides aminés a une longueur de 5 à 100 acides aminés, de préférence une longueur de 10 à 30 acides aminés.

4. Composition selon la revendication 2, dans laquelle ladite molécule de connecteur de poly acides aminés est constituée d'acides aminés choisis dans le groupe constitué par Gly, Ser, Asn, Thr, Ala et Pro.

5. Composition selon la revendication 2, dans laquelle ledit connecteur d'acides aminés comprend une séquence de résidus d'acides aminés ayant la formule :
(Serₙ-Glyₓ)_{y}
dans laquelle n ≥ 1,
dans laquelle x ≥ 1, et
dans laquelle y ≥ 1.

6. Composition selon la revendication 5, dans laquelle n = 1, dans laquelle x = 4 et dans laquelle y ≥ 1.

7. Composition selon la revendication 5, dans laquelle y = 1, 2, 3, 4, 5, 6, 7 ou 8.

8. Composition selon la revendication 1, dans laquelle ladite protéine biocide est choisie dans le groupe constitué par : le lysozyme, la phospholipase A2, la lactoférine, la lactoperoxydase, la protéine augmentant la perméabilité bactérienne, la lysostaphine et l'aprotinine.

9. Assemblage de construction d'un vecteur comprenant une séquence d'acide nucléique codant pour une molécule ciblant un micro-organisme liée à une partie d'une molécule biocide, où ladite molécule ciblant un micro-organisme se lie à des bactéries, et où ladite molécule ciblant un micro-organisme est CD14, et où ladite protéine de fusion neutralise lesdites bactéries.

10. Vecteur selon la revendication 9, où ledit vecteur est un vecteur rétroviral.

11. Procédé de traitement d'un objet, où ledit objet n'est pas un animal vivant, comprenant :
a) la fourniture
- i) d'une composition selon la revendication 1 ; et
- ii) d'un objet non vivant suspecté d'être contaminé par des bactéries ; et
b) l'application de ladite composition sur ledit objet dans des conditions telles que ladite protéine de fusion recombinante neutralise lesdites bactéries suspectées de contaminer ledit objet.

12. Procédé selon la revendication 11, dans lequel ledit objet est choisi dans le groupe constitué par un équipement de traitement d'aliments, un équipement militaire, un équipement de protection personnelle, des dispositifs médicaux, des objets domestiques et des structures de construction.

13. Procédé selon la revendication 12, dans lequel ledit objet domestique est choisi dans le groupe constitué par un appareil et une surface.

14. Procédé selon la revendication 12, dans lequel ladite structure de construction est choisie dans le groupe constitué par un équipement de chauffage et de ventilation, un mur ou une cavité murale, et un système de plomberie.

15. Procédé selon la revendication 11, dans lequel ledit objet est une carcasse d'animal ou une partie de celle-ci.

16. Procédé selon la revendication 15, dans lequel ladite carcasse d'animal comprend une carcasse bovine ou une partie de celle-ci, une carcasse porcine ou une partie de celle-ci, une carcasse aviaire ou une partie de celle-ci ou une carcasse d'animal aquatique ou une partie de celle-ci.

17. Procédé selon la revendication 11, dans lequel l'objet est un produit alimentaire.

18. Procédé selon la revendication 17, dans lequel ledit produit alimentaire est choisi dans le groupe constitué par un produit de viande, un produit de viande traitée, un légume, une feuille, une tige, une graine, un fruit, une racine, de la bière, du vin, un produit laitier et des aliments pour animaux.

19. Procédé selon la revendication 17, dans lequel ledit produit alimentaire est dans un processus de production.

20. Protéine de fusion comprenant une molécule ciblant un micro-organisme liée à une partie d'une molécule biocide, où ladite molécule ciblant un micro-organisme se lie à des bactéries, et où ladite molécule ciblant un micro-organisme est CD14, et où ladite protéine de fusion neutralise lesdites bactéries pour une utilisation dans le traitement d'un sujet infecté par des bactéries.

21. Protéine de fusion selon la revendication 20, où ledit sujet est un mammifère, un ruminant, un bovin, un être humain ou une espèce aviaire.

22. Protéine de fusion selon la revendication 20, où ledit sujet est suspecté d'être infecté par des bactéries résistantes aux antibiotiques.

23. Protéine de fusion selon la revendication 20, où ledit sujet est suspecté d'être infecté par des bactéries modifiées artificiellement.

24. Protéine de fusion selon la revendication 20, où ledit sujet est décédé.

25. Procédé de complémentation d'une ration alimentaire comprenant :
a) la fourniture
- i) d'une protéine de fusion recombinante comprenant une molécule ciblant un micro-organisme jointe à au moins une partie d'une molécule biocide par une molécule de connecteur de poly acides aminés, où ladite molécule ciblant un micro-organisme se lie aux bactéries, et où ladite molécule ciblant un micro-organisme est CD14, et où ladite protéine de fusion neutralise lesdites bactéries ; et
- ii) d'une ration alimentaire ; et
b) la complémentation de ladite ration alimentaire avec ladite protéine de fusion recombinante.

26. Aliment comprenant une composition selon la revendication 1 et au moins une substance alimentaire.

27. Aliment selon la revendication 26, où ledit aliment est destiné aux êtres humains, à un animal de ferme ou à un animal de compagnie.

28. Composition comprenant une protéine du lait et une composition selon la revendication 1.

29. Procédé de traitement d'une plante ou d'une partie d'une plante, comprenant :
a) la fourniture
- i) d'une protéine de fusion recombinante, où ladite protéine comprend une molécule ciblant un micro-organisme jointe à au moins une partie d'une molécule biocide, où ladite molécule de liaison à un micro-organisme est CD14 et se lie à des bactéries ; et
- ii) d'une plante suspectée d'être infectée ou contaminée par des bactéries ; et
b) l'application de ladite protéine de fusion recombinante sur ladite plante ou partie de plante dans des conditions telles que ladite protéine de fusion recombinante neutralise lesdites bactéries.

30. Procédé selon la revendication 29, dans lequel ladite plante ou partie de plante est choisie dans le groupe constitué par une graine, un légume, une tige, une feuille, une racine, une plante en croissance et un fruit.

31. Organisme transgénique non humain comprenant une séquence d'acide nucléique codant pour une molécule ciblant un micro-organisme liée à une partie d'une molécule de protéine biocide, où ladite molécule ciblant un micro-organisme se lie à des bactéries et où ladite molécule ciblant un micro-organisme est CD14, et où ladite protéine de fusion neutralise lesdites bactéries.

32. Organismes transgéniques selon la revendication 31, où ledit organisme transgénique est choisi dans le groupe constitué par un animal, une plante et un micro-organisme.
